# EUROPEAN PATENT APPLICATION

(11) **EP 3 228 187 A1**
(43) Date of publication of application: **11.10.2017**
(21) Application number: 16164132.9
(22) Date of filing: 06.04.2016
(51) Int. Cl.: A01H 5/10, C07K 14/415, C12N 15/82

(54) **NOVEL MEANS AND METHODS FOR CEREAL PATHOGEN RESISTANCE**

(71) Applicant: KWS SAAT SE, 37574 Einbeck (DE)
(72) Inventor: TAPSELL, Chris, Thriplow, Nr. Royston, Hertfordshire SG8 7RE (GB); BYRNE, Edward, Thriplow, Nr. Royston, Hertfordshire SG8 7RE (GB); SADANANDOM, Ari, Durham, DH1 3LE (GB); CAMPANARO, Alberto, Durham, DH1 3LE (GB); MILLYARD, Linda, Durham, DH1 3LE (GB); GATEHOUSE, Angharad Margaret, Newcastle upon Tyne, NE1 7RU (GB); EDWARDS, Martin, Newcastle upon Tyne, NE1 7RU (GB); LIEFERT, Carlo, Newcastle upon Tyne, NE1 7RU (GB); POLE, Allister, Newcastle upon Tyne, NE1 7RU (GB)

(57) **Abstract**

The present disclosure provides means and methods for conferring pathogen resistance to, or increasing pathogen resistance of, a cereal plant cell, or a cereal plant or part thereof, comprising inhibiting the expression or activity of the at least one WRKY protein in a cereal plant cell, or a cereal plant or part thereof, as compared to a corresponding control plant cell, or control plant or part thereof, lacking the inhibition of the expression or activity of the at least one WRKY protein, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. The present invention also provides cereal plant cells, or cereal plants or parts thereof, carrying the inhibition of the expression or activity of the at least one WRKY protein. In particular, the pathogen is Zymoseptoria tritici.

SEQUENCE LISTING

<110>  KWS SAAT SE

<120>  NOVEL MEANS AND METHODS FOR CEREAL PATHOGEN RESISTANCE

<130>  KWS0237EP

<160>  72

<170>  PatentIn version 3.5

<210>  1
<211>  11
<212>  PRT
<213>  Artificial Sequence

<220>
<223>  WRKY sequence motif


<220>
<221>  VARIANT
<222>  (2)..(2)
<223>  Gly or Glu

<220>
<221>  VARIANT
<222>  (3)..(3)
<223>  Xaa is a hydrophobic amino acid

<220>
<221>  VARIANT
<222>  (4)..(4)
<223>  Xaa is a polar, non-hydrophobic amino acid

<220>
<221>  VARIANT
<222>  (10)..(10)
<223>  Lys or Glu or Gln

<220>
<221>  VARIANT
<222>  (11)..(11)
<223>  Lys or Glu

<400>  1

Asp Xaa Xaa Xaa Trp Arg Lys Tyr Gly Xaa Xaa
1               5                   10


<210>  2
<211>  16
<212>  PRT
<213>  Artificial Sequence

<220>
<223>  WRKY sequence motif


<220>
<221>  VARIANT

<222>  (2)..(2)
<223>  Gly or Glu

<220>
<221>  VARIANT
<222>  (3)..(3)
<223>  Xaa is a hydrophobic amino acid

<220>
<221>  VARIANT
<222>  (4)..(4)
<223>  Xaa is a polar, non-hydrophobic amino acid

<220>
<221>  VARIANT
<222>  (10)..(10)
<223>  Lys or Glu or Gln

<220>
<221>  VARIANT
<222>  (11)..(11)
<223>  Lys or Glu

<220>
<221>  VARIANT
<222>  (12)..(13)
<223>  Xaa can be any naturally occuring amino acid

<220>
<221>  VARIANT
<222>  (14)..(14)
<223>  Xaa is a polar, non-aromatic amino acid

<220>
<221>  VARIANT
<222>  (15)..(15)
<223>  Xaa can be any naturally occuring amino acid

<220>
<221>  VARIANT
<222>  (16)..(16)
<223>  Xaa is a polar, non-hydrophobic amino acid

<400>  2

Asp Xaa Xaa Xaa Trp Arg Lys Tyr Gly Xaa Xaa Xaa Xaa Xaa Xaa Xaa
1               5                   10                  15


<210>  3
<211>  678
<212>  DNA
<213>  Triticum aestivum

<400>  3
atggcagctt cgctgggact taccggccat gagaccagct acccggccca cccggcggcc      60

tcctccttgt acaacttcca agccggtgat cccttcgcgg cagcggcggg cggtgccgcg     120

gagctgagct tcccggagct cgtggacgcc gctcggccct ccgactactc gctgcttccg     180

gcgttcggcg ccgctggagg cgaggcgatg aacatgtacg agcggagtgt cgtcttccct     240

atgacgacct cctactactg cgacggcgcc gggatgttcg acggcgacgc ttcggcgagg     300

gcgagaggag gagggatcgg tgctatggcg gggcggccgt cggggaggat cgggttccgg     360

actcggtcgg aggtggaggt gctagacgac gggttccggt ggaggaagta cggcaagaag     420

gcggtgaaga gcagccccaa cctgcgcaac tactaccggt gctccgccga ggggtgcggg     480

gtgaagaagc gggtcgagcg cgaccgcgac gacccgcact acgtcctcac cacctacgac     540

ggcgtccaca accacgtcac ccctggcagc acgccgaggg cgtccccggc gtactcggcg     600

ccggcggcac ccgcttggac ctggagcgag ctgcatgcgg cggcggcggc gggggctcac     660

tcctcggagt cctactga                                                   678

<210>  4
<211>  225
<212>  PRT
<213>  Triticum aestivum

<400>  4

Met Ala Ala Ser Leu Gly Leu Thr Gly His Glu Thr Ser Tyr Pro Ala
1               5                   10                  15

His Pro Ala Ala Ser Ser Leu Tyr Asn Phe Gln Ala Gly Asp Pro Phe
            20                  25                  30

Ala Ala Ala Ala Gly Gly Ala Ala Glu Leu Ser Phe Pro Glu Leu Val
        35                  40                  45

Asp Ala Ala Arg Pro Ser Asp Tyr Ser Leu Leu Pro Ala Phe Gly Ala
    50                  55                  60

Ala Gly Gly Glu Ala Met Asn Met Tyr Glu Arg Ser Val Val Phe Pro
65                  70                  75                  80

Met Thr Thr Ser Tyr Tyr Cys Asp Gly Ala Gly Met Phe Asp Gly Asp
                85                  90                  95

Ala Ser Ala Arg Ala Arg Gly Gly Gly Ile Gly Ala Met Ala Gly Arg
            100                 105                 110

Pro Ser Gly Arg Ile Gly Phe Arg Thr Arg Ser Glu Val Glu Val Leu
        115                 120                 125

Asp Asp Gly Phe Arg Trp Arg Lys Tyr Gly Lys Lys Ala Val Lys Ser
    130                 135                 140

Ser Pro Asn Leu Arg Asn Tyr Tyr Arg Cys Ser Ala Glu Gly Cys Gly
145                 150                 155                 160

Val Lys Lys Arg Val Glu Arg Asp Arg Asp Asp Pro His Tyr Val Leu
                165                 170                 175

Thr Thr Tyr Asp Gly Val His Asn His Val Thr Pro Gly Ser Thr Pro
            180                 185                 190

Arg Ala Ser Pro Ala Tyr Ser Ala Pro Ala Ala Pro Ala Trp Thr Trp
        195                 200                 205

Ser Glu Leu His Ala Ala Ala Ala Ala Gly Ala His Ser Ser Glu Ser
    210                 215                 220

Tyr
225

<210>  5
<211>  669
<212>  DNA
<213>  Triticum aestivum

<400>  5
atggccctgg actccgtccc ttcctacccc agcgacctgg gatccagcgg cagggcaacc      60

aggacccagc aaagaatccg gaaggaggag cgaacgtgga cggcggacac ctacgcgccg     120

tacgacgacg ggcaccagtg gaggaagtac ggcgagaaga agctctccaa ctcctacttc     180

ccgaggttct actacagatg tacctacaag actgacctga agtgccctgc gacaaaacaa     240

gtccaacaga aggacatgag cgacccacca ttgttcacgg tcacttactt caaccatcac     300

agctgcaaca ccacctcgag gcccatcggc agcgccccag acaccactga gcagtcgtcc     360

tcgaggaggg cagtgtccat ctgcttcggg tcgcatgcca ccggcgagca gccgacattc     420

ctaacgtcgc cgggcacact gcagtcacca gcgagcacaa ccaaccaaca gaacgacaga     480

ggcgcttacg gtcaccagtt tcagtggaca gacacatcac cgtcggcagg cgatgcccca     540

gtcaagatgg agaccgacag tctcgccgga acaggcgctt cgtctggcgc tgctagcggc     600

catgctctgt cgaggacact gctgccgatc ggccagtcga gatgcatcga gtactttcag     660

ttcttgtga                                                             669

<210>  6
<211>  222
<212>  PRT
<213>  Triticum aestivum

<400>  6

Met Ala Leu Asp Ser Val Pro Ser Tyr Pro Ser Asp Leu Gly Ser Ser
1               5                   10                  15

Gly Arg Ala Thr Arg Thr Gln Gln Arg Ile Arg Lys Glu Glu Arg Thr
            20                  25                  30

Trp Thr Ala Asp Thr Tyr Ala Pro Tyr Asp Asp Gly His Gln Trp Arg
        35                  40                  45

Lys Tyr Gly Glu Lys Lys Leu Ser Asn Ser Tyr Phe Pro Arg Phe Tyr
    50                  55                  60

Tyr Arg Cys Thr Tyr Lys Thr Asp Leu Lys Cys Pro Ala Thr Lys Gln
65                  70                  75                  80

Val Gln Gln Lys Asp Met Ser Asp Pro Pro Leu Phe Thr Val Thr Tyr
                85                  90                  95

Phe Asn His His Ser Cys Asn Thr Thr Ser Arg Pro Ile Gly Ser Ala
            100                 105                 110

Pro Asp Thr Thr Glu Gln Ser Ser Ser Arg Arg Ala Val Ser Ile Cys
        115                 120                 125

Phe Gly Ser His Ala Thr Gly Glu Gln Pro Thr Phe Leu Thr Ser Pro
    130                 135                 140

Gly Thr Leu Gln Ser Pro Ala Ser Thr Thr Asn Gln Gln Asn Asp Arg
145                 150                 155                 160

Gly Ala Tyr Gly His Gln Phe Gln Trp Thr Asp Thr Ser Pro Ser Ala
                165                 170                 175

Gly Asp Ala Pro Val Lys Met Glu Thr Asp Ser Leu Ala Gly Thr Gly
            180                 185                 190

Ala Ser Ser Gly Ala Ala Ser Gly His Ala Leu Ser Arg Thr Leu Leu
        195                 200                 205

Pro Ile Gly Gln Ser Arg Cys Ile Glu Tyr Phe Gln Phe Leu
    210                 215                 220

<210>  7
<211>  1407
<212>  DNA
<213>  Triticum aestivum

<400>  7
atggcggcgg ggcagtggtc aggcatcggc gacggcggcg gcctctgggc cccgcccgcg      60

ctcgacagcc tcttccccga cgaccagccg tcgccggccg cctcggcgct gggcttcttc     120

ggtggatccc tcgcgcagct cccttcccct ccgccgctct gcggcaccgc gctcctcggg     180

tacccccagg acaactttga tgtgttccat gaacaagacc tagcacagct ggcagcacaa 240

gtagctcaaa agaaagagtt gcgggaaaaa caaggggcgg gattgcatca caagattgga 300

cctcaactag ctttttctaa atacagtata cttgatcaag tggacaactc ttcttctttc 360

tcattggcaa cttcagtgct gacacctcag catgtcagtt cttccgtagg cgcggcatca 420

atgcagggac agactttgcc atcacacact ggtagtggta gtgtcaacac tggaccaact 480

ggagttttac aagtgctcca agattcatcc accactctgg acagtatcaa cactggatca 540

actggagttc tggaagcact ccaaggttca tccatcactc tggatagacc tgctgatgat 600

ggatacaact ggcgtaagta tggacaaaag gcagtcaagg gtgggaagta tccaaggagc 660

tattacaaat gtaccctgaa ttgcccggtc aggaaaaatg tagagcactc tgcagatgga 720

cgaattatta aaataattta tagaggtcag cactgccatg aacccccctc aaagaggttt 780

aaagattgtg gtgatttatt gaatgagtta aatgatttca atgatgccaa ggagccttca 840

actaaatcac aattaggttg tcaaggttat tatggaaaac ctataacgcc aaatggaatg 900

atgacggatg ttttattgcc aacgaaggaa gagggggatg agcaattatc tagtttaagt 960

gatatccggg aaggtgatgg tgaaataaga actgttgatg gagatgatgg tgatgccgat 1020

gcaaatgaaa ggaatgcacc aggtcaaaag attatcgtga gtacaacgag cgatgttgat 1080

cttttggacg acggctatag gtggcgcaag tatggacaga aagtggtgag aggaaatcct 1140

cacccaagga gctattacaa gtgcacttac caaggatgcg acgtcaagaa gcatatcgag 1200

agatcttccg aggaaccaca tgctgtgata actacatacg aagggaagca tacgcatgac 1260

gtgcctgagt ctaggaacag aagccaagcc acaggtcaac accactgcaa agagcagact 1320

tattcagaac aatcagctgc aagcttctgc agtagctcgg aaaagagaaa atatggaaca 1380

gccattctga acgatctcgc cttctag 1407

<210> 8
<211> 468
<212> PRT
<213> Triticum aestivum

<400> 8

Met Ala Ala Gly Gln Trp Ser Gly Ile Gly Asp Gly Gly Gly Leu Trp
1               5                   10                  15

Ala Pro Pro Ala Leu Asp Ser Leu Phe Pro Asp Asp Gln Pro Ser Pro
            20                  25                  30

Ala Ala Ser Ala Leu Gly Phe Phe Gly Gly Ser Leu Ala Gln Leu Pro
        35                  40                  45

Ser Pro Pro Pro Leu Cys Gly Thr Ala Leu Leu Gly Tyr Pro Gln Asp
    50                  55                  60

Asn Phe Asp Val Phe His Glu Gln Asp Leu Ala Gln Leu Ala Ala Gln
65                  70                  75                  80

Val Ala Gln Lys Lys Glu Leu Arg Glu Lys Gln Gly Ala Gly Leu His
                85                  90                  95

His Lys Ile Gly Pro Gln Leu Ala Phe Ser Lys Tyr Ser Ile Leu Asp
            100                 105                 110

Gln Val Asp Asn Ser Ser Ser Phe Ser Leu Ala Thr Ser Val Leu Thr
        115                 120                 125

Pro Gln His Val Ser Ser Ser Val Gly Ala Ala Ser Met Gln Gly Gln
    130                 135                 140

Thr Leu Pro Ser His Thr Gly Ser Gly Ser Val Asn Thr Gly Pro Thr
145                 150                 155                 160

Gly Val Leu Gln Val Leu Gln Asp Ser Ser Thr Thr Leu Asp Ser Ile
                165                 170                 175

Asn Thr Gly Ser Thr Gly Val Leu Glu Ala Leu Gln Gly Ser Ser Ile
            180                 185                 190

Thr Leu Asp Arg Pro Ala Asp Asp Gly Tyr Asn Trp Arg Lys Tyr Gly
        195                 200                 205

Gln Lys Ala Val Lys Gly Gly Lys Tyr Pro Arg Ser Tyr Tyr Lys Cys
    210                 215                 220

Thr Leu Asn Cys Pro Val Arg Lys Asn Val Glu His Ser Ala Asp Gly
225                 230                 235                 240

Arg Ile Ile Lys Ile Ile Tyr Arg Gly Gln His Cys His Glu Pro Pro
                245                 250                 255

Ser Lys Arg Phe Lys Asp Cys Gly Asp Leu Leu Asn Glu Leu Asn Asp
            260                 265                 270

Phe Asn Asp Ala Lys Glu Pro Ser Thr Lys Ser Gln Leu Gly Cys Gln
        275                 280                 285

Gly Tyr Tyr Gly Lys Pro Ile Thr Pro Asn Gly Met Met Thr Asp Val
    290                 295                 300

Leu Leu Pro Thr Lys Glu Glu Gly Asp Glu Gln Leu Ser Ser Leu Ser
305                 310                 315                 320

Asp Ile Arg Glu Gly Asp Gly Glu Ile Arg Thr Val Asp Gly Asp Asp
                325                 330                 335

Gly Asp Ala Asp Ala Asn Glu Arg Asn Ala Pro Gly Gln Lys Ile Ile
            340                 345                 350

Val Ser Thr Thr Ser Asp Val Asp Leu Leu Asp Asp Gly Tyr Arg Trp
        355                 360                 365

Arg Lys Tyr Gly Gln Lys Val Val Arg Gly Asn Pro His Pro Arg Ser
    370                 375                 380

Tyr Tyr Lys Cys Thr Tyr Gln Gly Cys Asp Val Lys Lys His Ile Glu
385                 390                 395                 400

Arg Ser Ser Glu Glu Pro His Ala Val Ile Thr Thr Tyr Glu Gly Lys
                405                 410                 415

His Thr His Asp Val Pro Glu Ser Arg Asn Arg Ser Gln Ala Thr Gly
            420                 425                 430

Gln His His Cys Lys Glu Gln Thr Tyr Ser Glu Gln Ser Ala Ala Ser
        435                 440                 445

Phe Cys Ser Ser Ser Glu Lys Arg Lys Tyr Gly Thr Ala Ile Leu Asn
    450                 455                 460

Asp Leu Ala Phe
465

<210>  9
<211>  1905
<212>  DNA
<213>  Triticum aestivum

<400>  9
atgtcagatc tgggcctccc agccagctcg ccctttttta tacgcgcccc gtcctaccgg      60

ggagagactt cagactcctc cctctttcgg tctctacgac acgtttcttt ctctctccct     120

ctcccctcgg ctaccaaggc gtctctctct tctccaagga ggctctcgat ttgcatggac     180

aagggccacc tcggtggtgg cggaggagga ggaggactgc tagccctgga cgcctcgccg     240

cggcagctcg gcttcttgaa cttgctatcg ccggcgccgt tccataggag catggaggcg     300

gacgacggcg gaggcggtgg cggcggcggc aggggaggga ggtccatcga ggttgacttc     360

ttctccgacg agaagaagaa catgaagaag agccgggctt cggccggggc cgacgcggag 420

gaccacaagg atcaagcctc cgccgccgga ctcgccatca agaaggagga cctcaccatt 480

aacctccttc ccgggaacaa cacgaggagc gaccggtcca tggtggtcga cgacgacgga 540

gcgtcccggg ccgaccaaga caggaacggc aggaacaccg gcgagctggc ggtgatacag 600

gccgagctga gccgcatgaa cgaggagaac cagcggctgc gagggatgtt gacccaggtc 660

aacaacagct accatgcgct gcagatgcat ctcgtcgcgc tcatgcagca aaggacccag 720

atgcctccgg tccagccgca acaacctccg acccacgagg atggcaagaa cgagagtgcc 780

atcgtgccga ggcagttcct gggtctgggc ccgtcgggag ccagcgctga cgtggcggaa 840

gagccctcca actcgtccac ggaggtcggg agcccgcggc ggtcgtcgtc caacggaaac 900

gaggaccctg agcgcggcga aaaccctgac ggcccgtcca ccgcggggtg gttgcccgga 960

cgcgggatga gccagcagca gcagcagcag ctgggggcgg cggcgaaggg ccacgaccag 1020

caagcgcagg aggccaccat gaggaaggcc cgtgtctccg ttcgcgcccg atccgaagcc 1080

ccgattatcg ccgatggatg ccaatggagg aagtacggtc agaagatggc caagggaaac 1140

ccttgcccac gcgcctacta ccggtgcacc atggccaccg gctgcccggt gcgcaagcag 1200

gtgcaacgat gcgccgagga ccggaccatc ctcatcacca cgtacgaggg cacgcacaac 1260

cacccgctcc cgccggccgc catggccatg gcgtctacca cgtcggccgc tgcgtccatg 1320

ctgctctccg gctccatgcc cagcgccgac ggcgcggggc tcatgagctc caacttcctc 1380

gcgcgcaccg tgcttccgtg ctcgtccagc atggccacca tctcggcgtc cgcgccgttc 1440

ccgacggtca cgctcgacct cacccacgcc ccgcccgggg cgcccaacgc catgccgcta 1500

aacgtcgccc gaccgcatgc accggggcag ttccatgttc cgatgcccgg cggtgggatg 1560

gccccggcct tcgcgatgcc gccgcacatg ctgtacaacc agtccaagtt ctctgggctg 1620

cagatgtcct ccgactcggt ggacgccggg cagttcgcgc agcctaggcc gccgatgggc 1680

ctgccaggcc agctgtccga caccgtcagc gcagcggcgg ccgcgatcac cgcggaccca 1740

aacttcacag tggcgctcgc cgcggccatc tcgtcgatca tggccggcca gcacgccgcc 1800

ggcaacagca acgcgaacaa cagcaacaac aacaccagta acaacaacgt gacgacgact 1860

agtaacaaca cgacgagcaa caacaccaac agcgagaccc aatga 1905

<210> 10
<211> 634
<212> PRT
<213> Triticum aestivum

<400> 10

Met Ser Asp Leu Gly Leu Pro Ala Ser Ser Pro Phe Phe Ile Arg Ala
1               5                   10                  15

Pro Ser Tyr Arg Gly Glu Thr Ser Asp Ser Ser Leu Phe Arg Ser Leu
            20                  25                  30

Arg His Val Ser Phe Ser Leu Pro Leu Pro Ser Ala Thr Lys Ala Ser
        35                  40                  45

Leu Ser Ser Pro Arg Arg Leu Ser Ile Cys Met Asp Lys Gly His Leu
    50                  55                  60

Gly Gly Gly Gly Gly Gly Gly Gly Leu Leu Ala Leu Asp Ala Ser Pro
65                  70                  75                  80

Arg Gln Leu Gly Phe Leu Asn Leu Leu Ser Pro Ala Pro Phe His Arg
                85                  90                  95

Ser Met Glu Ala Asp Asp Gly Gly Gly Gly Gly Gly Gly Gly Arg Gly
            100                 105                 110

Arg Arg Ser Ile Glu Val Asp Phe Phe Ser Asp Glu Lys Lys Asn Met
        115                 120                 125

Lys Lys Ser Arg Ala Ser Ala Gly Ala Asp Ala Glu Asp His Lys Asp
    130                 135                 140

Gln Ala Ser Ala Ala Gly Leu Ala Ile Lys Lys Glu Asp Leu Thr Ile
145                 150                 155                 160

Asn Leu Leu Pro Gly Asn Asn Thr Arg Ser Asp Arg Ser Met Val Val
                165                 170                 175

Asp Asp Asp Gly Ala Ser Arg Ala Asp Gln Asp Arg Asn Gly Arg Asn
            180                 185                 190

Thr Gly Glu Leu Ala Val Ile Gln Ala Glu Leu Ser Arg Met Asn Glu
        195                 200                 205

Glu Asn Gln Arg Leu Arg Gly Met Leu Thr Gln Val Asn Asn Ser Tyr
    210                 215                 220

His Ala Leu Gln Met His Leu Val Ala Leu Met Gln Gln Arg Thr Gln
225                 230                 235                 240

Met Pro Pro Val Gln Pro Gln Gln Pro Pro Thr His Glu Asp Gly Lys
                245                 250                 255

Asn Glu Ser Ala Ile Val Pro Arg Gln Phe Leu Gly Leu Gly Pro Ser
            260                 265                 270

Gly Ala Ser Ala Asp Val Ala Glu Glu Pro Ser Asn Ser Ser Thr Glu
        275                 280                 285

Val Gly Ser Pro Arg Arg Ser Ser Ser Asn Gly Asn Glu Asp Pro Glu
    290                 295                 300

Arg Gly Glu Asn Pro Asp Gly Pro Ser Thr Ala Gly Trp Leu Pro Gly
305                 310                 315                 320

Arg Gly Met Ser Gln Gln Gln Gln Gln Gln Leu Gly Ala Ala Ala Lys
                325                 330                 335

Gly His Asp Gln Gln Ala Gln Glu Ala Thr Met Arg Lys Ala Arg Val
            340                 345                 350

Ser Val Arg Ala Arg Ser Glu Ala Pro Ile Ile Ala Asp Gly Cys Gln
        355                 360                 365

Trp Arg Lys Tyr Gly Gln Lys Met Ala Lys Gly Asn Pro Cys Pro Arg
    370                 375                 380

Ala Tyr Tyr Arg Cys Thr Met Ala Thr Gly Cys Pro Val Arg Lys Gln
385                 390                 395                 400

Val Gln Arg Cys Ala Glu Asp Arg Thr Ile Leu Ile Thr Thr Tyr Glu
                405                 410                 415

Gly Thr His Asn His Pro Leu Pro Pro Ala Ala Met Ala Met Ala Ser
            420                 425                 430

Thr Thr Ser Ala Ala Ala Ser Met Leu Leu Ser Gly Ser Met Pro Ser
        435                 440                 445

Ala Asp Gly Ala Gly Leu Met Ser Ser Asn Phe Leu Ala Arg Thr Val
    450                 455                 460

Leu Pro Cys Ser Ser Ser Met Ala Thr Ile Ser Ala Ser Ala Pro Phe
465                 470                 475                 480

Pro Thr Val Thr Leu Asp Leu Thr His Ala Pro Pro Gly Ala Pro Asn
                485                 490                 495

Ala Met Pro Leu Asn Val Ala Arg Pro His Ala Pro Gly Gln Phe His
            500                 505                 510

Val Pro Met Pro Gly Gly Gly Met Ala Pro Ala Phe Ala Met Pro Pro

515                 520                 525

His Met Leu Tyr Asn Gln Ser Lys Phe Ser Gly Leu Gln Met Ser Ser
    530                 535                 540

Asp Ser Val Asp Ala Gly Gln Phe Ala Gln Pro Arg Pro Pro Met Gly
545                 550                 555                 560

Leu Pro Gly Gln Leu Ser Asp Thr Val Ser Ala Ala Ala Ala Ala Ile
                565                 570                 575

Thr Ala Asp Pro Asn Phe Thr Val Ala Leu Ala Ala Ala Ile Ser Ser
            580                 585                 590

Ile Met Ala Gly Gln His Ala Ala Gly Asn Ser Asn Ala Asn Asn Ser
        595                 600                 605

Asn Asn Asn Thr Ser Asn Asn Asn Val Thr Thr Thr Ser Asn Asn Thr
    610                 615                 620

Thr Ser Asn Asn Thr Asn Ser Glu Thr Gln
625                 630

<210>  11
<211>  660
<212>  DNA
<213>  Triticum aestivum

<400>  11
atggacgagc agtggatgat ggggcagact tccctcagcc tcggcctcaa cgtcggccgg      60

ccgacggcgc ggcggggtgc ccctccggtg accaagatcc tcgtggagga ggacttcatg      120

tcctccaaga agaatcacga ggtcgaggcg ctggaggccg agctccggcg cgtgggcgag      180

gagaacagga ggctcagcga catgctccgc gcgctggtgg ccaagtactc cgaccttcag      240

ggcagggtca gcggcatgat ggcggcggcg gccgcggcca acaaccgcca gtcacccacg      300

acgtctgagg gcggctccgc ggcctcgccg tccaggaagc gcgctcgcag cgacagtccc      360

gacaccgcag tccaggaagc gtaccctgcc agcggcggct tcgccgtcag cgtcaacgtc      420

ggccccgacc aggcggagtg cacgtccgtc cacgagccgg gcaacagcaa gcgcgtccgc      480

gccgacgagt gcaaggccag ccgggtctcc aagctctacg tccacgccga ccccgccgac      540

ctcagcctcg tggtgaagga tgggtaccaa tggcgaaagt acggccaaga aggtgaccaa      600

ggacaaccca tgcccgaggg cctacttccg gtgctccgtt cgcgccgtcc tgccaggtga      660

<210>  12
<211>  219
<212>  PRT

<213>  Triticum aestivum

<400>  12

Met Asp Glu Gln Trp Met Met Gly Gln Thr Ser Leu Ser Leu Gly Leu
1               5                   10                  15

Asn Val Gly Arg Pro Thr Ala Arg Arg Gly Ala Pro Pro Val Thr Lys
            20                  25                  30

Ile Leu Val Glu Glu Asp Phe Met Ser Ser Lys Lys Asn His Glu Val
        35                  40                  45

Glu Ala Leu Glu Ala Glu Leu Arg Arg Val Gly Glu Glu Asn Arg Arg
    50                  55                  60

Leu Ser Asp Met Leu Arg Ala Leu Val Ala Lys Tyr Ser Asp Leu Gln
65                  70                  75                  80

Gly Arg Val Ser Gly Met Met Ala Ala Ala Ala Ala Ala Asn Asn Arg
                85                  90                  95

Gln Ser Pro Thr Thr Ser Glu Gly Gly Ser Ala Ala Ser Pro Ser Arg
            100                 105                 110

Lys Arg Ala Arg Ser Asp Ser Pro Asp Thr Ala Val Gln Glu Ala Tyr
        115                 120                 125

Pro Ala Ser Gly Gly Phe Ala Val Ser Val Asn Val Gly Pro Asp Gln
    130                 135                 140

Ala Glu Cys Thr Ser Val His Glu Pro Gly Asn Ser Lys Arg Val Arg
145                 150                 155                 160

Ala Asp Glu Cys Lys Ala Ser Arg Val Ser Lys Leu Tyr Val His Ala
                165                 170                 175

Asp Pro Ala Asp Leu Ser Leu Val Val Lys Asp Gly Tyr Gln Trp Arg
            180                 185                 190

Lys Tyr Gly Gln Glu Gly Asp Gln Gly Gln Pro Met Pro Glu Gly Leu
        195                 200                 205

Leu Pro Val Leu Arg Ser Arg Arg Pro Ala Arg
    210                 215

<210>  13
<211>  1323
<212>  DNA

<213>  Triticum aestivum

<400>  13
atgtcctcct ccacggggag cttggaccac gcagggttca cgttcacgcc gccgccgttc      60

atcacgtcct tcaccgagct tctgtcgggg tccggcgccg gcgacgcgga gcggtcgccg     120

agggggttca accgaggggg ccgggccggg gcgcccaagt tcaagtccgc gcagccgccc     180

agcctgccca tctcgtcgcc cttctcctgc ttctccgtcc ctgcaggtct cagccctgcc     240

gagctgctcg actcccccgt tctcctcaac tactctcaca tcttggcgtc tccgactacc     300

ggtgcgatcc ctgcgcagag gtgcgactgg caggtgagcg ccgatctgaa cacctttcag     360

caggatgagc tcggcctctc tggcttctcc tttcacgcag tcaagtccaa cgccacggtc     420

aacgctcaag caaactgctt acctttattc aaggagcagc aggagcaaca acaagaagaa     480

gtggttcaag tgagcaacaa gagcagcagc agcagcggca acaacaagca ggttgaggac     540

ggatacaatt ggaggaagta cgggcagaag caagttaagg ggagcgagaa cccgcggagc     600

tactacaagt gcacctacaa caattgctcc atgaagaaga aagtggagcg ctctctcgcc     660

gacggccgca tcacgcagat cgtctacaag ggcgcacatg accacccgaa gcccctctcc     720

acgcgccgca actcctccgg ctgcgcggcg gtcgttgcgg aggatcacac caacggctcg     780

gagcactctg gcccgacgcc cgagaattca tccgtcactt tcggagacga tgaggccgac     840

aagcccgaga ccaagcgccg gaaggagcat ggtgacaacg agggcagttc aggcggcacc     900

ggcggctgcg ggaagcccgt gcgcgagccc aggctcgtgg tgcagacgct gagcgatata     960

gacatactcg acgacggctt ccggtggagg aagtacgggc agaaggttgt caagggcaat    1020

cccaacccca ggagctacta caagtgcaca acggtgggct gcccggtgcg caagcatgtg    1080

gagcgggcct cgcacgacaa ccgcgcggtg atcaccacct acgagggtaa gcacagccac    1140

gacgtgccga tcggccgggg ccgcgcgctg ccggcgtcat cttcctccga cagctcggcc    1200

gtcatctggc ctgccgccgc cgtgcaagcc ccgtgcaccc tcgagatgct cgccggacac    1260

ccaggctacg cggccaagga cgagccccgg gacgacatgt tcgtcgagtc gctcctctgc    1320

tag                                                                  1323

<210>  14
<211>  440
<212>  PRT
<213>  Triticum aestivum

<400>  14

Met Ser Ser Ser Thr Gly Ser Leu Asp His Ala Gly Phe Thr Phe Thr
1               5                   10                  15

Pro Pro Pro Phe Ile Thr Ser Phe Thr Glu Leu Leu Ser Gly Ser Gly
            20                  25                  30

Ala Gly Asp Ala Glu Arg Ser Pro Arg Gly Phe Asn Arg Gly Gly Arg
        35                  40                  45

Ala Gly Ala Pro Lys Phe Lys Ser Ala Gln Pro Pro Ser Leu Pro Ile
    50                  55                  60

Ser Ser Pro Phe Ser Cys Phe Ser Val Pro Ala Gly Leu Ser Pro Ala
65                  70                  75                  80

Glu Leu Leu Asp Ser Pro Val Leu Leu Asn Tyr Ser His Ile Leu Ala
                85                  90                  95

Ser Pro Thr Thr Gly Ala Ile Pro Ala Gln Arg Cys Asp Trp Gln Val
            100                 105                 110

Ser Ala Asp Leu Asn Thr Phe Gln Gln Asp Glu Leu Gly Leu Ser Gly
        115                 120                 125

Phe Ser Phe His Ala Val Lys Ser Asn Ala Thr Val Asn Ala Gln Ala
    130                 135                 140

Asn Cys Leu Pro Leu Phe Lys Glu Gln Gln Glu Gln Gln Gln Glu Glu
145                 150                 155                 160

Val Val Gln Val Ser Asn Lys Ser Ser Ser Ser Ser Gly Asn Asn Lys
                165                 170                 175

Gln Val Glu Asp Gly Tyr Asn Trp Arg Lys Tyr Gly Gln Lys Gln Val
            180                 185                 190

Lys Gly Ser Glu Asn Pro Arg Ser Tyr Tyr Lys Cys Thr Tyr Asn Asn
        195                 200                 205

Cys Ser Met Lys Lys Lys Val Glu Arg Ser Leu Ala Asp Gly Arg Ile
    210                 215                 220

Thr Gln Ile Val Tyr Lys Gly Ala His Asp His Pro Lys Pro Leu Ser
225                 230                 235                 240

Thr Arg Arg Asn Ser Ser Gly Cys Ala Ala Val Val Ala Glu Asp His
                245                 250                 255

Thr Asn Gly Ser Glu His Ser Gly Pro Thr Pro Glu Asn Ser Ser Val
            260                 265                 270

Thr Phe Gly Asp Asp Glu Ala Asp Lys Pro Glu Thr Lys Arg Arg Lys

275                 280                 285

Glu His Gly Asp Asn Glu Gly Ser Ser Gly Gly Thr Gly Gly Cys Gly
    290                 295                 300

Lys Pro Val Arg Glu Pro Arg Leu Val Val Gln Thr Leu Ser Asp Ile
305                 310                 315                 320

Asp Ile Leu Asp Asp Gly Phe Arg Trp Arg Lys Tyr Gly Gln Lys Val
                325                 330                 335

Val Lys Gly Asn Pro Asn Pro Arg Ser Tyr Tyr Lys Cys Thr Thr Val
            340                 345                 350

Gly Cys Pro Val Arg Lys His Val Glu Arg Ala Ser His Asp Asn Arg
        355                 360                 365

Ala Val Ile Thr Thr Tyr Glu Gly Lys His Ser His Asp Val Pro Ile
    370                 375                 380

Gly Arg Gly Arg Ala Leu Pro Ala Ser Ser Ser Ser Asp Ser Ser Ala
385                 390                 395                 400

Val Ile Trp Pro Ala Ala Ala Val Gln Ala Pro Cys Thr Leu Glu Met
                405                 410                 415

Leu Ala Gly His Pro Gly Tyr Ala Ala Lys Asp Glu Pro Arg Asp Asp
            420                 425                 430

Met Phe Val Glu Ser Leu Leu Cys
        435                 440

<210>  15
<211>  987
<212>  DNA
<213>  Triticum aestivum

<400>  15
atggccgtgg accccatggg ctgctacacc cctcgccgcg ccgacgacca gctcgccatc      60

caggaggccg ccaccgccgg cctccgcagc ctggagctcc tcgtctcctc cctctccggc     120

gcggcgccgt ccaaggcgcc gcagcagcac ctgcagcagc cgttcggcga gatcgccgac     180

caggccgtct ccaagttccg caaggtgatc tccatcctcg accgcaccgg ccacgcccgc     240

ttccgccgcg gccccgtcca gtcgcctacc ccgcctcctc cggctccggt cgctcctccg     300

ccgcccccac cgcgccctct ggccgtcgtc gagccggcca ggcccgctcc cttgaccgcc     360

gtggcgccgg tgtcggtggc cgccccggtc cctctcccgc agccgcagag cctgacgctg     420

gacttcacca agccgaacct gaccatgtca ggcgcgacgt ccgtgacgtc cacgtccttc 480

tttctctcgg tgaccgccgg cgagggcagc gtgtccaagg gccgcagcct ggtctccgcc 540

ggcaagccgc cgctgtccgg gcacaagaga aagccgtgcg ccggcgcgca ctcggaggcc 600

aacaccaccg gcagccgatg ccactgctcc aagagaagga agaaccgcgt gaagacgacg 660

gtgagggtgc ccgcggtgag cgcgaagatc gccgacatcc cgccggacga gtactcgtgg 720

aggaagtacg gccagaagcc catcaaggta tccccttacc cacggggcta ctacaagtgc 780

agcacagtgc gagggtgccc ggcgcggaag cacgtggagc gcgccctgga cgacccggcg 840

atgctggtgg tgacgtacga gggcgagcac cgccactcgc cggggccgat gccgatgcag 900

atggcgccgt cgccggtgcc gattccgatg ccgatgggcg cgcccgtagc cgtagctagt 960

gtgtccgccg gcaacgggca cgtctga 987

<210> 16
<211> 328
<212> PRT
<213> Triticum aestivum

<400> 16

Met Ala Val Asp Pro Met Gly Cys Tyr Thr Pro Arg Arg Ala Asp Asp
1               5                   10                  15

Gln Leu Ala Ile Gln Glu Ala Ala Thr Ala Gly Leu Arg Ser Leu Glu
            20                  25                  30

Leu Leu Val Ser Ser Leu Ser Gly Ala Ala Pro Ser Lys Ala Pro Gln
        35                  40                  45

Gln His Leu Gln Gln Pro Phe Gly Glu Ile Ala Asp Gln Ala Val Ser
    50                  55                  60

Lys Phe Arg Lys Val Ile Ser Ile Leu Asp Arg Thr Gly His Ala Arg
65                  70                  75                  80

Phe Arg Arg Gly Pro Val Gln Ser Pro Thr Pro Pro Pro Pro Ala Pro
                85                  90                  95

Val Ala Pro Pro Pro Pro Pro Pro Arg Pro Leu Ala Val Val Glu Pro
            100                 105                 110

Ala Arg Pro Ala Pro Leu Thr Ala Val Ala Pro Val Ser Val Ala Ala
        115                 120                 125

Pro Val Pro Leu Pro Gln Pro Gln Ser Leu Thr Leu Asp Phe Thr Lys
    130                 135                 140

Pro Asn Leu Thr Met Ser Gly Ala Thr Ser Val Thr Ser Thr Ser Phe
145                 150                 155                 160

Phe Leu Ser Val Thr Ala Gly Glu Gly Ser Val Ser Lys Gly Arg Ser
                165                 170                 175

Leu Val Ser Ala Gly Lys Pro Pro Leu Ser Gly His Lys Arg Lys Pro
            180                 185                 190

Cys Ala Gly Ala His Ser Glu Ala Asn Thr Thr Gly Ser Arg Cys His
        195                 200                 205

Cys Ser Lys Arg Arg Lys Asn Arg Val Lys Thr Thr Val Arg Val Pro
    210                 215                 220

Ala Val Ser Ala Lys Ile Ala Asp Ile Pro Pro Asp Glu Tyr Ser Trp
225                 230                 235                 240

Arg Lys Tyr Gly Gln Lys Pro Ile Lys Val Ser Pro Tyr Pro Arg Gly
                245                 250                 255

Tyr Tyr Lys Cys Ser Thr Val Arg Gly Cys Pro Ala Arg Lys His Val
            260                 265                 270

Glu Arg Ala Leu Asp Asp Pro Ala Met Leu Val Val Thr Tyr Glu Gly
        275                 280                 285

Glu His Arg His Ser Pro Gly Pro Met Pro Met Gln Met Ala Pro Ser
    290                 295                 300

Pro Val Pro Ile Pro Met Pro Met Gly Ala Pro Val Ala Val Ala Ser
305                 310                 315                 320

Val Ser Ala Gly Asn Gly His Val
                325

<210>  17
<211>  672
<212>  DNA
<213>  Triticum aestivum

<220>
<221>  misc_feature
<222>  (290)..(290)
<223>  n is a, c, g, or t

<400>  17
atggcggctt cgctaggact taccagccat gaagcctgct gcacggtcca ccccaccggc        60

tcctccttgt acaacttcca agccgatgac cccttcgtgg cagcagcgga cggtgccgcg     120

gagctgagct tcccggagct cgtggaagcc gctcgggcct ccgactactc gccgcttccg     180

gcgttcggcg tcgctggagg cgagacgatg aacatgtacg ggcggagcgt cgtcttccct     240

atgacgacct cgtactactg cgacggcgcc gggatgttcg acggcgacgn cttcggcgag     300

ggggagatag gggaagggat cggtgctatg accgggcggc cgtcggggag gatcgggttc     360

cggactcggt cggaggtgga ggtgatggac gacgggttcc ggtggaggaa gtacggcaag     420

aaggcggtga agagcagccc caacctgcgg aactactacc ggtgctccgc cgaggggtgc     480

ggggtgaaga agcggatcga gcgcgaccgc gacgacccgc gctacgtcct caccacctac     540

gacggcgtcc acaaccacgt cacccccggc agcacgtcgt cgagggcagc gccggcgtac     600

tcggcgccga cggcgcccgc ttggacctgg agcgagctgc atgcggcggc gcactcctcg     660

gagtcctact ga                                                         672

<210>  18
<211>  223
<212>  PRT
<213>  Triticum aestivum

<220>
<221>  misc_feature
<222>  (97)..(97)
<223>  Xaa can be any naturally occurring amino acid

<400>  18

Met Ala Ala Ser Leu Gly Leu Thr Ser His Glu Ala Cys Cys Thr Val
1               5                   10                  15

His Pro Thr Gly Ser Ser Leu Tyr Asn Phe Gln Ala Asp Asp Pro Phe
            20                  25                  30

Val Ala Ala Ala Asp Gly Ala Ala Glu Leu Ser Phe Pro Glu Leu Val
        35                  40                  45

Glu Ala Ala Arg Ala Ser Asp Tyr Ser Pro Leu Pro Ala Phe Gly Val
    50                  55                  60

Ala Gly Gly Glu Thr Met Asn Met Tyr Gly Arg Ser Val Val Phe Pro
65                  70                  75                  80

Met Thr Thr Ser Tyr Tyr Cys Asp Gly Ala Gly Met Phe Asp Gly Asp
                85                  90                  95

Xaa Phe Gly Glu Gly Glu Ile Gly Glu Gly Ile Gly Ala Met Thr Gly
            100                 105                 110

Arg Pro Ser Gly Arg Ile Gly Phe Arg Thr Arg Ser Glu Val Glu Val
        115                 120                 125

Met Asp Asp Gly Phe Arg Trp Arg Lys Tyr Gly Lys Lys Ala Val Lys
    130                 135                 140

Ser Ser Pro Asn Leu Arg Asn Tyr Tyr Arg Cys Ser Ala Glu Gly Cys
145                 150                 155                 160

Gly Val Lys Lys Arg Ile Glu Arg Asp Arg Asp Asp Pro Arg Tyr Val
                165                 170                 175

Leu Thr Thr Tyr Asp Gly Val His Asn His Val Thr Pro Gly Ser Thr
            180                 185                 190

Ser Ser Arg Ala Ala Pro Ala Tyr Ser Ala Pro Thr Ala Pro Ala Trp
        195                 200                 205

Thr Trp Ser Glu Leu His Ala Ala Ala His Ser Ser Glu Ser Tyr
    210                 215                 220

<210>  19
<211>  669
<212>  DNA
<213>  Triticum aestivum

<400>  19
atggcggctt cgctaggact tacccccagc catgaagcct actactcggc ccacccggcg      60

gcctcctcct tgtacaattt cgaagccggt gatcccttcg tggcagcggc ggacggtgcc     120

gcggagctga gcttcccgga gctcgtggag gccgctcgtg cctccgacta ctcgccgctt     180

ccggcgttcg gcgccgctgg aggcgagacg atgaacatgt acgggcggag cgtcgtcttc     240

cctatgacgg cctcgtacta ctgcgacggc gcgtggatgt tcgaggacga cgcggcggcg     300

agggcgagag gaggagggat cggtgctatg gcagggcggc cgtcggggag gatcgggttc     360

cggactcggt cggaggtgga ggtgatggac gacggattcc ggtggaggaa gtacggcaag     420

aaggcggtga agagcagccc caacctgcgc aactactacc ggtgctccgc cgaggggtgc     480

ggggtgaaga agcgggtcga gcgggaccgc gacgacccgc tctacgtcct caccacctac     540

gacggcgtcc acaaccacgt cacccccggc agcacgtcgt cgagggcggc gccggcgtac     600

tcggcgccga cggcgcccgc ttggacctgg agcgagctgc aagcggcggc gcactcctcg     660

gagtcctag                                                             669

<210>  20
<211>  222
<212>  PRT

<213>  Triticum aestivum

<400>  20

Met Ala Ala Ser Leu Gly Leu Thr Pro Ser His Glu Ala Tyr Tyr Ser
1               5                   10                  15

Ala His Pro Ala Ala Ser Ser Leu Tyr Asn Phe Glu Ala Gly Asp Pro
            20                  25                  30

Phe Val Ala Ala Ala Asp Gly Ala Ala Glu Leu Ser Phe Pro Glu Leu
        35                  40                  45

Val Glu Ala Ala Arg Ala Ser Asp Tyr Ser Pro Leu Pro Ala Phe Gly
    50                  55                  60

Ala Ala Gly Gly Glu Thr Met Asn Met Tyr Gly Arg Ser Val Val Phe
65                  70                  75                  80

Pro Met Thr Ala Ser Tyr Tyr Cys Asp Gly Ala Trp Met Phe Glu Asp
                85                  90                  95

Asp Ala Ala Ala Arg Ala Arg Gly Gly Gly Ile Gly Ala Met Ala Gly
            100                 105                 110

Arg Pro Ser Gly Arg Ile Gly Phe Arg Thr Arg Ser Glu Val Glu Val
        115                 120                 125

Met Asp Asp Gly Phe Arg Trp Arg Lys Tyr Gly Lys Lys Ala Val Lys
    130                 135                 140

Ser Ser Pro Asn Leu Arg Asn Tyr Tyr Arg Cys Ser Ala Glu Gly Cys
145                 150                 155                 160

Gly Val Lys Lys Arg Val Glu Arg Asp Arg Asp Asp Pro Leu Tyr Val
                165                 170                 175

Leu Thr Thr Tyr Asp Gly Val His Asn His Val Thr Pro Gly Ser Thr
            180                 185                 190

Ser Ser Arg Ala Ala Pro Ala Tyr Ser Ala Pro Thr Ala Pro Ala Trp
        195                 200                 205

Thr Trp Ser Glu Leu Gln Ala Ala Ala His Ser Ser Glu Ser
    210                 215                 220

<210>  21
<211>  672
<212>  DNA

<213>  Secale cereale

<400>  21
atggcgactt cgctgggact taccaccagc catgaagcct gcttctcggc ccacccggcg      60

gcctcctcct tgtacaactt tcaagctgat gatcacttcc tggcagcggc ggacggtgcc     120

gcggcgctga gcttcccgga gctcgtcgac gccgctcggg cctccaacta cccgccgctt     180

ccggcgttcg gcgccgctgg aggcgaggcg atgaacatgt acgagcggag tgtcgtcttc     240

cccatgacgt cctcgtacta ctgcgacggc gctgggatgt tcgacggcga cgcttcggcg     300

agggcgagag gaggagggat cggcgctatg gccgggcggc cgtcggggag gatcgggttc     360

cggacccggt cggaggtgga ggtgatggac gacgggttcc ggtggaggaa gtacggcaag     420

aaggcggtga agagcagccc caacctgcgg aactactacc ggtgctccgc cgaggggtgc     480

ggggtgaaga agcgggtcga gcgggaccgc gacgacccgc tctacgtgct caccacctac     540

gacggcgtcc acaaccacgt cacccccggc agcacgtcgt cgcgggcggc gccggcgtac     600

tcggcgccgg ccgcgcccgc ttggacctgg agcgagctgc atgcggcggc gcactcctcg     660

gagtcctact ga                                                         672


<210>  22
<211>  223
<212>  PRT
<213>  Secale cereale

<400>  22

Met Ala Thr Ser Leu Gly Leu Thr Thr Ser His Glu Ala Cys Phe Ser
1               5                   10                  15


Ala His Pro Ala Ala Ser Ser Leu Tyr Asn Phe Gln Ala Asp Asp His
            20                  25                  30


Phe Leu Ala Ala Ala Asp Gly Ala Ala Ala Leu Ser Phe Pro Glu Leu
        35                  40                  45


Val Asp Ala Ala Arg Ala Ser Asn Tyr Pro Pro Leu Pro Ala Phe Gly
    50                  55                  60


Ala Ala Gly Gly Glu Ala Met Asn Met Tyr Glu Arg Ser Val Val Phe
65                  70                  75                  80


Pro Met Thr Ser Ser Tyr Tyr Cys Asp Gly Ala Gly Met Phe Asp Gly
                85                  90                  95


Asp Ala Ser Ala Arg Ala Arg Gly Gly Gly Ile Gly Ala Met Ala Gly
            100                 105                 110

Arg Pro Ser Gly Arg Ile Gly Phe Arg Thr Arg Ser Glu Val Glu Val
        115                 120                 125

Met Asp Asp Gly Phe Arg Trp Arg Lys Tyr Gly Lys Lys Ala Val Lys
    130                 135                 140

Ser Ser Pro Asn Leu Arg Asn Tyr Tyr Arg Cys Ser Ala Glu Gly Cys
145                 150                 155                 160

Gly Val Lys Lys Arg Val Glu Arg Asp Arg Asp Asp Pro Leu Tyr Val
                165                 170                 175

Leu Thr Thr Tyr Asp Gly Val His Asn His Val Thr Pro Gly Ser Thr
            180                 185                 190

Ser Ser Arg Ala Ala Pro Ala Tyr Ser Ala Pro Ala Ala Pro Ala Trp
        195                 200                 205

Thr Trp Ser Glu Leu His Ala Ala Ala His Ser Ser Glu Ser Tyr
    210                 215                 220

<210>  23
<211>  690
<212>  DNA
<213>  Hordeum vulgare

<400>  23
atggcggcct cgctgggact taccagccat gaagaagcct gctactctgc ccacccggcc      60

ggctcctccc tgtacaactt ccaagacggc gaacccttcg gggcggcggc cgacggtgcc     120

gcggcgccga gcttccagga gctcgtggac gccgctcgac cctccgacta cgcgccgctt     180

ccggcgttcg gcgccgctgg aggcgaggcg atgagcatgt acgagcggag cgtcgtcttc     240

cctatgacga cgtcctcgta ctactgcgac ggcgccggga tgttcgacga cgacgcggcg     300

gcgagggcga gagggggagg ggtcggtgct atggcagggc ggccgtcggg gaggatcggg     360

ttccggaccc ggtcggaggt ggaggtgatg gacgacgggt tccggtggag gaagtacggc     420

aagaaggcgg tgaagagcag ccccaacctg cggaactact accgctgctc cgccgacggg     480

tgcggggtga agaagcgggt cgagcgggac cgcgacgacc cgcgctacgt cctcaccacc     540

tacgacggcg tccacaacca cgtcgccccc ggcggcggca cgccgtcgcg ggcggcgccg     600

gcgtactcgg cgccggccgc gccagcttgg acctggagcg agctgcatgc ggcggcggcg     660

gcggcggctc actcctcgga gtcctactga                                      690

<210>  24
<211>  229
<212>  PRT
<213>  Hordeum vulgare

<400> 24

Met Ala Ala Ser Leu Gly Leu Thr Ser His Glu Glu Ala Cys Tyr Ser
1               5                   10                  15

Ala His Pro Ala Gly Ser Ser Leu Tyr Asn Phe Gln Asp Gly Glu Pro
            20                  25                  30

Phe Gly Ala Ala Ala Asp Gly Ala Ala Ala Pro Ser Phe Gln Glu Leu
        35                  40                  45

Val Asp Ala Ala Arg Pro Ser Asp Tyr Ala Pro Leu Pro Ala Phe Gly
    50                  55                  60

Ala Ala Gly Gly Glu Ala Met Ser Met Tyr Glu Arg Ser Val Val Phe
65                  70                  75                  80

Pro Met Thr Thr Ser Ser Tyr Tyr Cys Asp Gly Ala Gly Met Phe Asp
                85                  90                  95

Asp Asp Ala Ala Ala Arg Ala Arg Gly Gly Gly Val Gly Ala Met Ala
            100                 105                 110

Gly Arg Pro Ser Gly Arg Ile Gly Phe Arg Thr Arg Ser Glu Val Glu
        115                 120                 125

Val Met Asp Asp Gly Phe Arg Trp Arg Lys Tyr Gly Lys Lys Ala Val
    130                 135                 140

Lys Ser Ser Pro Asn Leu Arg Asn Tyr Tyr Arg Cys Ser Ala Asp Gly
145                 150                 155                 160

Cys Gly Val Lys Lys Arg Val Glu Arg Asp Arg Asp Asp Pro Arg Tyr
                165                 170                 175

Val Leu Thr Thr Tyr Asp Gly Val His Asn His Val Ala Pro Gly Gly
            180                 185                 190

Gly Thr Pro Ser Arg Ala Ala Pro Ala Tyr Ser Ala Pro Ala Ala Pro
        195                 200                 205

Ala Trp Thr Trp Ser Glu Leu His Ala Ala Ala Ala Ala Ala Ala His
    210                 215                 220

Ser Ser Glu Ser Tyr
225

<210>  25
<211>  669
<212>  DNA
<213>  Triticum aestivum

<400>  25
atggccctgg actccgtccc ttcctacccc agcgacctgg gatccagcgg cagggcaacc      60

aggacccagc aaagaatccg gaaggaggag cgaacgtgga cggcggacac ctacgcgccg     120

tacgacgacg ggcaccagtg gaggaagtac ggcgagaaga agctctccaa ctcctacttc     180

ccgaggttct actacagatg tacctacaag actgacctga agtgccctgc gacaaaacaa     240

gtccaacaga aggacatgag cgacccacca ttgttcacgg tcacttactt caaccatcac     300

agctgcaaca ccacctcgag gcccatcggc agcgccccag acaccactga gcagtcgtcc     360

tcgaggaggg cagtgtccat ctgcttcggg tcgcatgcca ccggcgagca gccgacattc     420

ctaacgtcgc cgggcacact gcagtcacca gcgagcacaa ccaaccaaca gaacgacaga     480

ggcgcttacg gtcaccagtt tcagtggaca gacacatcac cgtcggcagg cgatgcccca     540

gtcaagatgg agaccgacag tctcgccgga acaggcgctt cgtctggcgc tgctagcggc     600

catgctctgt cgaggacact gctgccgatc ggccagtcga gatgcatcga gtactttcag     660

ttcttgtga                                                             669

<210>  26
<211>  222
<212>  PRT
<213>  Triticum aestivum

<400>  26

Met Ala Leu Asp Ser Val Pro Ser Tyr Pro Ser Asp Leu Gly Ser Ser
1               5                   10                  15

Gly Arg Ala Thr Arg Thr Gln Gln Arg Ile Arg Lys Glu Glu Arg Thr
            20                  25                  30

Trp Thr Ala Asp Thr Tyr Ala Pro Tyr Asp Asp Gly His Gln Trp Arg
        35                  40                  45

Lys Tyr Gly Glu Lys Lys Leu Ser Asn Ser Tyr Phe Pro Arg Phe Tyr
    50                  55                  60

Tyr Arg Cys Thr Tyr Lys Thr Asp Leu Lys Cys Pro Ala Thr Lys Gln
65                  70                  75                  80

Val Gln Gln Lys Asp Met Ser Asp Pro Pro Leu Phe Thr Val Thr Tyr
                85                  90                  95

Phe Asn His His Ser Cys Asn Thr Thr Ser Arg Pro Ile Gly Ser Ala

100                 105                 110

Pro Asp Thr Thr Glu Gln Ser Ser Ser Arg Arg Ala Val Ser Ile Cys
        115                 120                 125

Phe Gly Ser His Ala Thr Gly Glu Gln Pro Thr Phe Leu Thr Ser Pro
    130                 135                 140

Gly Thr Leu Gln Ser Pro Ala Ser Thr Thr Asn Gln Gln Asn Asp Arg
145                 150                 155                 160

Gly Ala Tyr Gly His Gln Phe Gln Trp Thr Asp Thr Ser Pro Ser Ala
                165                 170                 175

Gly Asp Ala Pro Val Lys Met Glu Thr Asp Ser Leu Ala Gly Thr Gly
            180                 185                 190

Ala Ser Ser Gly Ala Ala Ser Gly His Ala Leu Ser Arg Thr Leu Leu
        195                 200                 205

Pro Ile Gly Gln Ser Arg Cys Ile Glu Tyr Phe Gln Phe Leu
    210                 215                 220

<210>  27
<211>  591
<212>  DNA
<213>  Secale cereale

<400>  27
aggaaggagg agcggacgtg gacggcggac acctacgcgc cgtacgacga cgggcaccag      60

tggaggaagt acggcgagaa gaagctctcc aactccaact tcccgaggtt ctactacaga     120

tgtacgtaca agactgacct gaagtgccct gcgacaaaac aagtccagca gaaggacatg     180

agcgacccac cattgttcac ggtcacttac ttcaaccatc acagctgcaa caccacctcg     240

aggcccatcg gcagcgcccc agacaccacc gagcagtcct cctcgaggag ggcagtgtcc     300

atctgcttcg ggtcgcatgc cactggcgag cagccgacat tcctaacgtc gccgggcaca     360

ctgcagtcac cagcgagcac aaccaaccaa cagagcgaca gaggcgctta cggtcaccag     420

tttcagtgga cagacacatc accgtccgca ggcgatgccc cagtcaagat ggagaccgac     480

agtctcgccg gaacaggctc gtcgtctggc gctgctagcg gccatgctct gtcgaggaca     540

cttctgccga tcggccagtc gagatgcatc gagtactttc agttcttgtg a              591

<210>  28
<211>  196
<212>  PRT
<213>  Secale cereale

<400>  28

Arg Lys Glu Glu Arg Thr Trp Thr Ala Asp Thr Tyr Ala Pro Tyr Asp
1               5                   10                  15

Asp Gly His Gln Trp Arg Lys Tyr Gly Glu Lys Lys Leu Ser Asn Ser
            20                  25                  30

Asn Phe Pro Arg Phe Tyr Tyr Arg Cys Thr Tyr Lys Thr Asp Leu Lys
        35                  40                  45

Cys Pro Ala Thr Lys Gln Val Gln Gln Lys Asp Met Ser Asp Pro Pro
    50                  55                  60

Leu Phe Thr Val Thr Tyr Phe Asn His His Ser Cys Asn Thr Thr Ser
65                  70                  75                  80

Arg Pro Ile Gly Ser Ala Pro Asp Thr Thr Glu Gln Ser Ser Ser Arg
                85                  90                  95

Arg Ala Val Ser Ile Cys Phe Gly Ser His Ala Thr Gly Glu Gln Pro
            100                 105                 110

Thr Phe Leu Thr Ser Pro Gly Thr Leu Gln Ser Pro Ala Ser Thr Thr
        115                 120                 125

Asn Gln Gln Ser Asp Arg Gly Ala Tyr Gly His Gln Phe Gln Trp Thr
    130                 135                 140

Asp Thr Ser Pro Ser Ala Gly Asp Ala Pro Val Lys Met Glu Thr Asp
145                 150                 155                 160

Ser Leu Ala Gly Thr Gly Ser Ser Ser Gly Ala Ala Ser Gly His Ala
                165                 170                 175

Leu Ser Arg Thr Leu Leu Pro Ile Gly Gln Ser Arg Cys Ile Glu Tyr
            180                 185                 190

Phe Gln Phe Leu
        195


<210>  29
<211>  672
<212>  DNA
<213>  Hordeum vulgare

<400>  29
atggccctgg actccgtccc gtcctacccc agcgacctgg gatccagcag cagtaggaca      60

accaggaccc agcaaagaat ccggaaggag gagcggacgt ggacggcgga cacctacgca     120

ccgtacgacg acgggcacca gtggaggaag tacggcgaga agaagctctc caactccaac 180

ttcccgaggt tctactacag atgtacctac aagactgacc tgaagtgccc tgcgacaaaa 240

caagtccagc agaaggacat gagcgaccca ccattgttca cagtcactta cttcaaccat 300

cacagctgca acaccacctc gaggcccatc ggcagcgccc cagacaccac cgagcagtcg 360

tcctcgagga gggcggtgtc catctgcttc gggtcgcatg ccaccggcga gcagccaacg 420

ttcttaacgt cgccgggtac actacagtca ccagcaagca caaccaacca acagagcgac 480

ataggcgctt acggtcacca gtttcagtgg acagatacat caccgtcggc agacgatgcc 540

ccggtcaaga tggagaccga cagtctcgca ggaacaggtg cttcgtctgg cggtgctagc 600

ggccatgctc tgtcgaggac actgctgccg atcggccagt cgagatgcat cgagtacttc 660

cacttcttgt ga 672

<210> 30
<211> 223
<212> PRT
<213> Hordeum vulgare

<400> 30

Met Ala Leu Asp Ser Val Pro Ser Tyr Pro Ser Asp Leu Gly Ser Ser
1               5                   10                  15

Ser Ser Arg Thr Thr Arg Thr Gln Gln Arg Ile Arg Lys Glu Glu Arg
            20                  25                  30

Thr Trp Thr Ala Asp Thr Tyr Ala Pro Tyr Asp Asp Gly His Gln Trp
        35                  40                  45

Arg Lys Tyr Gly Glu Lys Lys Leu Ser Asn Ser Asn Phe Pro Arg Phe
    50                  55                  60

Tyr Tyr Arg Cys Thr Tyr Lys Thr Asp Leu Lys Cys Pro Ala Thr Lys
65                  70                  75                  80

Gln Val Gln Gln Lys Asp Met Ser Asp Pro Pro Leu Phe Thr Val Thr
                85                  90                  95

Tyr Phe Asn His His Ser Cys Asn Thr Thr Ser Arg Pro Ile Gly Ser
            100                 105                 110

Ala Pro Asp Thr Thr Glu Gln Ser Ser Ser Arg Arg Ala Val Ser Ile
        115                 120                 125

Cys Phe Gly Ser His Ala Thr Gly Glu Gln Pro Thr Phe Leu Thr Ser
    130                 135                 140

Pro Gly Thr Leu Gln Ser Pro Ala Ser Thr Thr Asn Gln Gln Ser Asp
145                 150                 155                 160

Ile Gly Ala Tyr Gly His Gln Phe Gln Trp Thr Asp Thr Ser Pro Ser
                165                 170                 175

Ala Asp Asp Ala Pro Val Lys Met Glu Thr Asp Ser Leu Ala Gly Thr
            180                 185                 190

Gly Ala Ser Ser Gly Gly Ala Ser Gly His Ala Leu Ser Arg Thr Leu
        195                 200                 205

Leu Pro Ile Gly Gln Ser Arg Cys Ile Glu Tyr Phe His Phe Leu
    210                 215                 220

<210>  31
<211>  1293
<212>  DNA
<213>  Triticum aestivum

<400>  31
ccgccgctgg ccgcgtcggc gctcggcttc ttcgggggac ccctatccct cgcgcagctc      60

ccttcccctc cgccgctcct cgggtacccc caggacaact ttgatgtgtt ccatgaacaa     120

gacctagcac agctggcagc acaagtagct caaaagaaag agttgcggga aaaacaaggg     180

gcgggattgc atcacaagat tggacctcaa ctagcttttt cgaaatacag tatacttgat     240

caagtggaca actcttcttc tttctcattg gcaacttcag tgctgacacc tcagcatgtc     300

agttcttccg taggcgcggc atcaatgcag ggacagactt tgccatcaca cactggtagt     360

ggtagtgtca acactggacc aactggagtt ttacaagtgc tccaagattc atccaccact     420

ctggacagta tcaacactgg atcaactgga gttctggaag cactccaagg ttcatccatc     480

actctggata gacctgctga tgatggatac aactggcgta agtatggaca aaaggcagtc     540

aagggtggga agtatccaaa gagctattac aaatgcaccc tgaactgccc ggtcaggaaa     600

aatgtagagc actctgcaga tggacgaatt attaaaataa tttatagagg tcagcactgc     660

catgaacccc cctcaaagag gtttaaagat tgtggtgatt tattgaatga gttaaatgat     720

ttcaatgatg ccaaggagcc ttcaactaaa tcacaattag gttgtcaagg ttattatgga     780

aaacctataa cgccaaatgg aatgatgacg gatgttttat tgccaacgaa ggaagagggg     840

gatgagcaat tatctagttt aagtgatatc cgggaaggtg atggtgaaat aagaactgtt     900

gatggagatg atggtgatgc cgatgcaaat gaaaggaatg caccaggtca aaagatcatc     960

gtgagtacaa cgagcgatgt tgatcttttg gacgacggct ataggtggcg caagtatgga    1020

cagaaagtgg tgagaggaaa tcctcaccca aggagctatt acaagtgcac ttaccaaggc    1080

tgcgacgtca agaagcatat cgagagatct tccgaggaac cacatgctgt gataaccaca 1140

tacgaaggga agcataccca tgacgtgcct gagtctggaa cagaagccaa ggcacaggtc 1200

aacaccactg caaagagcag acttattcag aacaaccagc tgctagcttc tgcagtagct 1260

cggaaaagag aaaatacgga acagccattc tga 1293

<210> 32
<211> 430
<212> PRT
<213> Triticum aestivum

<400> 32

Pro Pro Leu Ala Ala Ser Ala Leu Gly Phe Phe Gly Gly Pro Leu Ser
1               5                   10                  15

Leu Ala Gln Leu Pro Ser Pro Pro Pro Leu Leu Gly Tyr Pro Gln Asp
            20                  25                  30

Asn Phe Asp Val Phe His Glu Gln Asp Leu Ala Gln Leu Ala Ala Gln
        35                  40                  45

Val Ala Gln Lys Lys Glu Leu Arg Glu Lys Gln Gly Ala Gly Leu His
    50                  55                  60

His Lys Ile Gly Pro Gln Leu Ala Phe Ser Lys Tyr Ser Ile Leu Asp
65                  70                  75                  80

Gln Val Asp Asn Ser Ser Ser Phe Ser Leu Ala Thr Ser Val Leu Thr
                85                  90                  95

Pro Gln His Val Ser Ser Ser Val Gly Ala Ala Ser Met Gln Gly Gln
            100                 105                 110

Thr Leu Pro Ser His Thr Gly Ser Gly Ser Val Asn Thr Gly Pro Thr
        115                 120                 125

Gly Val Leu Gln Val Leu Gln Asp Ser Ser Thr Thr Leu Asp Ser Ile
    130                 135                 140

Asn Thr Gly Ser Thr Gly Val Leu Glu Ala Leu Gln Gly Ser Ser Ile
145                 150                 155                 160

Thr Leu Asp Arg Pro Ala Asp Asp Gly Tyr Asn Trp Arg Lys Tyr Gly
                165                 170                 175

Gln Lys Ala Val Lys Gly Gly Lys Tyr Pro Lys Ser Tyr Tyr Lys Cys
            180                 185                 190

Thr Leu Asn Cys Pro Val Arg Lys Asn Val Glu His Ser Ala Asp Gly
        195                 200                 205

Arg Ile Ile Lys Ile Ile Tyr Arg Gly Gln His Cys His Glu Pro Pro
    210                 215                 220

Ser Lys Arg Phe Lys Asp Cys Gly Asp Leu Leu Asn Glu Leu Asn Asp
225                 230                 235                 240

Phe Asn Asp Ala Lys Glu Pro Ser Thr Lys Ser Gln Leu Gly Cys Gln
                245                 250                 255

Gly Tyr Tyr Gly Lys Pro Ile Thr Pro Asn Gly Met Met Thr Asp Val
            260                 265                 270

Leu Leu Pro Thr Lys Glu Glu Gly Asp Glu Gln Leu Ser Ser Leu Ser
        275                 280                 285

Asp Ile Arg Glu Gly Asp Gly Glu Ile Arg Thr Val Asp Gly Asp Asp
    290                 295                 300

Gly Asp Ala Asp Ala Asn Glu Arg Asn Ala Pro Gly Gln Lys Ile Ile
305                 310                 315                 320

Val Ser Thr Thr Ser Asp Val Asp Leu Leu Asp Asp Gly Tyr Arg Trp
                325                 330                 335

Arg Lys Tyr Gly Gln Lys Val Val Arg Gly Asn Pro His Pro Arg Ser
            340                 345                 350

Tyr Tyr Lys Cys Thr Tyr Gln Gly Cys Asp Val Lys Lys His Ile Glu
        355                 360                 365

Arg Ser Ser Glu Glu Pro His Ala Val Ile Thr Thr Tyr Glu Gly Lys
    370                 375                 380

His Thr His Asp Val Pro Glu Ser Gly Thr Glu Ala Lys Ala Gln Val
385                 390                 395                 400

Asn Thr Thr Ala Lys Ser Arg Leu Ile Gln Asn Asn Gln Leu Leu Ala
                405                 410                 415

Ser Ala Val Ala Arg Lys Arg Glu Asn Thr Glu Gln Pro Phe
            420                 425                 430

<210>  33

<211>  1218
<212>  DNA
<213>  Secale cereale

<400>  33
gacaattttg atgtgttcca tgaacaagac ctagcacagc tggcagcaca agtagctcaa      60

aagaaagagt tgcgggaaag acaaggggcg ggattgcttc acaagattgg acctcaacta     120

gctttttcaa aatacagtat acttgatcaa gtgggcaact cttcttcttt ctcattggca     180

acttcagtgc taacacctca gcatgttagt tcttccgtag gcgcgtcatc aatgcaggga     240

cagactttgc catcacacac tggtagtggt agtgtcaaca ctgaaccaac tggagtttta     300

caagtgctcc aagattcatc caccactctg gacagtatca acactggatc agctggagtt     360

ctggaagcac tccaaggtgc atccatcact ctggatagac ctgctgatga tggatacact     420

tggcgtaagt atggacaaaa ggcagtcaag ggtgggaagt atccaaagag ctattacaaa     480

tgcaccctga actgcccggt caggaaaaat gtagagcact ctgcagatgg acgaattatt     540

aaaataattt atagaggtca gcactgccat gaacctccct caaagaggtt taaagattgt     600

ggtgatttat tgaatgagtt agatgatttc aatgatgcca aggagccttc aactggatca     660

caattaggtt gtcaaggtta ttatggaaaa cctataacgc caaatggcac gatggcggat     720

ggtttattgc caacgaagga agagggagat gagcaattat ctagtttaag tgatatccgg     780

gaaggtgatg atgaaataag aactgttgat ggagatgttg gtgatgccga tgcaaatgaa     840

aggaatgcac caggtcaaaa gataatcgtg agtacaacga gcgatgttga tcttttggac     900

gacggctata ggtggcgcaa gtatggacag aaagtggtga gaggaaatcc tcacccaagg     960

agctattaca agtgcactta ccaaggctgc gacgtcaaga agcatatcga gagatcttcc    1020

gaggaaccac atgctgtgat aactacatac gaagggaagc atacccatga cgtgcctgag    1080

tctaggacca gaagccaagc cacaggtcaa caccactgca aagagcagac ttattcagaa    1140

caaccagctg ctagcttctg cagtagctcg gaaaagagaa aatacggaac agccattctg    1200

aacgatctcg ccttctag                                                  1218


<210>  34
<211>  405
<212>  PRT
<213>  Secale cereale

<400>  34

Asp Asn Phe Asp Val Phe His Glu Gln Asp Leu Ala Gln Leu Ala Ala
1               5                   10                  15


Gln Val Ala Gln Lys Lys Glu Leu Arg Glu Arg Gln Gly Ala Gly Leu
            20                  25                  30

Leu His Lys Ile Gly Pro Gln Leu Ala Phe Ser Lys Tyr Ser Ile Leu
        35                  40                  45

Asp Gln Val Gly Asn Ser Ser Ser Phe Ser Leu Ala Thr Ser Val Leu
    50                  55                  60

Thr Pro Gln His Val Ser Ser Ser Val Gly Ala Ser Ser Met Gln Gly
65                  70                  75                  80

Gln Thr Leu Pro Ser His Thr Gly Ser Gly Ser Val Asn Thr Glu Pro
                85                  90                  95

Thr Gly Val Leu Gln Val Leu Gln Asp Ser Ser Thr Thr Leu Asp Ser
            100                 105                 110

Ile Asn Thr Gly Ser Ala Gly Val Leu Glu Ala Leu Gln Gly Ala Ser
        115                 120                 125

Ile Thr Leu Asp Arg Pro Ala Asp Asp Gly Tyr Thr Trp Arg Lys Tyr
    130                 135                 140

Gly Gln Lys Ala Val Lys Gly Gly Lys Tyr Pro Lys Ser Tyr Tyr Lys
145                 150                 155                 160

Cys Thr Leu Asn Cys Pro Val Arg Lys Asn Val Glu His Ser Ala Asp
                165                 170                 175

Gly Arg Ile Ile Lys Ile Ile Tyr Arg Gly Gln His Cys His Glu Pro
            180                 185                 190

Pro Ser Lys Arg Phe Lys Asp Cys Gly Asp Leu Leu Asn Glu Leu Asp
        195                 200                 205

Asp Phe Asn Asp Ala Lys Glu Pro Ser Thr Gly Ser Gln Leu Gly Cys
    210                 215                 220

Gln Gly Tyr Tyr Gly Lys Pro Ile Thr Pro Asn Gly Thr Met Ala Asp
225                 230                 235                 240

Gly Leu Leu Pro Thr Lys Glu Glu Gly Asp Glu Gln Leu Ser Ser Leu
                245                 250                 255

Ser Asp Ile Arg Glu Gly Asp Asp Glu Ile Arg Thr Val Asp Gly Asp
            260                 265                 270

Val Gly Asp Ala Asp Ala Asn Glu Arg Asn Ala Pro Gly Gln Lys Ile
        275                 280                 285

Ile Val Ser Thr Thr Ser Asp Val Asp Leu Leu Asp Asp Gly Tyr Arg
    290                 295                 300

Trp Arg Lys Tyr Gly Gln Lys Val Val Arg Gly Asn Pro His Pro Arg
305                 310                 315                 320

Ser Tyr Tyr Lys Cys Thr Tyr Gln Gly Cys Asp Val Lys Lys His Ile
                325                 330                 335

Glu Arg Ser Ser Glu Glu Pro His Ala Val Ile Thr Thr Tyr Glu Gly
            340                 345                 350

Lys His Thr His Asp Val Pro Glu Ser Arg Thr Arg Ser Gln Ala Thr
        355                 360                 365

Gly Gln His His Cys Lys Glu Gln Thr Tyr Ser Glu Gln Pro Ala Ala
    370                 375                 380

Ser Phe Cys Ser Ser Ser Glu Lys Arg Lys Tyr Gly Thr Ala Ile Leu
385                 390                 395                 400

Asn Asp Leu Ala Phe
                405

<210>  35
<211>  1341
<212>  DNA
<213>  Hordeum vulgare

<400>  35
atggcggccg gccagtggtc ggccatcggc gacggcggcg gcctctggtc cccgcaggcg      60

ctcgagagcc tcttccccga cgagcagccg gcggcggcgc agctgggctt cttcgggggg     120

tccctcgcgc agctcgctcc ccctccgccg ctctgcgcca tcgcgctcct cgggtacccc     180

caggataact ttgatgtgtt caatgaacaa gacctagcac aggtggcagc acaggtagct     240

caaaagaagg agttgcagga aaaacaagcg gcgttattac atccgaaggg tatgtcgact     300

ttgccatcac atactggtag tggtagtatg aacactggac caactggaat tctacaagtg     360

ctccaaggtt catccaccac tctggacagt atcaacactg gatcagctgg agttctacaa     420

gtgctccaag gttcatccac cactctggac agtatcaaca ctggatcagc tggatttctg     480

gaagcactcc aaggttcttc catcactctg gataaacctg ctgatgatgg ctacaattgg     540

cgtaaatatg gacagaaggc agtcaagggc gggaagtgtc caaggagcta ttacaaatgt     600

accctgaact gcccggtcag aaaaaatgtg gagcactctg cagatggacg aattattaaa     660

atagtttata gaggtcagca ctgtcatgaa cctccctcaa agaggtttaa agattgtggt     720

gatttattga acgagttaga tgaactcaat gatgccgagg aaccttcaac cagatcacta 780

ttaggttgtc aaggttatta tggaaaacca aaacctataa cgccaaatgg cacgatggtg 840

gatggtttat tgccaacgaa ggaagaggga gatgagcaat tatctagttt aagtgatatc 900

cgggaagatg atggtgaaat aagaactgtt gatggagatg ttggtgatgc cgatgcaaat 960

gaaaggaatg caccaggtca aaaaattatt gtgagtacaa caagcgatgt tgatcttttg 1020

gacgatggct ataggtggcg caagtatgga cagaaagtgg tgagaggaaa tcctcaccca 1080

aggagctatt acaagtgcac ttaccaaggc tgcgacgtca agaagcatgt cgagagatct 1140

tcccaggaac cacatgctgt gataactaca tacgaaggga agcataccca tgacgtgcct 1200

gagtctagga acagaagcca ggccacaggt tcacaccact gcaaagagca gacttattca 1260

gaacaaccag ctgctagctt ctgcagtagc tcggaaaaaa gaaaatacgg aacggtcatt 1320

ctgaacgatc tcgctttcta g 1341

<210> 36
<211> 446
<212> PRT
<213> Hordeum vulgare

<400> 36

Met Ala Ala Gly Gln Trp Ser Ala Ile Gly Asp Gly Gly Gly Leu Trp
1               5                   10                  15

Ser Pro Gln Ala Leu Glu Ser Leu Phe Pro Asp Glu Gln Pro Ala Ala
            20                  25                  30

Ala Gln Leu Gly Phe Phe Gly Gly Ser Leu Ala Gln Leu Ala Pro Pro
        35                  40                  45

Pro Pro Leu Cys Ala Ile Ala Leu Leu Gly Tyr Pro Gln Asp Asn Phe
    50                  55                  60

Asp Val Phe Asn Glu Gln Asp Leu Ala Gln Val Ala Ala Gln Val Ala
65                  70                  75                  80

Gln Lys Lys Glu Leu Gln Glu Lys Gln Ala Ala Leu Leu His Pro Lys
                85                  90                  95

Gly Met Ser Thr Leu Pro Ser His Thr Gly Ser Gly Ser Met Asn Thr
            100                 105                 110

Gly Pro Thr Gly Ile Leu Gln Val Leu Gln Gly Ser Ser Thr Thr Leu
        115                 120                 125

Asp Ser Ile Asn Thr Gly Ser Ala Gly Val Leu Gln Val Leu Gln Gly

130                 135                 140

Ser Ser Thr Thr Leu Asp Ser Ile Asn Thr Gly Ser Ala Gly Phe Leu
145                 150                 155                 160

Glu Ala Leu Gln Gly Ser Ser Ile Thr Leu Asp Lys Pro Ala Asp Asp
                165                 170                 175

Gly Tyr Asn Trp Arg Lys Tyr Gly Gln Lys Ala Val Lys Gly Gly Lys
            180                 185                 190

Cys Pro Arg Ser Tyr Tyr Lys Cys Thr Leu Asn Cys Pro Val Arg Lys
        195                 200                 205

Asn Val Glu His Ser Ala Asp Gly Arg Ile Ile Lys Ile Val Tyr Arg
    210                 215                 220

Gly Gln His Cys His Glu Pro Pro Ser Lys Arg Phe Lys Asp Cys Gly
225                 230                 235                 240

Asp Leu Leu Asn Glu Leu Asp Glu Leu Asn Asp Ala Glu Glu Pro Ser
                245                 250                 255

Thr Arg Ser Leu Leu Gly Cys Gln Gly Tyr Tyr Gly Lys Pro Lys Pro
            260                 265                 270

Ile Thr Pro Asn Gly Thr Met Val Asp Gly Leu Leu Pro Thr Lys Glu
        275                 280                 285

Glu Gly Asp Glu Gln Leu Ser Ser Leu Ser Asp Ile Arg Glu Asp Asp
    290                 295                 300

Gly Glu Ile Arg Thr Val Asp Gly Asp Val Gly Asp Ala Asp Ala Asn
305                 310                 315                 320

Glu Arg Asn Ala Pro Gly Gln Lys Ile Ile Val Ser Thr Thr Ser Asp
                325                 330                 335

Val Asp Leu Leu Asp Asp Gly Tyr Arg Trp Arg Lys Tyr Gly Gln Lys
            340                 345                 350

Val Val Arg Gly Asn Pro His Pro Arg Ser Tyr Tyr Lys Cys Thr Tyr
        355                 360                 365

Gln Gly Cys Asp Val Lys Lys His Val Glu Arg Ser Ser Gln Glu Pro
    370                 375                 380

His Ala Val Ile Thr Thr Tyr Glu Gly Lys His Thr His Asp Val Pro
385                 390                 395                 400

Glu Ser Arg Asn Arg Ser Gln Ala Thr Gly Ser His His Cys Lys Glu
                405                 410                 415

Gln Thr Tyr Ser Glu Gln Pro Ala Ala Ser Phe Cys Ser Ser Ser Glu
            420                 425                 430

Lys Arg Lys Tyr Gly Thr Val Ile Leu Asn Asp Leu Ala Phe
        435                 440                 445

<210>  37
<211>  1785
<212>  DNA
<213>  Secale cereale

<400>  37
ctctccctcc cctctcggct agcaagcgtc tcttctccca ggaggctctc gatttgcatg      60

gacaagggcc accacggtgg tggcggagga ggaggaggac tgctcgccct ggacgcctcg     120

ccgcggcagc tcggcttctt gaacttgcta tcgccggcgc cgttccacag gagcatggag     180

gcggacgacg gcggtggcgg tggcggcggc agggggagga ggtccatcga ggttgacttc     240

ttctccgacg agaagaagaa catgaagaag agccgggcat cggccggtgc cgacgcggag     300

gaccagaaag atcaggcatc cgccgccgga ctcgccatca agaaggagga cctcaccatt     360

aacctccttc ccgggaacaa cacgaggagc gaccggtcca tggtggtcga cgacgacggg     420

gcgtcccggg cagacgacgg caggaacacc ggcgagctcg cggcgatgca ggccgagctg     480

agccgcatga acgaggagaa ccagcggctg cgagggatgt tgacccaggt caacagcagc     540

taccatgcgc tgcagatgca tctcgtcgcg ctcatgcagc aaaggaccca gatgcctccg     600

gtccaaccgc aacaacctcc gacccacgag gatggcaaga acgagagtgc catcgtgccg     660

aggcagttcc tgggtctggg cccatccgga gccagcgctg acgtggcgga ggagccgtcc     720

aactcgtcca cggaggtcgg gagcccgcgc cggtcgtcgt ccaacgggaa cgaggaccct     780

gagcgcggcg acaaccctga cggcccgtcc accgcggggt ggttgcccgg acgcgggatg     840

acccagcagc agcagcagca gcagcagctg ggagcggcgg cgaagggcca cgaccagcaa     900

gcgcaggagg ccaccatgag gaaggcccgt gtctccgttc gcgcacgatc cgaagcaccg     960

attatcgccg atggatgcca atggaggaag tacggccaga agatggccaa gggaaaccct    1020

tgcccacgcg cctactaccg ctgcaccatg gccaccggct gcccggtgcg caagcaggtg    1080

caacgatgcg ctgaggaccg gactatcctc atcaccacgt acgagggcac gcacaaccac    1140

ccgctcccgc cggccgccat ggccatggcg tctaccacgt cggccgccgc gtccatgctg    1200

ctctcgggct ccatgcccag cgccgacggc gcggggctca tgagctccaa cttcctcgcg    1260

cgcaccgtgc tgccgtgctc gtccagcatg gccaccatct cggcgtccgc gccgttcccg 1320

acggtcacgc tcgacctcac ccacgccccg cccggggcgc ccaacgccat gccgctgaac 1380

gtcgctcgac cgcatgcacc ggggcagttc catgttccga tgcccggcgg tgggatggcc 1440

ccggccttcg cgatgccgcc gcacatgctg tacaaccagt caaagttctc tggtctgcag 1500

atgtcctccg actcggtgga ccccgggcaa ttcgcgcagc ctaggccgca gatgggcctg 1560

ccaggccagc tgtccgacac cgtcagcgca gcggcggctg cgatcaccgc ggacccgaac 1620

ttcaccgtgg cacttgccgc ggccatctcg tcgatcatgg ccggccagca cgccgcgggc 1680

aacagcaacg cgaacaacag caacaacaac accaccagta acaacaacgt gacgacgacc 1740

agtaacaaca cgacgagcaa caacaccaac agcgagactc aatga 1785

<210> 38
<211> 594
<212> PRT
<213> Secale cereale

<400> 38

Leu Ser Leu Pro Ser Arg Leu Ala Ser Val Ser Ser Pro Arg Arg Leu
1               5                   10                  15

Ser Ile Cys Met Asp Lys Gly His His Gly Gly Gly Gly Gly Gly Gly
            20                  25                  30

Gly Leu Leu Ala Leu Asp Ala Ser Pro Arg Gln Leu Gly Phe Leu Asn
        35                  40                  45

Leu Leu Ser Pro Ala Pro Phe His Arg Ser Met Glu Ala Asp Asp Gly
    50                  55                  60

Gly Gly Gly Gly Gly Gly Arg Gly Arg Arg Ser Ile Glu Val Asp Phe
65                  70                  75                  80

Phe Ser Asp Glu Lys Lys Asn Met Lys Lys Ser Arg Ala Ser Ala Gly
                85                  90                  95

Ala Asp Ala Glu Asp Gln Lys Asp Gln Ala Ser Ala Ala Gly Leu Ala
            100                 105                 110

Ile Lys Lys Glu Asp Leu Thr Ile Asn Leu Leu Pro Gly Asn Asn Thr
        115                 120                 125

Arg Ser Asp Arg Ser Met Val Val Asp Asp Asp Gly Ala Ser Arg Ala
    130                 135                 140

Asp Asp Gly Arg Asn Thr Gly Glu Leu Ala Ala Met Gln Ala Glu Leu
145                 150                 155                 160

Ser Arg Met Asn Glu Glu Asn Gln Arg Leu Arg Gly Met Leu Thr Gln
                165                 170                 175

Val Asn Ser Ser Tyr His Ala Leu Gln Met His Leu Val Ala Leu Met
            180                 185                 190

Gln Gln Arg Thr Gln Met Pro Pro Val Gln Pro Gln Gln Pro Pro Thr
        195                 200                 205

His Glu Asp Gly Lys Asn Glu Ser Ala Ile Val Pro Arg Gln Phe Leu
    210                 215                 220

Gly Leu Gly Pro Ser Gly Ala Ser Ala Asp Val Ala Glu Glu Pro Ser
225                 230                 235                 240

Asn Ser Ser Thr Glu Val Gly Ser Pro Arg Arg Ser Ser Ser Asn Gly
                245                 250                 255

Asn Glu Asp Pro Glu Arg Gly Asp Asn Pro Asp Gly Pro Ser Thr Ala
            260                 265                 270

Gly Trp Leu Pro Gly Arg Gly Met Thr Gln Gln Gln Gln Gln Gln Gln
        275                 280                 285

Gln Leu Gly Ala Ala Ala Lys Gly His Asp Gln Gln Ala Gln Glu Ala
    290                 295                 300

Thr Met Arg Lys Ala Arg Val Ser Val Arg Ala Arg Ser Glu Ala Pro
305                 310                 315                 320

Ile Ile Ala Asp Gly Cys Gln Trp Arg Lys Tyr Gly Gln Lys Met Ala
                325                 330                 335

Lys Gly Asn Pro Cys Pro Arg Ala Tyr Tyr Arg Cys Thr Met Ala Thr
            340                 345                 350

Gly Cys Pro Val Arg Lys Gln Val Gln Arg Cys Ala Glu Asp Arg Thr
        355                 360                 365

Ile Leu Ile Thr Thr Tyr Glu Gly Thr His Asn His Pro Leu Pro Pro
    370                 375                 380

Ala Ala Met Ala Met Ala Ser Thr Thr Ser Ala Ala Ala Ser Met Leu
385                 390                 395                 400

Leu Ser Gly Ser Met Pro Ser Ala Asp Gly Ala Gly Leu Met Ser Ser
                405                 410                 415

Asn Phe Leu Ala Arg Thr Val Leu Pro Cys Ser Ser Ser Met Ala Thr
            420                 425                 430

Ile Ser Ala Ser Ala Pro Phe Pro Thr Val Thr Leu Asp Leu Thr His
        435                 440                 445

Ala Pro Pro Gly Ala Pro Asn Ala Met Pro Leu Asn Val Ala Arg Pro
    450                 455                 460

His Ala Pro Gly Gln Phe His Val Pro Met Pro Gly Gly Gly Met Ala
465                 470                 475                 480

Pro Ala Phe Ala Met Pro Pro His Met Leu Tyr Asn Gln Ser Lys Phe
                485                 490                 495

Ser Gly Leu Gln Met Ser Ser Asp Ser Val Asp Pro Gly Gln Phe Ala
            500                 505                 510

Gln Pro Arg Pro Gln Met Gly Leu Pro Gly Gln Leu Ser Asp Thr Val
        515                 520                 525

Ser Ala Ala Ala Ala Ala Ile Thr Ala Asp Pro Asn Phe Thr Val Ala
    530                 535                 540

Leu Ala Ala Ala Ile Ser Ser Ile Met Ala Gly Gln His Ala Ala Gly
545                 550                 555                 560

Asn Ser Asn Ala Asn Asn Ser Asn Asn Asn Thr Thr Ser Asn Asn Asn
                565                 570                 575

Val Thr Thr Thr Ser Asn Asn Thr Thr Ser Asn Asn Thr Asn Ser Glu
            580                 585                 590

Thr Gln

<210>  39
<211>  1833
<212>  DNA
<213>  Hordeum vulgare

<400>  39
acttcagact cctccctctt tcggtctcta cgacacgttt ctctctcccc ctccctctcc      60

ccctcccctc gttccctccc ctcggctagc gtctctctct tctccaagag gctctcgatt     120

tgcatggaca agggccacct cggtggtggc ggaggaggag gagggctgct cgctctggac 180

gcgtcgccgc ggcagcttgg cttcttgaac ttgctttcgc cggcgccgtt cctcaggaac 240

atggaggcgg acgacggcgg tggcggcggc aggggcagga ggtctatcga ggttgacttc 300

ttctccgacg agaagaagaa catgaagaag agccgggctt cggccggtgc cgacgcggac 360

gaccagaagg atcaggcctc cgccgccgga ctcgccatca agaaggagga cctcaccatt 420

aacctccttt ccgggaacaa cacgaagcgc gaccggtcca tggtagtcga cgacgacggg 480

gcgtcccggg ccgatgagga cagtaacggc aggaacaccg gcgagctcgc ggcgatgcag 540

gccgagctga gccgcatgaa cgacgagaac cagcggctgc gagggatgtt gacccaggtc 600

aataacagct accatgcgct gcagatgcat ctcgtcacgc tcatgcagca aaggacccag 660

atgcctccgg cccaaccgca acagcctccg actcacgagg agggcaagaa cgagagtgcc 720

atcgtgccga ggcagttcct gggtctgggc ccgtccggag ccagcgctga ggtggcggaa 780

gagccgtcca actcgtccac ggaggtcggg agcccgcgcc ggtcgtcatc caacggaaac 840

gaggaccctg agcgtggcga caacccagac ggcccgtcca ccgcggggtg gttgcccgga 900

cgcgggatga cccagcagca gcagctggga gcggcggcca agggccacga ccagcaagcc 960

caggaggcca ccatgaggaa ggcccgtgtc tccgttcgcg cacgatcaga agccccgatt 1020

atcgccgatg gatgccaatg gaggaagtac ggtcagaaga tggcgaaggg aaacccttgc 1080

ccacgcgcct actaccgctg caccatggcc accggctgcc cggtgcgcaa gcaggtgcaa 1140

cgatgcgccg aggaccggac catcctcatc accacgtacg agggcacgca caaccacccg 1200

ctcccgccgg ccgccatggc catggcgtct accacgtcgg ccgccgcgtc catgctgctc 1260

tcgggctcca tgcccagcgc cgacggcgcg gggctcatga gctccaactt cctcgcgcgc 1320

accgtgctgc cgtgctcgtc cagcatggcc accatctcgg cgtccgcgcc gttcccgact 1380

gtcaccctcg acctcaccca cgccccgccg ggtgcgccca acgccatgcc gctaaacgtc 1440

gcccgaccgc atgcaccggg gcagttccat gttccgatgc ccggcggtgg gatggccccg 1500

gccttcgcca tgccgccgca gatgctctac aaccagtcga agttctctgg tctgcagatg 1560

tcctccgact cggtggacgc cgggcagttc gcgcagccta ggcagcccat gggcctgcca 1620

ggccagctgt ccgacagcgt cagtgcagcg gcggctgcga tcaccgcaga cccaaacttc 1680

acggtggcgc tcgccgcggc aatctcgtcc atcatggccg gccagcacgc cgccggcaac 1740

agcaacgcga acaacaacaa caccactaag aacaacgtga cgacgaccag taacaacacg 1800

acgagcaaca acaccaacag cgagactcaa tga 1833

<210> 40
<211> 610
<212> PRT
<213> Hordeum vulgare

<400>  40
   Thr Ser Asp Ser Ser Leu Phe Arg Ser Leu Arg His Val Ser Leu Ser
   1               5                   10                  15
   Pro Ser Leu Ser Pro Ser Pro Arg Ser Leu Pro Ser Ala Ser Val Ser
               20                  25                  30
   Leu Phe Ser Lys Arg Leu Ser Ile Cys Met Asp Lys Gly His Leu Gly
           35                  40                  45
   Gly Gly Gly Gly Gly Gly Gly Leu Leu Ala Leu Asp Ala Ser Pro Arg
       50                  55                  60
   Gln Leu Gly Phe Leu Asn Leu Leu Ser Pro Ala Pro Phe Leu Arg Asn
   65                  70                  75                  80
   Met Glu Ala Asp Asp Gly Gly Gly Gly Gly Arg Gly Arg Arg Ser Ile
                   85                  90                  95
   Glu Val Asp Phe Phe Ser Asp Glu Lys Lys Asn Met Lys Lys Ser Arg
               100                 105                 110
   Ala Ser Ala Gly Ala Asp Ala Asp Asp Gln Lys Asp Gln Ala Ser Ala
           115                 120                 125
   Ala Gly Leu Ala Ile Lys Lys Glu Asp Leu Thr Ile Asn Leu Leu Ser
       130                 135                 140
   Gly Asn Asn Thr Lys Arg Asp Arg Ser Met Val Val Asp Asp Asp Gly
   145                 150                 155                 160
   Ala Ser Arg Ala Asp Glu Asp Ser Asn Gly Arg Asn Thr Gly Glu Leu
                   165                 170                 175
   Ala Ala Met Gln Ala Glu Leu Ser Arg Met Asn Asp Glu Asn Gln Arg
               180                 185                 190
   Leu Arg Gly Met Leu Thr Gln Val Asn Asn Ser Tyr His Ala Leu Gln
           195                 200                 205
   Met His Leu Val Thr Leu Met Gln Gln Arg Thr Gln Met Pro Pro Ala
       210                 215                 220
   Gln Pro Gln Gln Pro Pro Thr His Glu Glu Gly Lys Asn Glu Ser Ala
   225                 230                 235                 240
   Ile Val Pro Arg Gln Phe Leu Gly Leu Gly Pro Ser Gly Ala Ser Ala
                   245                 250                 255
   Glu Val Ala Glu Glu Pro Ser Asn Ser Ser Thr Glu Val Gly Ser Pro
               260                 265                 270
   Arg Arg Ser Ser Ser Asn Gly Asn Glu Asp Pro Glu Arg Gly Asp Asn
           275                 280                 285
   Pro Asp Gly Pro Ser Thr Ala Gly Trp Leu Pro Gly Arg Gly Met Thr
       290                 295                 300
   Gln Gln Gln Gln Leu Gly Ala Ala Ala Lys Gly His Asp Gln Gln Ala
   305                 310                 315                 320
   Gln Glu Ala Thr Met Arg Lys Ala Arg Val Ser Val Arg Ala Arg Ser
                   325                 330                 335
   Glu Ala Pro Ile Ile Ala Asp Gly Cys Gln Trp Arg Lys Tyr Gly Gln
               340                 345                 350
   Lys Met Ala Lys Gly Asn Pro Cys Pro Arg Ala Tyr Tyr Arg Cys Thr
           355                 360                 365
   Met Ala Thr Gly Cys Pro Val Arg Lys Gln Val Gln Arg Cys Ala Glu
       370                 375                 380
   Asp Arg Thr Ile Leu Ile Thr Thr Tyr Glu Gly Thr His Asn His Pro
   385                 390                 395                 400
   Leu Pro Pro Ala Ala Met Ala Met Ala Ser Thr Thr Ser Ala Ala Ala
                   405                 410                 415
   Ser Met Leu Leu Ser Gly Ser Met Pro Ser Ala Asp Gly Ala Gly Leu
               420                 425                 430
   Met Ser Ser Asn Phe Leu Ala Arg Thr Val Leu Pro Cys Ser Ser Ser
           435                 440                 445
   Met Ala Thr Ile Ser Ala Ser Ala Pro Phe Pro Thr Val Thr Leu Asp
       450                 455                 460
   Leu Thr His Ala Pro Pro Gly Ala Pro Asn Ala Met Pro Leu Asn Val
   465                 470                 475                 480
   Ala Arg Pro His Ala Pro Gly Gln Phe His Val Pro Met Pro Gly Gly
                   485                 490                 495
   Gly Met Ala Pro Ala Phe Ala Met Pro Pro Gln Met Leu Tyr Asn Gln
               500                 505                 510
   Ser Lys Phe Ser Gly Leu Gln Met Ser Ser Asp Ser Val Asp Ala Gly
           515                 520                 525
   Gln Phe Ala Gln Pro Arg Gln Pro Met Gly Leu Pro Gly Gln Leu Ser
       530                 535                 540
   Asp Ser Val Ser Ala Ala Ala Ala Ala Ile Thr Ala Asp Pro Asn Phe
   545                 550                 555                 560
   Thr Val Ala Leu Ala Ala Ala Ile Ser Ser Ile Met Ala Gly Gln His
                   565                 570                 575
   Ala Ala Gly Asn Ser Asn Ala Asn Asn Asn Asn Thr Thr Lys Asn Asn
               580                 585                 590
   Val Thr Thr Thr Ser Asn Asn Thr Thr Ser Asn Asn Thr Asn Ser Glu
           595                 600                 605
   Thr Gln
       610

<210>  41
<211>  636
<212>  DNA
<213>  Triticum aestivum

<220>
<221>  misc_feature
<222>  (494)..(494)
<223>  n is a, c, g, or t

<220>
<221>  misc_feature
<222>  (568)..(568)
<223>  n is a, c, g, or t

<400>  41
atggacgagc agtggatgat ggggcagact tccctcagcc tcggcctcaa cgtcggccgg      60

ccgacggcgc cgcggggtgc ccctccggtg accaaggttc tcgtggagga ggacttcatg     120

tcctccaaga agaatcacga ggtcgaggcg ctggaggcgg agctccggcg cgtgggcgag     180

gagaacagga ggcttagcga catgctccgc gcgctggtgg ccaagtactg cgacctgcag     240

ggcagggtca gcggcatgat ggcggcggcg gtggcggcca acaaccacca gtcacccacg     300

acatctgagg gcggctccgc ggcctcgccg tccaggaagc gcgcccgcag cgatagcccc     360

gacaccgccg tccaggaagc gcgccctgcc agcggcggct tcgccgtcag cgtcaacgtc     420

ggccccgacc aggcggagtg cacgtccgtc cacgagccgt gcaacagcaa gcgcgtccgc 480

gccgacgagt gcanggccag cagggtatcc aagctgtatg tccacgccga tccctcggac 540

ctcagcctcg tcgtgaaaga tgggtacnca tggcgaaagt acgggcagaa ggtgacccaa 600

gaacaaccct ttgccctgag ggctacttcc gggtgc 636

<210> 42
<211> 212
<212> PRT
<213> Triticum aestivum

<220>
<221> misc_feature
<222> (165)..(165)
<223> Xaa can be any naturally occurring amino acid

<220>
<221> misc_feature
<222> (190)..(190)
<223> Xaa can be any naturally occurring amino acid

<400> 42

Met Asp Glu Gln Trp Met Met Gly Gln Thr Ser Leu Ser Leu Gly Leu
1               5                   10                  15

Asn Val Gly Arg Pro Thr Ala Pro Arg Gly Ala Pro Pro Val Thr Lys
            20                  25                  30

Val Leu Val Glu Glu Asp Phe Met Ser Ser Lys Lys Asn His Glu Val
        35                  40                  45

Glu Ala Leu Glu Ala Glu Leu Arg Arg Val Gly Glu Glu Asn Arg Arg
    50                  55                  60

Leu Ser Asp Met Leu Arg Ala Leu Val Ala Lys Tyr Cys Asp Leu Gln
65                  70                  75                  80

Gly Arg Val Ser Gly Met Met Ala Ala Ala Val Ala Ala Asn Asn His
                85                  90                  95

Gln Ser Pro Thr Thr Ser Glu Gly Gly Ser Ala Ala Ser Pro Ser Arg
            100                 105                 110

Lys Arg Ala Arg Ser Asp Ser Pro Asp Thr Ala Val Gln Glu Ala Arg
        115                 120                 125

Pro Ala Ser Gly Gly Phe Ala Val Ser Val Asn Val Gly Pro Asp Gln
    130                 135                 140

Ala Glu Cys Thr Ser Val His Glu Pro Cys Asn Ser Lys Arg Val Arg
145                 150                 155                 160

Ala Asp Glu Cys Xaa Ala Ser Arg Val Ser Lys Leu Tyr Val His Ala
                165                 170                 175

Asp Pro Ser Asp Leu Ser Leu Val Val Lys Asp Gly Tyr Xaa Trp Arg
            180                 185                 190

Lys Tyr Gly Gln Lys Val Thr Gln Glu Gln Pro Phe Ala Leu Arg Ala
        195                 200                 205

Thr Ser Gly Cys
    210

<210>  43
<211>  660
<212>  DNA
<213>  Triticum aestivum

<400>  43
atggacgagc agtggatgat cgggcagact tcgctgagcc tcggcctcaa cgtcggccgg      60

ccgacggcgc ggcggggtgc ccctccggtg accaaggtcc tcgtggagga ggacttcatg     120

tcttccaaga agaaccacga ggtcgaggcg ctgcaggcgg agctccggcg cgtgggcgag     180

gagaacagga ggctcagcga catgctccgc gcgctcgtgg ccaagtactc cgacctccag     240

ggcaaggtca gcggcatgat ggcggcggcg gccgcggcca acaaccacca gtcgttgtct     300

acgtctgagg gcggctccgc ggcctcgccc tccaggaagc gcgcccgcag cgacagcctc     360

gacaccgccg gccgcaatcc ttcgccgccg ctcgccgcgg ccgtcagcat caacgtcgtc     420

cccgaccgcg accaggtgga gtgcacgtcc gtccatgagc cgtgcaacag caagcgcgtc     480

cgagccgacg agtgcaaggc cagcagggtc tccaagctct acgtccacgc cgacccctcc     540

gacctcagcc tcgtcgtgaa ggatgggtac caatggcgga agtacggcca gaaggtgacc     600

aaggacaacc cgtgcccgag ggcctacttc cggtgctcgt tcgcgccgtc gtgccaggtg     660

<210>  44
<211>  220
<212>  PRT
<213>  Triticum aestivum

<400>  44
   Met Asp Glu Gln Trp Met Ile Gly Gln Thr Ser Leu Ser Leu Gly Leu
   1               5                   10                  15
   Asn Val Gly Arg Pro Thr Ala Arg Arg Gly Ala Pro Pro Val Thr Lys
               20                  25                  30
   Val Leu Val Glu Glu Asp Phe Met Ser Ser Lys Lys Asn His Glu Val
   35 40 45
   Glu Ala Leu Gln Ala Glu Leu Arg Arg Val Gly Glu Glu Asn Arg Arg
   50 55 60
   Leu Ser Asp Met Leu Arg Ala Leu Val Ala Lys Tyr Ser Asp Leu Gln
   65 70 75 80
   Gly Lys Val Ser Gly Met Met Ala Ala Ala Ala Ala Ala Asn Asn His
   85 90 95
   Gln Ser Leu Ser Thr Ser Glu Gly Gly Ser Ala Ala Ser Pro Ser Arg
   100 105 110
   Lys Arg Ala Arg Ser Asp Ser Leu Asp Thr Ala Gly Arg Asn Pro Ser
   115 120 125
   Pro Pro Leu Ala Ala Ala Val Ser Ile Asn Val Val Pro Asp Arg Asp
   130 135 140
   Gln Val Glu Cys Thr Ser Val His Glu Pro Cys Asn Ser Lys Arg Val
   145 150 155 160
   Arg Ala Asp Glu Cys Lys Ala Ser Arg Val Ser Lys Leu Tyr Val His
   165 170 175
   Ala Asp Pro Ser Asp Leu Ser Leu Val Val Lys Asp Gly Tyr Gln Trp
   180 185 190
   Arg Lys Tyr Gly Gln Lys Val Thr Lys Asp Asn Pro Cys Pro Arg Ala
   195 200 205
   Tyr Phe Arg Cys Ser Phe Ala Pro Ser Cys Gln Val
   210 215 220

<210>  45
<211>  657
<212>  DNA
<213>  Triticum aestivum

<400>  45
atggacgagc agtggatgat ggggcagact tccctcagcc tcggcctcaa cgtcggccgg      60

ccgacggcgc ggcggggtgc ccctccggtg accaagatcc tcgtggagga ggacttcatg     120

tcctccaaga agaatcacga ggtcgaggcg ctggaggccg agctccggcg cgtgggcgag     180

gagaacagga ggctcagcga catgctccgc gcgctggtgg ccaagtactc cgaccttcag     240

ggcagggtca gcggcatgat ggcggcggcg gccgcggcca acaaccgcca gtcacccacg 300

acgtctgagg gcggctccgc ggcctcgccg tccaggaagc gcgctcgcag cgacagtccc 360

gacaccgcag tccaggaagc gtaccctgcc agcggcggct tcgccgtcag cgtcaacgtc 420

ggccccgacc aggcggagtg cacgtccgtc cacgagccgg gcaacagcaa gcgcgtccgc 480

gccgacgagt gcaaggccag ccgggtctcc aagctctacg tccacgccga ccccgccgac 540

ctcagcctcg tggtgaagga tgggtaccaa tggcgaaagt acggcaagaa ggtgaccaag 600

gacaacccat gcccgagggc ctacttccgg tgctccttcg cgccgtcctg ccaggtg 657

<210> 46
<211> 219
<212> PRT
<213> Triticum aestivum

<400> 46
   Met Asp Glu Gln Trp Met Met Gly Gln Thr Ser Leu Ser Leu Gly Leu
   1 5 10 15
   Asn Val Gly Arg Pro Thr Ala Arg Arg Gly Ala Pro Pro Val Thr Lys
   20 25 30
   Ile Leu Val Glu Glu Asp Phe Met Ser Ser Lys Lys Asn His Glu Val
   35 40 45
   Glu Ala Leu Glu Ala Glu Leu Arg Arg Val Gly Glu Glu Asn Arg Arg
   50 55 60
   Leu Ser Asp Met Leu Arg Ala Leu Val Ala Lys Tyr Ser Asp Leu Gln
   65 70 75 80
   Gly Arg Val Ser Gly Met Met Ala Ala Ala Ala Ala Ala Asn Asn Arg
   85 90 95
   Gln Ser Pro Thr Thr Ser Glu Gly Gly Ser Ala Ala Ser Pro Ser Arg
   100 105 110
   Lys Arg Ala Arg Ser Asp Ser Pro Asp Thr Ala Val Gln Glu Ala Tyr
   115 120 125
   Pro Ala Ser Gly Gly Phe Ala Val Ser Val Asn Val Gly Pro Asp Gln
   130 135 140
   Ala Glu Cys Thr Ser Val His Glu Pro Gly Asn Ser Lys Arg Val Arg
   145 150 155 160
   Ala Asp Glu Cys Lys Ala Ser Arg Val Ser Lys Leu Tyr Val His Ala
                   165                 170                 175
   Asp Pro Ala Asp Leu Ser Leu Val Val Lys Asp Gly Tyr Gln Trp Arg
               180                 185                 190
   Lys Tyr Gly Lys Lys Val Thr Lys Asp Asn Pro Cys Pro Arg Ala Tyr
           195                 200                 205
   Phe Arg Cys Ser Phe Ala Pro Ser Cys Gln Val
       210                 215

<210>  47
<211>  687
<212>  DNA
<213>  Secale cereale

<400>  47
atggacgagc agtggatgat cgggcagact tccctcagcc tcggccttaa catcgcccgg      60

ccgacggcgc ggctaggtgc tcccccggtg accaaggtcc tcgtggagga ggacttcatg     120

tcctccagga agaatcacga ggtcgaggca ctggaggcgg agctccggcg agtgggcgag     180

gagaaccgga ggctcagcga catgctccgc gcgctggtgg ccaagtacgc tgacctgcag     240

ggcaaggtca gcggcatgat ggcggcggcg tcagccgctg ccaacaaccg ccagtcgtcc     300

accacgtctg agggcggctc cgcggcctcg ccgtccagga agcgcgcccg cagcgacagc     360

ctcgacaccg ccggccgcaa tccttcgccg ccgctcgccg cggccgcaag tggcggcttc     420

gccgtcagcg tcaacgtcaa cgtcggcccc gaccgcgatc aggcggagtg cacgtccgtt     480

cacgagcacg gcaacagcaa gcgtgtccgc gccgacgagt gcaaggccag cagggtctca     540

aagctctacg tccacgccga cccctccgac ctcagcctcg tcgtgaagga tgggtaccaa     600

tggcgaaagt acgggcagaa ggtgaccaag gacaacccgt gcccgagggc ctacttccgg     660

tgctcgttcg cgccgtcgtg ccaggtg                                         687

<210>  48
<211>  229
<212>  PRT
<213>  Secale cereale

<400>  48

Met Asp Glu Gln Trp Met Ile Gly Gln Thr Ser Leu Ser Leu Gly Leu
1               5                   10                  15

Asn Ile Ala Arg Pro Thr Ala Arg Leu Gly Ala Pro Pro Val Thr Lys
            20                  25                  30

Val Leu Val Glu Glu Asp Phe Met Ser Ser Arg Lys Asn His Glu Val

        35                  40                  45

Glu Ala Leu Glu Ala Glu Leu Arg Arg Val Gly Glu Glu Asn Arg Arg
    50                  55                  60

Leu Ser Asp Met Leu Arg Ala Leu Val Ala Lys Tyr Ala Asp Leu Gln
65                  70                  75                  80

Gly Lys Val Ser Gly Met Met Ala Ala Ala Ser Ala Ala Ala Asn Asn
                85                  90                  95

Arg Gln Ser Ser Thr Thr Ser Glu Gly Gly Ser Ala Ala Ser Pro Ser
            100                 105                 110

Arg Lys Arg Ala Arg Ser Asp Ser Leu Asp Thr Ala Gly Arg Asn Pro
        115                 120                 125

Ser Pro Pro Leu Ala Ala Ala Ala Ser Gly Gly Phe Ala Val Ser Val
    130                 135                 140

Asn Val Asn Val Gly Pro Asp Arg Asp Gln Ala Glu Cys Thr Ser Val
145                 150                 155                 160

His Glu His Gly Asn Ser Lys Arg Val Arg Ala Asp Glu Cys Lys Ala
                165                 170                 175

Ser Arg Val Ser Lys Leu Tyr Val His Ala Asp Pro Ser Asp Leu Ser
            180                 185                 190

Leu Val Val Lys Asp Gly Tyr Gln Trp Arg Lys Tyr Gly Gln Lys Val
        195                 200                 205

Thr Lys Asp Asn Pro Cys Pro Arg Ala Tyr Phe Arg Cys Ser Phe Ala
    210                 215                 220

Pro Ser Cys Gln Val
225

<210> 49
<211> 684
<212> DNA
<213> Hordeum vulgare

<400> 49
atggacgagc agtggatgat ggggcagact tccctcagcc tcggcctcaa cgtgggccgg 60

ccgacggcgc ggggaggggg tcctccggtg ccgtccaagg tgctcgtgga ggaggacttc 120

atgtcctcca agaaaagcca cgaggtcgag gcgctggagg cggagctccg gcgtgtgggc 180

gaggagaaca ggaggctggg cgacatgctc cgcgcgctgg tggtaaagta ctccgacctg 240

cagggcaagg tcagcggcat gatggcggcg gcggcggcca ataaccacca gtcgttgacg 300

ttgactacgt ctgagggcgg ctccgcggcc tcgccgtcca ggaagcgcgc ccgtagcgac 360

agcctcgaca ccgccggccg caatccttcg cctccgctcg ccgcggccgc caccggcggc 420

ttcgccatca gcgtcaacgt cggccccggc cgcgaccagg cggagtgcac gtccgtccac 480

gagccgtgca acagcaagcg cgtccgcgcc gacgagtgca aggccagcag ggtctccaag 540

ctctacgtcc acgccgaccc ctccgacctc agcctcgtgg tgaaggatgg gtaccaatgg 600

cggaagtacg ggcagaaggt gaccaaggac aatccgtgcc cgagggccta cttccggtgc 660

tcgttcgcgc cgtcatgcca ggtg 684

<210> 50
<211> 228
<212> PRT
<213> Hordeum vulgare

<400> 50

Met Asp Glu Gln Trp Met Met Gly Gln Thr Ser Leu Ser Leu Gly Leu
1               5                   10                  15

Asn Val Gly Arg Pro Thr Ala Arg Gly Gly Gly Pro Pro Val Pro Ser
            20                  25                  30

Lys Val Leu Val Glu Glu Asp Phe Met Ser Ser Lys Lys Ser His Glu
        35                  40                  45

Val Glu Ala Leu Glu Ala Glu Leu Arg Arg Val Gly Glu Glu Asn Arg
    50                  55                  60

Arg Leu Gly Asp Met Leu Arg Ala Leu Val Val Lys Tyr Ser Asp Leu
65                  70                  75                  80

Gln Gly Lys Val Ser Gly Met Met Ala Ala Ala Ala Ala Asn Asn His
                85                  90                  95

Gln Ser Leu Thr Leu Thr Thr Ser Glu Gly Gly Ser Ala Ala Ser Pro
            100                 105                 110

Ser Arg Lys Arg Ala Arg Ser Asp Ser Leu Asp Thr Ala Gly Arg Asn
        115                 120                 125

Pro Ser Pro Pro Leu Ala Ala Ala Ala Thr Gly Gly Phe Ala Ile Ser
    130                 135                 140

Val Asn Val Gly Pro Gly Arg Asp Gln Ala Glu Cys Thr Ser Val His

145                 150                 155                 160

Glu Pro Cys Asn Ser Lys Arg Val Arg Ala Asp Glu Cys Lys Ala Ser
                165                 170                 175

Arg Val Ser Lys Leu Tyr Val His Ala Asp Pro Ser Asp Leu Ser Leu
            180                 185                 190

Val Val Lys Asp Gly Tyr Gln Trp Arg Lys Tyr Gly Gln Lys Val Thr
        195                 200                 205

Lys Asp Asn Pro Cys Pro Arg Ala Tyr Phe Arg Cys Ser Phe Ala Pro
    210                 215                 220

Ser Cys Gln Val
225

<210>  51
<211>  1233
<212>  DNA
<213>  Triticum aestivum

<400>  51
atgtcctcct ccacggggag cttggaccac gcagggttca cgttcacgcc gccgccgttc      60

atcacgtcct tcaccgagct tctgtcgggg tccggcgccg gcgacgcgga gcggtcgccg     120

agggggttca accgaggggg ccgggccggg gcgcccaagt tcaagtccgc gcagccgccc     180

agcctgccca tctcgtcgcc cttctcctgc ttctccgtcc ctgcaggtct cagccctgcc     240

gagctgctcg actcccccgt tctcctcaac tactctcaca tcttggcgtc tccgactacg     300

ggtgcgatcc ctgcgcagag gtgcgactgg caggcgagcg ccgatctgaa cacctttcag     360

caggatgagc tcggcctctc tggcttctcc tttcacgcag tcaagtccaa cgccacggtc     420

aacgctcaag taaactactt acctttattc aaggagcagc aggagcaaca acaagaagaa     480

gtggttcaag tgagcaacaa gagcagcagc agcagcggca acaacaagca ggttgaggac     540

ggatacaatt ggaggaagta cgggcagaag caagttaagg ggagcgagaa cccgcggagc     600

tactacaagt gcacctacaa caattgctcc atgaagaaga aagtggagcg ctctctcgcc     660

gacggccgca tcacgcagat cgtctacaag ggcgcacatg accacccgaa gcccctctcc     720

acgcgccgca actcctccgg ctgcgcggcg gtcgttgcgg aggatcatac caacggctcg     780

gagcactctg gcccgacgcc cgagaattca tccgtcactt tcggagacga tgaggccgac     840

aagcccgaga ccaagcgccg gaaggagcat ggtgacaacg agggcagttc aggcggcacc     900

ggcggctgcg ggaagcccgt gcgcgagccc aggctcgtgg tgcagacgct gagcgatata     960

gacatactcg acgacggctt ccggtggagg aagtacgggc agaaggttgt caagggcaat    1020

cccaacccca ggagctacta caagtgcaca acggtgggct gcccggtgcg caagcatgtg 1080

gagcgggcct cgcacgacaa ccgcgcggtg atcaccacct acgagggccc cgtgcaccct 1140

cgagatgctc gccggacacc caggctacgc ggccaaggac gagccccggg acgacatgtt 1200

cgtcgagtcg ctcctctgct agctaggcag gct 1233

<210> 52
<211> 411
<212> PRT
<213> Triticum aestivum

<400> 52

Met Ser Ser Ser Thr Gly Ser Leu Asp His Ala Gly Phe Thr Phe Thr
1               5                   10                  15

Pro Pro Pro Phe Ile Thr Ser Phe Thr Glu Leu Leu Ser Gly Ser Gly
            20                  25                  30

Ala Gly Asp Ala Glu Arg Ser Pro Arg Gly Phe Asn Arg Gly Gly Arg
        35                  40                  45

Ala Gly Ala Pro Lys Phe Lys Ser Ala Gln Pro Pro Ser Leu Pro Ile
    50                  55                  60

Ser Ser Pro Phe Ser Cys Phe Ser Val Pro Ala Gly Leu Ser Pro Ala
65                  70                  75                  80

Glu Leu Leu Asp Ser Pro Val Leu Leu Asn Tyr Ser His Ile Leu Ala
                85                  90                  95

Ser Pro Thr Thr Gly Ala Ile Pro Ala Gln Arg Cys Asp Trp Gln Ala
            100                 105                 110

Ser Ala Asp Leu Asn Thr Phe Gln Gln Asp Glu Leu Gly Leu Ser Gly
        115                 120                 125

Phe Ser Phe His Ala Val Lys Ser Asn Ala Thr Val Asn Ala Gln Val
    130                 135                 140

Asn Tyr Leu Pro Leu Phe Lys Glu Gln Gln Glu Gln Gln Gln Glu Glu
145                 150                 155                 160

Val Val Gln Val Ser Asn Lys Ser Ser Ser Ser Ser Gly Asn Asn Lys
                165                 170                 175

Gln Val Glu Asp Gly Tyr Asn Trp Arg Lys Tyr Gly Gln Lys Gln Val
            180                 185                 190

Lys Gly Ser Glu Asn Pro Arg Ser Tyr Tyr Lys Cys Thr Tyr Asn Asn
        195                 200                 205

Cys Ser Met Lys Lys Lys Val Glu Arg Ser Leu Ala Asp Gly Arg Ile
    210                 215                 220

Thr Gln Ile Val Tyr Lys Gly Ala His Asp His Pro Lys Pro Leu Ser
225                 230                 235                 240

Thr Arg Arg Asn Ser Ser Gly Cys Ala Ala Val Val Ala Glu Asp His
                245                 250                 255

Thr Asn Gly Ser Glu His Ser Gly Pro Thr Pro Glu Asn Ser Ser Val
            260                 265                 270

Thr Phe Gly Asp Asp Glu Ala Asp Lys Pro Glu Thr Lys Arg Arg Lys
        275                 280                 285

Glu His Gly Asp Asn Glu Gly Ser Ser Gly Gly Thr Gly Gly Cys Gly
    290                 295                 300

Lys Pro Val Arg Glu Pro Arg Leu Val Val Gln Thr Leu Ser Asp Ile
305                 310                 315                 320

Asp Ile Leu Asp Asp Gly Phe Arg Trp Arg Lys Tyr Gly Gln Lys Val
                325                 330                 335

Val Lys Gly Asn Pro Asn Pro Arg Ser Tyr Tyr Lys Cys Thr Thr Val
            340                 345                 350

Gly Cys Pro Val Arg Lys His Val Glu Arg Ala Ser His Asp Asn Arg
        355                 360                 365

Ala Val Ile Thr Thr Tyr Glu Gly Pro Val His Pro Arg Asp Ala Arg
    370                 375                 380

Arg Thr Pro Arg Leu Arg Gly Gln Gly Arg Ala Pro Gly Arg His Val
385                 390                 395                 400

Arg Arg Val Ala Pro Leu Leu Ala Arg Gln Ala
                405                 410

<210>  53
<211>  1416
<212>  DNA
<213>  Triticum aestivum

<400>  53

atgtcctcct tcacggggag cttggaccac gcagggttca cgttcacgcc gccgccgttc 60

atcacgtcct tgactgagct tctatcaggg tctggcgccg gcgacgtgga ggggtcgcca 120

agggggttca accgaggggg tcggaccggg gtgcccaagt tcaagtccgc gcagccgccc 180

agcctgccca tctcgtcgcc agcgtcgccc ttctcctgct tctccatccc tgcaggtctc 240

agccctgctg agctgctcca ctctcccgtt ctcctcaact actctcatat attggcgtct 300

ccgactacag gtgcgatccc tgcgcggaga tacgactggc aggcgagcgc cgatctgaac 360

acctttcagc aggatgaggt cggccgtgga gacagcggcc tctttggctt ctcctttcac 420

gcagtgaagc ccaacgccac ggtcaacgct caaacaaatt acttaccttt attcaaggaa 480

catcagcagc agcagcaaca acaacaagtg gtagaagtga gcaacaagag cagcagcggc 540

gacaacaaca agcaggttga ggacggatac aattggagga agtacggcca gaagcaagtt 600

aagggcagcg agaacccgcg gagctactac aagtgcacct acaacaattg ctccatgaag 660

aagaaagtgg aacgctctct tgcagacggc cgcatcacgc agattgtcta caagggcgcg 720

catgatcacc cgaagccccc ctccacgcgc cgcaactcct ccggctgtgc ggcggtcatt 780

gcggaggatc ataccaacgg ctcggagcac tctggcccga cgcctgagaa ttcatccgtc 840

acattcggag acgatgagac cgacaatggc gctgagcctg agaccaagcg ccggaaggag 900

cacggtgaca acgagggcag ttcgggtggc accggcgcct gcgtgaagcc cgtgcgcgag 960

cccaggcttg tggtgcagac gctgagcgat atagacatac tcgacgacgg cttccggtgg 1020

aggaagtacg ggcagaaggt tgtcaagggc aatcccaacc ccaggagcta ctacaagtgc 1080

acaacggtgg gttgcccggt gcgcaagcac gtggagcggg cctcgcacga caaccgcgcg 1140

gtgattacca cctacgaggg taggcacagc cacgacgtgc cggtcggcag gggggccggt 1200

gctagccgcg cgctgccgac gtcgtcttcc tccgacagct cggtcgtcgt ctgtcctgcc 1260

gccgccgggc aggccccgta caccctcgag atgctcgcca accctgccgc cggacaccga 1320

ggctacgcgg ccaaggacga accccggggc ggcgccttcc agcgcaccaa ggacgagccc 1380

cgggacgaca tgttcgtcga gtcgctcctc tgctag 1416

<210> 54
<211> 471
<212> PRT
<213> Triticum aestivum

<400> 54

Met Ser Ser Phe Thr Gly Ser Leu Asp His Ala Gly Phe Thr Phe Thr
1               5                   10                  15

Pro Pro Pro Phe Ile Thr Ser Leu Thr Glu Leu Leu Ser Gly Ser Gly
            20                  25                  30

Ala Gly Asp Val Glu Gly Ser Pro Arg Gly Phe Asn Arg Gly Gly Arg
        35                  40                  45

Thr Gly Val Pro Lys Phe Lys Ser Ala Gln Pro Pro Ser Leu Pro Ile
    50                  55                  60

Ser Ser Pro Ala Ser Pro Phe Ser Cys Phe Ser Ile Pro Ala Gly Leu
65                  70                  75                  80

Ser Pro Ala Glu Leu Leu His Ser Pro Val Leu Leu Asn Tyr Ser His
                85                  90                  95

Ile Leu Ala Ser Pro Thr Thr Gly Ala Ile Pro Ala Arg Arg Tyr Asp
            100                 105                 110

Trp Gln Ala Ser Ala Asp Leu Asn Thr Phe Gln Gln Asp Glu Val Gly
        115                 120                 125

Arg Gly Asp Ser Gly Leu Phe Gly Phe Ser Phe His Ala Val Lys Pro
    130                 135                 140

Asn Ala Thr Val Asn Ala Gln Thr Asn Tyr Leu Pro Leu Phe Lys Glu
145                 150                 155                 160

His Gln Gln Gln Gln Gln Gln Gln Gln Val Val Glu Val Ser Asn Lys
                165                 170                 175

Ser Ser Ser Gly Asp Asn Asn Lys Gln Val Glu Asp Gly Tyr Asn Trp
            180                 185                 190

Arg Lys Tyr Gly Gln Lys Gln Val Lys Gly Ser Glu Asn Pro Arg Ser
        195                 200                 205

Tyr Tyr Lys Cys Thr Tyr Asn Asn Cys Ser Met Lys Lys Lys Val Glu
    210                 215                 220

Arg Ser Leu Ala Asp Gly Arg Ile Thr Gln Ile Val Tyr Lys Gly Ala
225                 230                 235                 240

His Asp His Pro Lys Pro Pro Ser Thr Arg Arg Asn Ser Ser Gly Cys
                245                 250                 255

Ala Ala Val Ile Ala Glu Asp His Thr Asn Gly Ser Glu His Ser Gly
            260                 265                 270

Pro Thr Pro Glu Asn Ser Ser Val Thr Phe Gly Asp Asp Glu Thr Asp
        275                 280                 285

Asn Gly Ala Glu Pro Glu Thr Lys Arg Arg Lys Glu His Gly Asp Asn
    290                 295                 300

Glu Gly Ser Ser Gly Gly Thr Gly Ala Cys Val Lys Pro Val Arg Glu
305                 310                 315                 320

Pro Arg Leu Val Val Gln Thr Leu Ser Asp Ile Asp Ile Leu Asp Asp
                325                 330                 335

Gly Phe Arg Trp Arg Lys Tyr Gly Gln Lys Val Val Lys Gly Asn Pro
            340                 345                 350

Asn Pro Arg Ser Tyr Tyr Lys Cys Thr Thr Val Gly Cys Pro Val Arg
        355                 360                 365

Lys His Val Glu Arg Ala Ser His Asp Asn Arg Ala Val Ile Thr Thr
    370                 375                 380

Tyr Glu Gly Arg His Ser His Asp Val Pro Val Gly Arg Gly Ala Gly
385                 390                 395                 400

Ala Ser Arg Ala Leu Pro Thr Ser Ser Ser Ser Asp Ser Ser Val Val
                405                 410                 415

Val Cys Pro Ala Ala Ala Gly Gln Ala Pro Tyr Thr Leu Glu Met Leu
            420                 425                 430

Ala Asn Pro Ala Ala Gly His Arg Gly Tyr Ala Ala Lys Asp Glu Pro
        435                 440                 445

Arg Gly Gly Ala Phe Gln Arg Thr Lys Asp Glu Pro Arg Asp Asp Met
    450                 455                 460

Phe Val Glu Ser Leu Leu Cys
465                 470

<210>  55
<211>  1410
<212>  DNA
<213>  Secale cereale

<400>  55
atgtcgtcct cctccacagg gagcttggac cacgtagggt tcaccttcac gccgccgccg      60

ttcatcacgt ccttcaccga gcttctgtca gggtgtggcg ccggcgacat ggagcggttc     120

aaccgagggg gtcgggccgg ggcgcccaag ttcaagtcgg cgcagccgcc cagcctgccc     180

atctcgccgc ccttctcctg cttctccatc cctgcaggtc tcagccctgc tgagctgctc     240

gactctcccg ttctcctcaa ctactctcat atcttggcgt ctccgactac aggtgcgatc 300

cctgcgcaga gacagagata cgactggcag gcgagcgccg atctcaacac ctttcagcag 360

caggatgagc tctgccgagg agacagcggc ctctttggct tctcctttca cgcagtcaag 420

tccaacgcca cagtcatgag ctctgccgag gagttgtttg tgcaggagca gcaacaacaa 480

caacaagaag tggtagaagt gagcaacaac agcagcagcg gcaacaacaa caagcaggtt 540

gaggacggat acaactggag gaagtacgga cagaagcaag tcaaggggag cgagaacccg 600

cggagctact acaagtgcac ctacaacaat tgctccatga agaagaaagt ggaacgctct 660

cttgccgacg gccgcatcac ggagattgtg tacaagggcg cgcatgacca ccccaagccc 720

ctctccacgc gccgcaactc ctcctccggc tgtgcggcgg cggtcgttgc ggaggatcat 780

gccaacggct cggagcactc cggcccgacg cccgcgaatt catctgtcac tttcggagac 840

gatgaggccg acaacgggtt gcagctcagt gatggggctg agcccgaggc caagcgccgg 900

aaggagcatg gtgacaacaa cgagggcagt tcaggtggca ccggcggcgg ctgcgggaag 960

cccgtgcgcg agcccaggct tgtggtgcag acgctgagcg atatagacat actcgacgac 1020

ggcttccggt ggaggaagta cgggcagaag gttgtcaagg gcaatcccaa ccccaggagc 1080

tactacaagt gcacaacggt gggttgtccg gtgcgcaagc atgtggagcg ggccgcgcac 1140

gacaaccgcg cggtgatcac cacctacgag ggtaagcaca accacgacat gccggtcggc 1200

cggggtgccg gtgccagccg ggcgctgccg acgtcatctt cctcggacag ctcggtcgta 1260

acctggcctg ccgccgtgca ggccccgtac accctcgaga tgctcaccaa ccctgccgcc 1320

ggccaccgag gctatgcagc cggcggcgcc ttccagcgca ccaaggacga gccccgggac 1380

gacatgttcg tcgagtcgct cctctgctag 1410

<210> 56
<211> 469
<212> PRT
<213> Secale cereale

<400> 56

Met Ser Ser Ser Ser Thr Gly Ser Leu Asp His Val Gly Phe Thr Phe
1               5                   10                  15

Thr Pro Pro Pro Phe Ile Thr Ser Phe Thr Glu Leu Leu Ser Gly Cys
            20                  25                  30

Gly Ala Gly Asp Met Glu Arg Phe Asn Arg Gly Gly Arg Ala Gly Ala
        35                  40                  45

Pro Lys Phe Lys Ser Ala Gln Pro Pro Ser Leu Pro Ile Ser Pro Pro
    50                  55                  60

Phe Ser Cys Phe Ser Ile Pro Ala Gly Leu Ser Pro Ala Glu Leu Leu
65                  70                  75                  80

Asp Ser Pro Val Leu Leu Asn Tyr Ser His Ile Leu Ala Ser Pro Thr
                85                  90                  95

Thr Gly Ala Ile Pro Ala Gln Arg Gln Arg Tyr Asp Trp Gln Ala Ser
            100                 105                 110

Ala Asp Leu Asn Thr Phe Gln Gln Gln Asp Glu Leu Cys Arg Gly Asp
        115                 120                 125

Ser Gly Leu Phe Gly Phe Ser Phe His Ala Val Lys Ser Asn Ala Thr
    130                 135                 140

Val Met Ser Ser Ala Glu Glu Leu Phe Val Gln Glu Gln Gln Gln Gln
145                 150                 155                 160

Gln Gln Glu Val Val Glu Val Ser Asn Asn Ser Ser Ser Gly Asn Asn
                165                 170                 175

Asn Lys Gln Val Glu Asp Gly Tyr Asn Trp Arg Lys Tyr Gly Gln Lys
            180                 185                 190

Gln Val Lys Gly Ser Glu Asn Pro Arg Ser Tyr Tyr Lys Cys Thr Tyr
        195                 200                 205

Asn Asn Cys Ser Met Lys Lys Lys Val Glu Arg Ser Leu Ala Asp Gly
    210                 215                 220

Arg Ile Thr Glu Ile Val Tyr Lys Gly Ala His Asp His Pro Lys Pro
225                 230                 235                 240

Leu Ser Thr Arg Arg Asn Ser Ser Ser Gly Cys Ala Ala Ala Val Val
                245                 250                 255

Ala Glu Asp His Ala Asn Gly Ser Glu His Ser Gly Pro Thr Pro Ala
            260                 265                 270

Asn Ser Ser Val Thr Phe Gly Asp Asp Glu Ala Asp Asn Gly Leu Gln
        275                 280                 285

Leu Ser Asp Gly Ala Glu Pro Glu Ala Lys Arg Arg Lys Glu His Gly
    290                 295                 300

Asp Asn Asn Glu Gly Ser Ser Gly Gly Thr Gly Gly Gly Cys Gly Lys

305                 310                 315                 320

Pro Val Arg Glu Pro Arg Leu Val Val Gln Thr Leu Ser Asp Ile Asp
                325                 330                 335

Ile Leu Asp Asp Gly Phe Arg Trp Arg Lys Tyr Gly Gln Lys Val Val
            340                 345                 350

Lys Gly Asn Pro Asn Pro Arg Ser Tyr Tyr Lys Cys Thr Thr Val Gly
        355                 360                 365

Cys Pro Val Arg Lys His Val Glu Arg Ala Ala His Asp Asn Arg Ala
    370                 375                 380

Val Ile Thr Thr Tyr Glu Gly Lys His Asn His Asp Met Pro Val Gly
385                 390                 395                 400

Arg Gly Ala Gly Ala Ser Arg Ala Leu Pro Thr Ser Ser Ser Ser Asp
                405                 410                 415

Ser Ser Val Val Thr Trp Pro Ala Ala Val Gln Ala Pro Tyr Thr Leu
            420                 425                 430

Glu Met Leu Thr Asn Pro Ala Ala Gly His Arg Gly Tyr Ala Ala Gly
        435                 440                 445

Gly Ala Phe Gln Arg Thr Lys Asp Glu Pro Arg Asp Asp Met Phe Val
    450                 455                 460

Glu Ser Leu Leu Cys
465

<210>  57
<211>  948
<212>  DNA
<213>  Triticum aestivum

<400>  57
atggccgtgg acctcatggg ctgctacacc cctcgccgcg ccgacgacca gctcgccatc      60

caggaggccg ccaccgccgg cctccgcagc ctggagctcc tcgtctcctc cctctccggc     120

gcggcgccgt ccaaggcgcc gcagcagcac ccgcagcagc cgttcggcga gatcgccgac     180

caggccgtct ccaagttccg caaggtgatc tccatcctcg accgcaccgg ccacgcccgc     240

ttccgccgcg gccccgtcca gtcgcctacc ccgcctcctc cggctccggt cgctcctccg     300

ccgcccccac cgcgccctct ggccgtcgtc gagccggcca ggcccgctcc cttgaccgtc     360

gtggcgccgg tgtcggtggc cgccccggtc ccgcagccgc agagcctgac gctggacttc     420

accaagccga acctgaccat gtcgggcgcg acgtccgtca cgtccacgtc cttcttctcg 480

tcggtgaccg ccggcgaggg cagcgtgtcc aagggccgca gcctggtctc cgccggcaag 540

ccgccgctgt ccgggcacaa gagaaagccc tgcgccggcg cgcactcgga ggccaacacc 600

accggcagcc gatgccactg ctccaagaga aggaagaacc gcgtgaagac gacggtgagg 660

gtgcccgcgg tgagcgcgaa gatcgccgac atcccgccgg acgagtactc gtggaggaag 720

tacggccaga agcccatcaa gggatcccct tacccacggg gtactacaag tgcagcacag 780

tgcgggggtg cccggcgcgg gaagcacgtg gagcgcgccc tggacgaccc ggcgatgctg 840

gtggtgacct acgagggcgg agcaccgcca ctcgccgggg ccgatgccga tgcagatggc 900

gccgtcgcct atgccgatgc cgatgggcgc tcccgtcgcc gtagctag 948

<210> 58
<211> 315
<212> PRT
<213> Triticum aestivum

<400> 58
   Met Ala Val Asp Leu Met Gly Cys Tyr Thr Pro Arg Arg Ala Asp Asp
   1               5                   10                  15
   Gln Leu Ala Ile Gln Glu Ala Ala Thr Ala Gly Leu Arg Ser Leu Glu
               20                  25                  30
   Leu Leu Val Ser Ser Leu Ser Gly Ala Ala Pro Ser Lys Ala Pro Gln
           35                  40                  45
   Gln His Pro Gln Gln Pro Phe Gly Glu Ile Ala Asp Gln Ala Val Ser
       50                  55                  60
   Lys Phe Arg Lys Val Ile Ser Ile Leu Asp Arg Thr Gly His Ala Arg
   65                  70                  75                  80
   Phe Arg Arg Gly Pro Val Gln Ser Pro Thr Pro Pro Pro Pro Ala Pro
                   85                  90                  95
   Val Ala Pro Pro Pro Pro Pro Pro Arg Pro Leu Ala Val Val Glu Pro
               100                 105                 110
   Ala Arg Pro Ala Pro Leu Thr Val Val Ala Pro Val Ser Val Ala Ala
           115                 120                 125
   Pro Val Pro Gln Pro Gln Ser Leu Thr Leu Asp Phe Thr Lys Pro Asn
       130                 135                 140
   Leu Thr Met Ser Gly Ala Thr Ser Val Thr Ser Thr Ser Phe Phe Ser
   145                 150                 155                 160
   Ser Val Thr Ala Gly Glu Gly Ser Val Ser Lys Gly Arg Ser Leu Val
                   165                 170                 175
   Ser Ala Gly Lys Pro Pro Leu Ser Gly His Lys Arg Lys Pro Cys Ala
               180                 185                 190
   Gly Ala His Ser Glu Ala Asn Thr Thr Gly Ser Arg Cys His Cys Ser
           195                 200                 205
   Lys Arg Arg Lys Asn Arg Val Lys Thr Thr Val Arg Val Pro Ala Val
       210                 215                 220
   Ser Ala Lys Ile Ala Asp Ile Pro Pro Asp Glu Tyr Ser Trp Arg Lys
   225                 230                 235                 240
   Tyr Gly Gln Lys Pro Ile Lys Gly Ser Pro Tyr Pro Arg Gly Thr Thr
                   245                 250                 255
   Ser Ala Ala Gln Cys Gly Gly Ala Arg Arg Gly Lys His Val Glu Arg
               260                 265                 270
   Ala Leu Asp Asp Pro Ala Met Leu Val Val Thr Tyr Glu Gly Gly Ala
           275                 280                 285
   Pro Pro Leu Ala Gly Ala Asp Ala Asp Ala Asp Gly Ala Val Ala Tyr
       290                 295                 300
   Ala Asp Ala Asp Gly Arg Ser Arg Arg Arg Ser
   305                 310                 315

<210>  59
<211>  987
<212>  DNA
<213>  Triticum aestivum

<400>  59
atggccgtgg acctcatggg ctgctacacc cctcgccgcg ccgacgacca gctcgccatc      60

caggaggccg ccaccgccgg cctccgcagc ctggagctcc tcgtctcctc cctctccggc     120

gcggcgccgt ccaaggcgcc gcagcagcac ccgcagcagc cgttcggcga gatcgccgac     180

caggccgtct ccaagttccg caaggtgatc tccatcctcg accgcaccgg ccacgcccgc     240

ttccgccgcg gccccgtcca gtcgcctacc ccgcctcctc cggctccggt cgctcctccg     300

ccgcccccac cgcgccctct ggccgtcgtc gagccggcca ggcccgctcc cttgaccgtc     360

gtggcgccgg tgtcggtggc cgccccggtc cctctcccgc agccgcagag cctgacgctg     420

gacttcacca agccgaacct gaccatgtca ggcgcgacgt ccgtgacgtc cacgtccttc 480

ttctcgtcgg tgaccgccgg cgagggcagc gtgtccaagg gccgcagcct ggtctccgcc 540

ggcaagccgc cgctgtccgg gcacaagaga aagccgtgcg ccggcgcgca ctcggaggcc 600

aacaccaccg gcagccgatg ccactgctcc aagagaagga agaaccgcgt gaagacgacg 660

gtgagggtgc ccgcggtgag cgcgaagatc gccgacatcc cgccggacga gtactcgtgg 720

aggaagtacg gccagaagcc catcaaggga tccccttacc cacggggcta ctacaagtgc 780

agcacagtgc gaggggcccc ggcgcggaag cacgtggagc gccccctgga cgacccggcg 840

atcctggtgg tgacgtacga gggcgagcac cgccactccc cggggccgat gccgatgcag 900

atggcgccgt cgccggtgcc gattccgatg ccgatgggcg cgcccgtagc cgtagctagt 960

gtgtccgccg gcaacgggca cgtctga 987

<210> 60
<211> 328
<212> PRT
<213> Triticum aestivum

<400> 60

Met Ala Val Asp Leu Met Gly Cys Tyr Thr Pro Arg Arg Ala Asp Asp
1               5                   10                  15

Gln Leu Ala Ile Gln Glu Ala Ala Thr Ala Gly Leu Arg Ser Leu Glu
            20                  25                  30

Leu Leu Val Ser Ser Leu Ser Gly Ala Ala Pro Ser Lys Ala Pro Gln
        35                  40                  45

Gln His Pro Gln Gln Pro Phe Gly Glu Ile Ala Asp Gln Ala Val Ser
    50                  55                  60

Lys Phe Arg Lys Val Ile Ser Ile Leu Asp Arg Thr Gly His Ala Arg
65                  70                  75                  80

Phe Arg Arg Gly Pro Val Gln Ser Pro Thr Pro Pro Pro Pro Ala Pro
                85                  90                  95

Val Ala Pro Pro Pro Pro Pro Pro Arg Pro Leu Ala Val Val Glu Pro
            100                 105                 110

Ala Arg Pro Ala Pro Leu Thr Val Val Ala Pro Val Ser Val Ala Ala
        115                 120                 125

Pro Val Pro Leu Pro Gln Pro Gln Ser Leu Thr Leu Asp Phe Thr Lys
    130                 135                 140

Pro Asn Leu Thr Met Ser Gly Ala Thr Ser Val Thr Ser Thr Ser Phe
145                 150                 155                 160

Phe Ser Ser Val Thr Ala Gly Glu Gly Ser Val Ser Lys Gly Arg Ser
                165                 170                 175

Leu Val Ser Ala Gly Lys Pro Pro Leu Ser Gly His Lys Arg Lys Pro
            180                 185                 190

Cys Ala Gly Ala His Ser Glu Ala Asn Thr Thr Gly Ser Arg Cys His
        195                 200                 205

Cys Ser Lys Arg Arg Lys Asn Arg Val Lys Thr Thr Val Arg Val Pro
    210                 215                 220

Ala Val Ser Ala Lys Ile Ala Asp Ile Pro Pro Asp Glu Tyr Ser Trp
225                 230                 235                 240

Arg Lys Tyr Gly Gln Lys Pro Ile Lys Gly Ser Pro Tyr Pro Arg Gly
                245                 250                 255

Tyr Tyr Lys Cys Ser Thr Val Arg Gly Gly Pro Ala Arg Lys His Val
            260                 265                 270

Glu Arg Pro Leu Asp Asp Pro Ala Ile Leu Val Val Thr Tyr Glu Gly
        275                 280                 285

Glu His Arg His Ser Pro Gly Pro Met Pro Met Gln Met Ala Pro Ser
    290                 295                 300

Pro Val Pro Ile Pro Met Pro Met Gly Ala Pro Val Ala Val Ala Ser
305                 310                 315                 320

Val Ser Ala Gly Asn Gly His Val
                325

<210>  61
<211>  945
<212>  DNA
<213>  Triticum aestivum

<400>  61
atggccgtgg acctcatggg ctgctacacc cctcgccgcg ccgacgacca gctcgccatc      60

caggaggccg ccaccgccgg cctccgcagc ctggagctcc tggtctcctc cctctccggc     120

gcggcgccgt ccaaggcgcc ccagcagcac ccgcagcagc cgttcggcga gatcgccgac     180

caggccgtct ccaagttccg caaggtgatc tccatcctcg accgcaccgg tcacgcccgc     240

ttccgccgcg gccccgtcca gtcggcgccg ccgccgccgc ctcctccagc accggtcgct 300

ccacctcctc ccccaccttt gaccgtcgtg gcgccggtgt cggtggccgc cccgctcccg 360

cagccgcaga gcctgacgct ggacttcacc aagccgaacc tgaccatgtc gggcgcgacg 420

tccgtgacat ccacgtcctt cttctcttcg gtgaccgccg gcgagggcag cgtgtccaag 480

ggccggagcc tggtctccgc cggcaagccg ccgctgtccg ggcacaagag aaagccctgc 540

gccggcgcgc actccgaggc caacaccacc ggcagccgat gccactgctc caagagaagg 600

aagaaccgcg tgaagacaac ggtgagggtg ccggcggtga gcgcgaaaat cgccgacatc 660

ccgccggacg agtactcgtg gaggaagtac ggccagaagc ccatcaaggg atccccttac 720

ccacgggggc tactacagtg cagcacagtg cgagggtgcc ctgcccggaa gcacgtggag 780

cgcgccctgg acgacccgcc gatgctggtg gtgacttacg agggcgtcgc acgccactcg 840

ccggggccga tgccgatgca gattgcggcg tcgccgatgc caatgcgatg ggcgctccgt 900

agccgtgcta gtgtgtccgc gggcacgggc agtctgacta gttaa 945

<210> 62
<211> 314
<212> PRT
<213> Triticum aestivum

<400> 62

Met Ala Val Asp Leu Met Gly Cys Tyr Thr Pro Arg Arg Ala Asp Asp
1               5                   10                  15

Gln Leu Ala Ile Gln Glu Ala Ala Thr Ala Gly Leu Arg Ser Leu Glu
            20                  25                  30

Leu Leu Val Ser Ser Leu Ser Gly Ala Ala Pro Ser Lys Ala Pro Gln
        35                  40                  45

Gln His Pro Gln Gln Pro Phe Gly Glu Ile Ala Asp Gln Ala Val Ser
    50                  55                  60

Lys Phe Arg Lys Val Ile Ser Ile Leu Asp Arg Thr Gly His Ala Arg
65                  70                  75                  80

Phe Arg Arg Gly Pro Val Gln Ser Ala Pro Pro Pro Pro Pro Pro Pro
                85                  90                  95

Ala Pro Val Ala Pro Pro Pro Pro Pro Pro Leu Thr Val Val Ala Pro
            100                 105                 110

Val Ser Val Ala Ala Pro Leu Pro Gln Pro Gln Ser Leu Thr Leu Asp
        115                 120                 125

Phe Thr Lys Pro Asn Leu Thr Met Ser Gly Ala Thr Ser Val Thr Ser
    130                 135                 140

Thr Ser Phe Phe Ser Ser Val Thr Ala Gly Glu Gly Ser Val Ser Lys
145                 150                 155                 160

Gly Arg Ser Leu Val Ser Ala Gly Lys Pro Pro Leu Ser Gly His Lys
                165                 170                 175

Arg Lys Pro Cys Ala Gly Ala His Ser Glu Ala Asn Thr Thr Gly Ser
            180                 185                 190

Arg Cys His Cys Ser Lys Arg Arg Lys Asn Arg Val Lys Thr Thr Val
        195                 200                 205

Arg Val Pro Ala Val Ser Ala Lys Ile Ala Asp Ile Pro Pro Asp Glu
    210                 215                 220

Tyr Ser Trp Arg Lys Tyr Gly Gln Lys Pro Ile Lys Gly Ser Pro Tyr
225                 230                 235                 240

Pro Arg Gly Leu Leu Gln Cys Ser Thr Val Arg Gly Cys Pro Ala Arg
                245                 250                 255

Lys His Val Glu Arg Ala Leu Asp Asp Pro Pro Met Leu Val Val Thr
            260                 265                 270

Tyr Glu Gly Val Ala Arg His Ser Pro Gly Pro Met Pro Met Gln Ile
        275                 280                 285

Ala Ala Ser Pro Met Pro Met Arg Trp Ala Leu Arg Ser Arg Ala Ser
    290                 295                 300

Val Ser Ala Gly Thr Gly Ser Leu Thr Ser
305                 310

<210>  63
<211>  978
<212>  DNA
<213>  Secale cereale

<400>  63
atggccgtgg acctcatggg ctgctacacc cctcgccgcg ccgacgacca gctcgccatc      60

caggaggccg ccaccgccgg cctccgcagc ctggagctcc tggtctcctc cctctccggc     120

gcggcgccgt ccaaggcgcc gcagcagcac cagcagcagt tcggcgagat cgccgaccag     180

gccgtctcca agttccgcaa ggtgatctcc atcctcgacc gcaccggaca cgcccgcttc     240

cgccgcggcc ccgtccagtc cccgccgccg cctcctcctc cagctccggt cgctcctccc 300

ccgccaccac cgcgccctct ggccatcgag ccagccaggc ccgctccctt gaccgtcgtg 360

gcgccggtgt cggtggccgc cccggtcccg cagccgcaga gcctgacgct ggacttcacc 420

aagccgaacc tgaccatgtc gggcgcgacg tccgtgacgt ccacgtcctt cttctcgtcg 480

gtgaccgccg gcgagggcag cgtgtccaag ggccgcagcc tggtctccgc cggcaagccg 540

ccgctgtccg ggcacaagag aaagccctgc gcgggcgcgc actcggaggc caacaccacg 600

ggcagccgat gccactgctc caagagaagg aagaaccggg tgaagacgac ggtgagggtg 660

ccggcggtga gcgcgaagat cgccgacatc ccggcggacg agtactcgtg gaggaagtac 720

gggcagaagc ccatcaaggg atccccatac ccacggggct actacaagtg cagcaccgtg 780

cgagggtgcc cggcccggaa gcacgtggag cgcgccctgg acgacccggc gatgctggtg 840

gtgacctacg agggcgagca ccgccactcg ccggcgccga tgccgatgcc gatgcagatg 900

gccccgtcgc cggtgccgat gccgatgggc gctcccgtag ccgtagctag tgtgtccgcc 960

ggcaacgggc acgtctga 978

<210> 64
<211> 325
<212> PRT
<213> Secale cereale

<400> 64

Met Ala Val Asp Leu Met Gly Cys Tyr Thr Pro Arg Arg Ala Asp Asp
1               5                   10                  15

Gln Leu Ala Ile Gln Glu Ala Ala Thr Ala Gly Leu Arg Ser Leu Glu
            20                  25                  30

Leu Leu Val Ser Ser Leu Ser Gly Ala Ala Pro Ser Lys Ala Pro Gln
        35                  40                  45

Gln His Gln Gln Gln Phe Gly Glu Ile Ala Asp Gln Ala Val Ser Lys
    50                  55                  60

Phe Arg Lys Val Ile Ser Ile Leu Asp Arg Thr Gly His Ala Arg Phe
65                  70                  75                  80

Arg Arg Gly Pro Val Gln Ser Pro Pro Pro Pro Pro Pro Pro Ala Pro
                85                  90                  95

Val Ala Pro Pro Pro Pro Pro Pro Arg Pro Leu Ala Ile Glu Pro Ala
            100                 105                 110

Arg Pro Ala Pro Leu Thr Val Val Ala Pro Val Ser Val Ala Ala Pro

115                 120                 125

Val Pro Gln Pro Gln Ser Leu Thr Leu Asp Phe Thr Lys Pro Asn Leu
    130                 135                 140

Thr Met Ser Gly Ala Thr Ser Val Thr Ser Thr Ser Phe Phe Ser Ser
145                 150                 155                 160

Val Thr Ala Gly Glu Gly Ser Val Ser Lys Gly Arg Ser Leu Val Ser
                165                 170                 175

Ala Gly Lys Pro Pro Leu Ser Gly His Lys Arg Lys Pro Cys Ala Gly
            180                 185                 190

Ala His Ser Glu Ala Asn Thr Thr Gly Ser Arg Cys His Cys Ser Lys
        195                 200                 205

Arg Arg Lys Asn Arg Val Lys Thr Thr Val Arg Val Pro Ala Val Ser
    210                 215                 220

Ala Lys Ile Ala Asp Ile Pro Ala Asp Glu Tyr Ser Trp Arg Lys Tyr
225                 230                 235                 240

Gly Gln Lys Pro Ile Lys Gly Ser Pro Tyr Pro Arg Gly Tyr Tyr Lys
                245                 250                 255

Cys Ser Thr Val Arg Gly Cys Pro Ala Arg Lys His Val Glu Arg Ala
            260                 265                 270

Leu Asp Asp Pro Ala Met Leu Val Val Thr Tyr Glu Gly Glu His Arg
        275                 280                 285

His Ser Pro Ala Pro Met Pro Met Pro Met Gln Met Ala Pro Ser Pro
    290                 295                 300

Val Pro Met Pro Met Gly Ala Pro Val Ala Val Ala Ser Val Ser Ala
305                 310                 315                 320

Gly Asn Gly His Val
                325

<210>  65
<211>  97
<212>  DNA
<213>  Artificial Sequence

<220>
<223>  TaWRKY10 Fragment A

<400>  65
actgaactga gctactgatc gatcagtacg agtacgtagc tgtggttagt tttatgatcg      60

attacggttc agaaatctca cggatgcatg tacacga                               97


<210>  66
<211>  164
<212>  DNA
<213>  Artificial Sequence

<220>
<223>  TaWRKY13/46 Fragment A

<400>  66
ggtcacttac ttcaaccatc actcgaggag ggcggtgtcc atctgcttcg ggtcgcatgc      60

caccggcgag cagccgacat tcctaacgtc gccgggcaca ctgcagtcac cagcgagcac     120

aaccaaccaa cagaacgaca gaggcgctta cggtcaccag tttc                      164


<210>  67
<211>  174
<212>  DNA
<213>  Artificial Sequence

<220>
<223>  TaWRKY13/46 Fragment B

<400>  67
cttacggtca ccagtttcag tggacagaca catcaccgtc ggcaggcgat gccccagtca      60

agatggagac cgacagtctc gccggaacag gcgcttcgtc tggcgctgct agcggccatg     120

ctctgtcgag gacactgctg ccgatcggcc agtcgagatg catcgagtac tttc           174


<210>  68
<211>  99
<212>  DNA
<213>  Artificial Sequence

<220>
<223>  TaWRKY19 Fragment A

<400>  68
ttggtccccg tgtcttcttt accgaccacg gtggtggctc gcgaaagaaa gaaagaaaca      60

caattcgatt ggttcttcgg tgacggctgg ttattgctc                             99


<210>  69
<211>  83
<212>  DNA
<213>  Artificial Sequence

<220>
<223>  TaWRKY19 Fragment B

<400>  69
tgctctgttt gctgtatatt ccccactcca gtaataactc ttgcatatgc agaaattgta      60

actgttgaac atgatgggtg gtc                                              83

<210>  70
<211>  105
<212>  PRT
<213>  Artificial Sequence

<220>
<223>  TaWRKY29 Fragment A

<400>  70

Thr Gly Thr Thr Gly Thr Gly Ala Gly Cys Cys Gly Thr Ala Cys Ala
1               5                   10                  15

Ala Ala Cys Ala Thr Ala Ala Thr Thr Gly Ala Gly Ala Cys Ala Ala
            20                  25                  30

Thr Gly Thr Thr Thr Thr Cys Ala Thr Thr Gly Thr Cys Ala Gly Thr
        35                  40                  45

Ala Gly Thr Thr Gly Thr Thr Thr Thr Ala Gly Gly Ala Gly Gly Ala
    50                  55                  60

Ala Ala Ala Cys Ala Thr Cys Thr Thr Gly Gly Thr Thr Thr Thr Gly
65                  70                  75                  80

Gly Ala Thr Ala Ala Thr Gly Gly Ala Ala Ala Thr Cys Gly Ala Ala
                85                  90                  95

Gly Ala Gly Gly Gly Thr Gly Cys Thr
            100                 105

<210>  71
<211>  70
<212>  DNA
<213>  Artificial Sequence

<220>
<223>  TaWRKY53B Fragment A

<400>  71
ccgcggccct tcgttccccc tgtggcgttt acatgtgggt ccacgtgtac aatataatag      60

atgctacatg                                                             70

<210>  72
<211>  80
<212>  PRT
<213>  Artificial Sequence

<220>
<223>  TaWRKY68A Fragment A

<400>  72

Thr Thr Cys Gly Thr Thr Cys Gly Cys Thr Cys Gly Cys Ala Ala Thr
1               5                   10                  15

Cys Thr Gly Thr Cys Thr Gly Ala Thr Gly Thr Cys Cys Gly Thr Gly
            20                  25                  30

Thr Ala Ala Ala Gly Ala Ala Gly Ala Ala Gly Ala Ala Ala Gly Ala
        35                  40                  45

Thr Cys Gly Thr Ala Gly Ala Gly Ala Gly Ala Cys Gly Gly Ala Gly
    50                  55                  60

Ala Gly Gly Gly Ala Ala Ala Cys Thr Thr Ala Ala Thr Gly Cys Cys
65                  70                  75                  80

## Description

### TECHNICAL FIELD

The present disclosure relates generally to the field of plant biotechnology, and more specifically to the field of cereal pathogen resistance. In particular, the present disclosure relates to novel means and methods for providing pathogen resistance to cereals based on the identification of key WRKYs whose expression or activity is fertilizer-responsive and/or which are characterized by the amino acid sequence motif D-G/E-X₁-X₂-W-R-K-Y-G-K/E/Q-K/E, wherein X₁ is a hydrophobic amino acid and X₂ is a polar, non-hydrophobic amino acid.

### BACKGROUND

The foundation of global food security is built on the three cereals wheat, rice and maize, where wheat is the leading source of vegetable protein in human food. In the UK for example this cereal is the most important crop grown with an annual value of about £1.2 billion. The average yield of wheat in the field in the UK is currently 8.4 tonnes/ha, but this yield is dependent on high levels of mineral fertilizer (especially Nitrogen, Phosphorus and Potassium) and pesticide usage.

Nitrogen (N) fertilizer use is of concern because it is associated both with high levels of energy use and greenhouse gas emissions (e.g. CO2, N2O) that cause climate change, in addition to eutrophication of fresh water and marine ecosystems. However, nitrogen fertilization is required for achieving high yield in wheat and other cereals, and increasing sustainability through decrease in nitrogen input is not commercially feasible due to the resulting fall in yield. Given that high-nitrogen nutrient regimes are reality in the field, decreasing pesticide usage is a target for enhancing sustainability of production of cereals, in particular wheat.

*Zymoseptoria tritici* is the fungal pathogen that causes Septoria leaf blotch disease (STB) in wheat. STB disease is the most economically important foliar disease of winter wheat in most western European countries. The disease is characterized by necrotic blotches on the foliage which significantly reduces the plants ability to efficiently grow and produce grain. There is increasing evidence that high soil nitrogen and other mineral fertilizers have the undesirable side-effect of enhancing the development of fungal diseases including Septoria (Simón M. R., et al. Influence of nitrogen supply on the susceptibility of wheat to Septoria tritici. Journal of Phytopathology 151.5 (2003): 283-289.; Loyce Chantal, et al. Interaction between cultivar and crop management effects on winter wheat diseases, lodging, and yield. Crop protection 27.7 (2008): 1131-1142). The mechanisms leading to the above mentioned nutrient induced changes in disease development are largely unknown.

*Zymoseptoria tritici* are Ascomycete pycnidia-producing fungi which unlike most other plant pathogens, infects through stomata rather than by direct penetration. The principle method of spread from leaf to leaf in the field is via rain splash. The disease is characterized by a long latent period of approximately two weeks during which the fungus manages to evades the host defences. Following the latent phase, there is a rapid switch to necrotrophy immediately prior to symptom expression.

Despite its importance as a disease, there is very little information available on the defense mechanisms or immune responses that allow cereals such as wheat to counter *Septoria* infection. Fungicides provide the only control measure for this devastating disease, but in addition to the high cost, the currently available fungicides are becoming less effective against new resistant strains of *Septoria.*

Susceptible varieties of wheat will be susceptible to the disease throughout the life cycle whenever there are healthy green leaves available for the pathogen to colonise. Examples where virulence is restricted to a particular development stage (i.e. adult or seedling stage) do exist but these are limited to isolate specific major gene relationships which are of limited interest to breeding for quantitative, durable disease resistance (Kema, Gert HJ, and Cor H. van Silfhout. Genetic variation for virulence and resistance in the wheat-Mycosphaerella graminicola pathosystem III. Comparative seedling and adult plant experiments. Phytopathology 87.3 (1997): 266-272). Growth stage 39 (GS39) on the Zadok scale, when the flag leaf is fully emerged is agronomically the most important stage since the flag leaf is the principle driver of yield; necrosis on the flag leaf reduces its photosynthetic capacity and consequently overall yield potential is reduced.

There is no other nutrient as important as nitrogen (N) to drive cereal yields, in particular wheat yields. Nitrogen and along with phosphorus (P) and potassium (K), make up the three macronutrients required for growth. A typical nitrogen fertilization regime for UK winter wheat may mean an application of ~200kg/ha of nitrogen, typically applied in a single or split application when the plants reach their most active growth in the spring (GS31, around mid April in Eastern UK), when the plants make the physiological switch between vegetative and reproductive development. Additional, smaller applications are often made for bread wheat crops later in development in order to boost protein yield.

A number of reports have linked increased nitrogen availability with higher susceptibility to S. *tritici* (Shipton, W. A., et al. The common Septoria diseases of wheat. The Botanical Review 37.2 (1971): 231-262; Prew, R. D., et al. Effects of eight factors on the growth and nutrient uptake of winter wheat and on the incidence of pests and diseases. The Journal of Agricultural Science 100.02 (1983): 363-382). The mechanisms leading to these nutrient induced changes in disease development are currently not known, although additional studies have suggested that this could be due to the increased nutritional status of the host tissue studies (Leitch, M. H., and P. D. Jenkins. Influence of nitrogen on the development of Septoria epidemics in winter wheat. The Journal of Agricultural Science 124.03 (1995): 361-368; Simón et al., 2003). In contrast, several studies have found that high nitrogen actually reduces disease severity (Gooding, M. J., and W. P. Davies. Foliar urea fertilization of cereals: a review. Fertilizer Research 32.2 (1992): 209-222; Johnston, H. W., J. A. MacLeod, and K. S. Clough. Effects of cycocel (CCC) and fungicide sprays on spring wheat grown at three nitrogen levels. Canadian Journal of Plant Science 59.4 (1979): 917-929; Broscious, S. C., J. A. Frank, and J. R. Frederick. Influence of winter wheat management practices on the severity of powdery mildew and Septoria blotch in Pennsylvania. Phytopathology 75.5 (1985): 538-542), although these studies were limited in scope or were only showing a partial effect. A more recent study investigated the effects of different fungicides in combination to different N rates on the severity of foliar diseases, senescence and yield (Ishikawa S. M. C. Hare and P. S. Kettlewell (2012). Effects of strobilurin fungicide programmes and fertilizer nitrogen rates on winter wheat: severity of Septoria tritici, leaf senescence and yield. The Journal of Agricultural Science, 150, pp 411-426. doi:10.1017/S0021859611000670). However, the authors were unable to draw any robust conclusions due to the limited data set available.

A single study has suggested that potassium fertilization was positively correlated with disease resistance (Arabi, M. I. E., N. MirAli, and M. Jawhar. Effect of foliar and soil potassium fertilisation on wheat yield and severity of Septoria tritici blotch. Australasian plant pathology 31.4 (2002): 359-362), with the effects being genotype dependent. A study by Paraschivu et al, (Influence of nitrogen and phosphorus on the risk of epidemics by septoria tritici in winter wheat. Annals of the University of Craiova-Agriculture, Montanology, Cadastre Series 41.2 (2011): 215-219), suggested that the increase in disease severity seen under high nitrogen can be limited by increasing phosphorus fertilization.

In short, there is less known about the mechanisms leading to the above mentioned nutrient induced changes in disease development. Therefore the objective of the present disclosure is to provide means and methods for providing or boosting pathogen resistance to cereals, in particular resistance against fungal pathogens such as *Septoria,* under fertilizer input growth conditions necessary for maintaining yield.

### SUMMARY OF THE DISCLOSURE

The present disclosure provides means and methods for conferring pathogen resistance to, or increasing pathogen resistance of, a cereal plant cell, or a cereal plant or part thereof, comprising inhibiting the expression or activity of the at least one WRKY protein in a cereal plant cell, or a cereal plant or part thereof, as compared to a corresponding control plant cell, or control plant or part thereof, lacking the inhibition of the expression or activity of the at least one WRKY protein, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In particular, the present disclosure provides a cereal plant cell having introduced into its genome at least one modification inhibiting the expression or activity of at least one WRKY protein as compared to a corresponding control plant cell lacking the at least one modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid, preferably wherein the at least one modification is introduced into the at least one endogenous WRKY gene encoding the at least one WRKY protein..

Aspects and embodiments encompassed by the present invention include the following, without being limited thereto:
[1] A cereal plant cell having introduced into its genome at least one modification inhibiting the expression or activity of at least one WRKY protein as compared to a corresponding control plant cell lacking the at least one modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid, preferably wherein the at least one modification is introduced into the at least one endogenous WRKY gene encoding the at least one WRKY protein.
[2] The plant cell of item [1], wherein the at least one WRKY protein is a negative regulator of the plant cell defense mechanism against a pathogen, preferably against a fungal pathogen.
[3] The cereal plant cell of item [1] or [2], wherein the amino acid sequence motif is D-G/E-X1-X2-W-R-K-Y-G-K/E/Q-K/E-X-X-X3-X-X2 shown in SEQ ID NO: 2, wherein X is any amino acid, X1 is a hydrophobic amino acid, X2 is a polar, non-hydrophobic amino acid, and X3 is a polar, non-aromatic amino acid.
[4] The plant cell of any one of items [1]-[3], wherein the cereal plant cell is the cell of a wheat plant (*Triticum aestivum*), the cell of a rye plant (*Secale cereale*), the cell of a barley plant (*Hordeum vulgare*), or the cell of a maize plant (*Zea mays*).
[5] The cereal plant cell of any one of items [1]-[4], wherein the at least one WRKY gene is selected from the group consisting of TaWRKY10 (SEQ ID NO: 3), TaWRKY13 (SEQ ID NO: 5), TaWRKY19 (SEQ ID NO: 7), TaWRKY29 (SEQ ID NO: 9), TaWRKY39 (SEQ ID NO: 11), TaWRKY53B (SEQ ID NO: 13), and TaWRKY68A (SEQ ID NO: 15), or homologs thereof.
[6] The cereal plant cell of any one of items [1]-[5], wherein the at least one WRKY protein is selected from the group consisting of TaWRKY10 (SEQ ID NO: 4), TaWRKY13 (SEQ ID NO: 6), TaWRKY19 (SEQ ID NO: 8), TaWRKY29 (SEQ ID NO: 10), TaWRKY39 (SEQ ID NO: 12), TaWRKY53B (SEQ ID NO: 14), and TaWRKY68A (SEQ ID NO: 16), or homologs thereof.
[7] The cereal plant cell of any one of items [1]-[6], wherein the at least one modification results in increased pathogen resistance of a cereal plant comprising said cereal plant cell as compared to a control plant, preferably wherein the at least one modification results in an increased resistance against a fungal pathogen.
[8] The cereal plant cell of any one of items [1]-[7], wherein the at least one modification is caused by RNA interference, sense suppression, insertional mutagenesis, mutagenesis or genome editing of the endogenous WRKY gene.
[9] The cereal plant cell of any one of items [1]-[8], wherein the at least one modification comprises one or more transposons, one or more insertions, one or more additions, one or more deletions or one or more point mutations in the at least one endogenous WRKY gene.
[10] The cereal plant cell of any one of items [1]-[9], wherein the at least one modification is introduced into the cereal plant cell by
   (a) introducing at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), or (ii) at least 20 contiguous nucleotides of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, into the plant cell such that the at least one polynucleotide sequence is operatively linked to a promoter in a sense or antisense orientation; or
   (b) introducing at least one polynucleotide sequence comprising one or more subsequences of a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), into the plant cell, wherein the at least one polynucleotide sequence is configured for RNA interference, preferably wherein the at least one polynucleotide sequence is configured for endogenous (post transcriptional) gene silencing or virus-induced gene silencing.
[11] The cereal plant cell of any one of items [1]-[10], which is in a plant comprising a phenotype of increased pathogen resistance, preferably a phenotype of increased resistance against a fungal pathogen.
[12] The cereal plant cell of any one of items [2], [7] or [11], wherein the fungal pathogen is *Zymoseptoria tritici.*
[13] A cereal plant containing the cereal plant cell of any one of items [1]-[12].
[14] A cereal plant having an increased pathogen resistance, preferably having an increased resistance against a fungal pathogen, wherein the increased pathogen resistance results from at least one modification introduced into the genome of the plant inhibiting the expression or activity of at least one WRKY protein as compared to a corresponding control plant lacking the at least one modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid, preferably wherein the at least one modification is introduced into the at least one endogenous WRKY gene encoding the at least one WRKY protein.
[15] The cereal plant of item [14], wherein the increased pathogen resistance results from:
   (a) a modification in at least one endogenous WRKY gene comprising a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), wherein the modification comprises one or more transposons, one or more insertions, one or more additions, one or more deletions or one or more point mutations in the at least one endogenous WRKY gene;
   (b) at least one introduced transgene comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), or (ii) at least 20 contiguous nucleotides of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, wherein the transgene is operatively linked to a promoter in a sense or antisense orientation; or (c) at least one introduced polynucleotide sequence comprising one or more subsequences of a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), wherein the at least one polynucleotide sequence is in RNA interference configuration, preferably wherein the at least one polynucleotide sequence is configured for endogenous (post transcriptional) gene silencing or virus-induced gene silencing.
[16] The cereal plant of item [15], wherein the plant is a hybrid cereal plant.
[17] The cereal plant of item [15b], wherein the promoter is a tissue-specific promoter, a temporally regulated promote, or an inducible promoter.
[18] A method for conferring pathogen resistance to, or increasing pathogen resistance of, a cereal plant cell, or a cereal plant or part thereof, the method comprising the step of inhibiting the expression or activity of at least one WRKY protein in a cereal plant cell, or a cereal plant or part thereof, as compared to a corresponding control plant cell, or plant or part thereof, lacking the modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid.
[19] A method of producing a cereal plant cell, or a cereal plant or part thereof, having an increased pathogen resistance as compared to a corresponding control plant cell, or plant or part thereof, the method comprising the step of inhibiting the expression or activity of at least one WRKY protein in a cereal plant cell, or a cereal plant or part thereof, as compared to a corresponding control plant cell, or plant or part thereof, lacking the modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid.
[20] The method of item 18 or 19, wherein the step of inhibiting the expression or activity of at least one WRKY protein comprises introducing into the genome of the cereal plant cell, or a cereal plant or part thereof, at least one modification inhibiting the expression and/or activity of the at least one WRKY protein as compared to a corresponding control plant cell, or a cereal plant or part thereof, lacking the at least one modification, preferably wherein the at least one modification is introduced into the at least one endogenous WRKY gene encoding the at least one WRKY protein.
[21] The method of any one of items [18]-[20], wherein the amino acid sequence motif is D-G/E-X1-X2-W-R-K-Y-G-K/E/Q-K/E-X-X-X3-X-X2 shown in SEQ ID NO: 2, wherein X is any amino acid, X1 is a hydrophobic amino acid, X2 is a polar, non-hydrophobic amino acid, and X3 is a polar, non-aromatic amino acid.
[22] The method of any one of items [18]-[21], wherein the WRKY protein is encoded by a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A).
[23] The method of any one of items [20]-[22], wherein the at least one modification comprises one or more transposons, one or more insertions, one or more additions, one or more deletions or one or more point mutations in the at least one endogenous WRKY gene.
[24] The method of any one of items [20]-[23], wherein the at least one modification is introduced into the cereal plant cell, or cereal plant or part thereof, by (a) introducing at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A) or (ii) at least 20 contiguous nucleotides of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, into the cereal plant cell, or cereal plant or part thereof, such that the at least one polynucleotide sequence is operatively linked to a promoter in a sense or antisense orientation; or (b) introducing at least one polynucleotide sequence comprising one or more subsequences of a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), into the plant cell, or plant or part thereof, wherein the at least one polynucleotide sequence is configured for RNA interference, preferably wherein the at least one polynucleotide sequence is configured for endogenous (post transcriptional) gene silencing or virus-induced gene silencing.
[25] A method of identifying a cereal plant cell, or a cereal plant or part thereof, having an increased pathogen resistance as compared to a corresponding control plant cell, or plant or part thereof, the method comprising the step of identifying a cereal plant cell, or a cereal plant or part thereof, wherein the expression or activity of at least one WRKY protein, is inhibited as compared to a corresponding control plant cell, or plant or part thereof, lacking the modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid.
[26] The method of item [25], wherein the amino acid sequence motif is D-G/E-X1-X2-W-R-K-Y-G-K/E/Q-K/E-X-X-X3-X-X2 shown in SEQ ID NO: 2, wherein X is any amino acid, X1 is a hydrophobic amino acid, X2 is a polar, non-hydrophobic amino acid, and X3 is a polar, non-aromatic amino acid.
[27] The method of item [25] or [26], wherein the WRKY protein is encoded by a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A).
[28] The method of any one of items [20]-[24], wherein the cereal plant cell, or cereal plant or part thereof, comprises a heterozygous or homozygous mutation in the at least one WRKY gene encoding the at least one WRKY protein.
[29] The method of item [28], wherein the mutant form of the the at least one WRKY gene is introduced by Agrobacterium-mediated transfer, electroporation, micro-projectile bombardment, or sexual cross.
[30] The method of any one of items [18]-[29], wherein the cereal plant cell, or cereal plant or part thereof, has an increased resistance against a fungal pathogen, preferably an increased resistance against Septoria leaf blotch disease (or *Septoria tritici blotch, STB*), Fusarium disease (*Fusarium sp.*) or rust diseases (*Puccinia sp.*).
[31] A cereal plant cell, or a cereal plant or part thereof, produced or identified by the method of any one of items [18]-[30].
[32] A recombinant DNA construct comprising a polynucleotide sequence, or a fragment thereof, operatively linked to a heterologous promoter, wherein the polynucleotide sequence, or a fragment thereof, produces upon expression in a cereal plant cell, or cereal a plant or part thereof: (a) a protein that interferes with the expression or activity of at least one endogenous WRKY protein; (b) an RNA molecule that suppresses the expression of at least one endogenous WRKY protein; or (c) a loss-of-function mutation in at least one endogenous WRKY gene, wherein the polynucleotide sequence comprises a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A) or a homolog thereof. An RNA molecule of (b) is for example a double-stranded RNA (dsRNA) molecule described in [40].
[33] A method of producing a (transgenic) cereal plant cell, or a cereal plant or part thereof, having an increased pathogen resistance as compared to a corresponding control plant cell, or plant or part thereof, the method comprising the steps of (a) incorporating into a cereal plant cell, or a cereal plant or part thereof, the recombinant DNA construct of item 32; and (b) selecting a cereal plant cell, or a cereal plant or part thereof, comprising the said recombinant DNA construct and having an increased pathogen resistance as compared to a corresponding control plant cell, or plant or part thereof, that does not comprise the said recombinant DNA construct.
[34] The method of item [33], wherein the cereal plant cell, or cereal plant or part thereof, has an increased resistance against a fungal pathogen, preferably an increased resistance against Septoria leaf blotch disease (*Septoria tritici blotch, STB*), Fusarium disease (*Fusarium sp.*) or rust diseases (*Puccinia sp.*).
[35] A cereal plant cell, or a cereal plant or part thereof, produced by the method of item [33] or [34].
[36] A cereal plant cell, or a cereal plant or part thereof, comprising the recombinant DNA construct of item [32] and having an increased pathogen resistance as compared to a corresponding control plant cell, or plant or part thereof, that does not comprise the said recombinant DNA construct.
[37] A method of producing a (transgenic) cereal plant having an increased pathogen resistance as compared to a corresponding control plant, the method comprising obtaining and planting seeds produced by the cereal plant of item [35] or [36], wherein the seed comprises the recombinant DNA construct of item [32], and wherein the plant grown from the planted seed is a progeny plant having an increased pathogen resistance as compared to a corresponding control plant that does not comprise the said recombinant DNA construct.
[38] The method of item [37], wherein the plant has an increased resistance against a fungal pathogen, preferably an increased resistance against Septoria leaf blotch disease (*Septoria tritici blotch, STB*), Fusarium disease (*Fusarium sp.*) or rust diseases (*Puccinia sp.*).
[39] A cereal plant produced by the method of item [37] or [38].
[40] A double-stranded RNA (dsRNA) molecule comprising: (a) a first strand comprising a nucleotide sequence having a length of at least 19, 20, 21, 22, 23, 24, 25, 30, 40 or 50 nucleotides, preferably at least 60, 70, 80, 90 or 100 nucleotides, more preferably at least 150, 200, 250, 300, 400, 500, 750 or 1000 nucleotides and having at least 90%, 91%, 92%, 93% or 94%, more preferably 95%, 96%, 97%, 98%, 99% or even 100% sequence identity to consecutive nucleotides of a target nucleic acid sequence; and (b) a second strand comprising in the 5' to 3' direction a nucleic acid sequence which is at least 90%, 91%, 92%, 93% or 94%, more preferably 95%, 96%, 97%, 98%, 99% or even 100% complementary to the first strand and/or is able to hybridize with the first strand, preferably under stringent conditions, wherein the target nucleic acid sequence is a coding region of an mRNA deduced from a nucleic acid sequence, or a complement thereof, comprising at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A).
[41] A composition comprising the dsRNA molecule of item 40.
[42] A method of regulating the expression of at least one WRKY gene in a cereal plant cell, or cereal plant or part thereof, the method comprising applying the composition of item [41] to a cereal plant cell, or cereal plant or part thereof, preferably wherein the composition is applied onto the surface of the plant cell, or plant or part thereof.
[43] A cereal plant cell, or a cereal plant or part thereof, produced by the method of item [42].
[44] A cereal plant cell, or a cereal plant or part thereof, comprising the dsRNA molecule of item [40] and having an increased pathogen resistance as compared to a corresponding control plant cell, or plant or part thereof, that does not comprise the said dsRNA molecule.
[45] Use of at least one polynucleotide sequence comprising a nucleic acid sequence, or a complement thereof, comprising (i) at least 70% sequence identity to a subsequence of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A) or a homolog thereof, as molecular marker for identifying a cereal plant cell, or a cereal plant or part thereof, having an increased pathogen resistance as compared to a corresponding control plant cell.
[46] A composition for topical application to a cereal plant cell, or a cereal plant or part thereof, comprising at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, wherein the at least one polynucleotide sequence is configured for RNA interference, preferably wherein the at least one polynucleotide sequence is configured for endogenous (post transcriptional) gene silencing or virus-induced gene silencing.
[47] Use of the recombinant DNA construct of item [32], the dsRNA molecule of item [40], or the composition of item [46], for conferring pathogen resistance to, or increasing pathogen resistance of, a cereal plant cell, or a cereal plant or part thereof.
[48] The cereal plant cell of any one of items [1]-[12], [31], [35]-[36], and [43]-[44], or the cereal plant or part thereof of any one of items [13]-[17], [31], [35]-[36], [39], and [43]-[44], or the method of any one of items [18]-[30], [33]-[34], [37]-[38], [40] and [42], or the composition of item [41], or the use of item [45] or [47], wherein the cereal plant cell, or cereal plant or part thereof, is a dicotyledonous or a monocotyledonous cereal plant cell, or cereal plant or part thereof, preferably wherein the cereal plant cell, or cereal plant or part thereof, is a wheat plant cell, or wheat plant or part thereof.
[49] The cereal plant cell of any one of items [1]-[12], [31], [35]-[36], and [43]-[44], or the cereal plant or part thereof of any one of items [13]-[17], [31], [35]-[36], [39], and [43]-[44], or the method of any one of items [18]-[30], [33]-[34], [37]-[38], [40] and [42], or the composition of item [41], or the use of item [45] or [47], wherein the WRKY gene or WRKY protein is a *Triticum aestivum* L. WRKY gene or *Triticum aestivum* L. WRKY protein, respectively.

The details of one or more embodiments of the disclosure are set forth in the accompanying drawings and the description below. Other features, objects, and advantages will be apparent from the description and drawings, and from the claims.

### BRIEF DESCRIPTION OF THE DRAWINGS

**FIGURE 1** shows sequence motifs (SEQ ID NOs: 1 and 2) common to WRKY proteins whose expression or activity is to be inhibited in accordance with the present disclosure.
**FIGURE 2** shows the nucleic acid and amino acid sequence of WRKY10 from *Triticum aestivum* (SEQ ID NO: 3 and SEQ ID NO: 4, respectively).
**FIGURE 3** shows the nucleic acid and amino acid sequence of WRKY13 from *Triticum aestivum* (SEQ ID NO: 5 and SEQ ID NO: 6, respectively).
**FIGURE 4** shows the nucleic acid and amino acid sequence of WRKY19 from *Triticum aestivum* (SEQ ID NO: 7 and SEQ ID NO: 8, respectively).
**FIGURE 5** shows the nucleic acid and amino acid sequence of WRKY29 from *Triticum aestivum* (SEQ ID NO: 9 and SEQ ID NO: 10, respectively).
**FIGURE 6** shows the nucleic acid and amino acid sequence of WRKY39 from *Triticum aestivum* (SEQ ID NO: 11 and SEQ ID NO: 12, respectively).
**FIGURE 7** shows the nucleic acid and amino acid sequence of WRKY53B from *Triticum aestivum* (SEQ ID NO: 13 and SEQ ID NO: 14, respectively).
**FIGURE 8** shows the nucleic acid and amino acid sequence of WRKY68A from *Triticum aestivum* (SEQ ID NO: 15 and SEQ ID NO: 16, respectively).

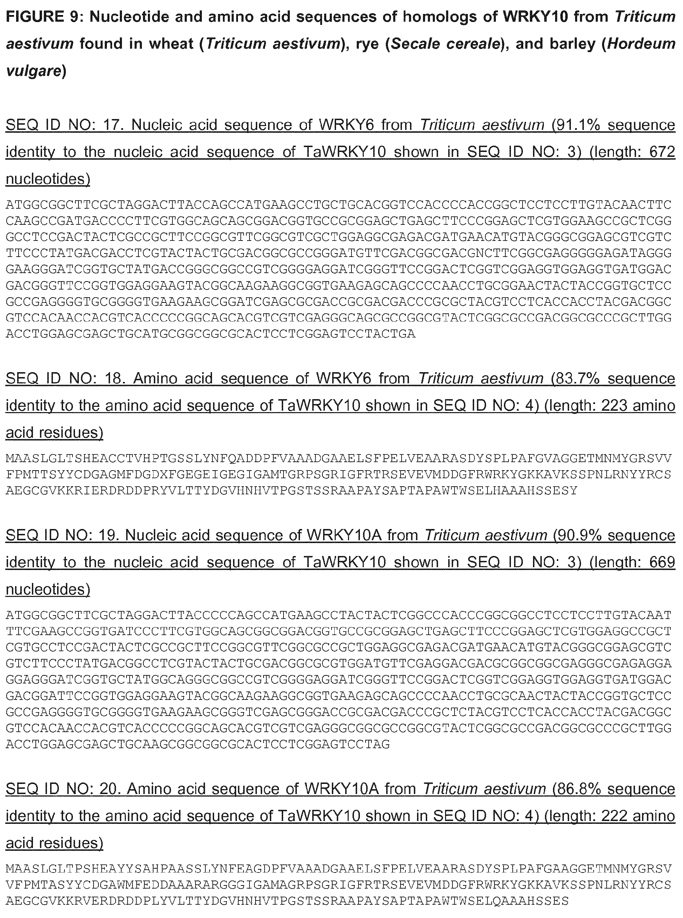

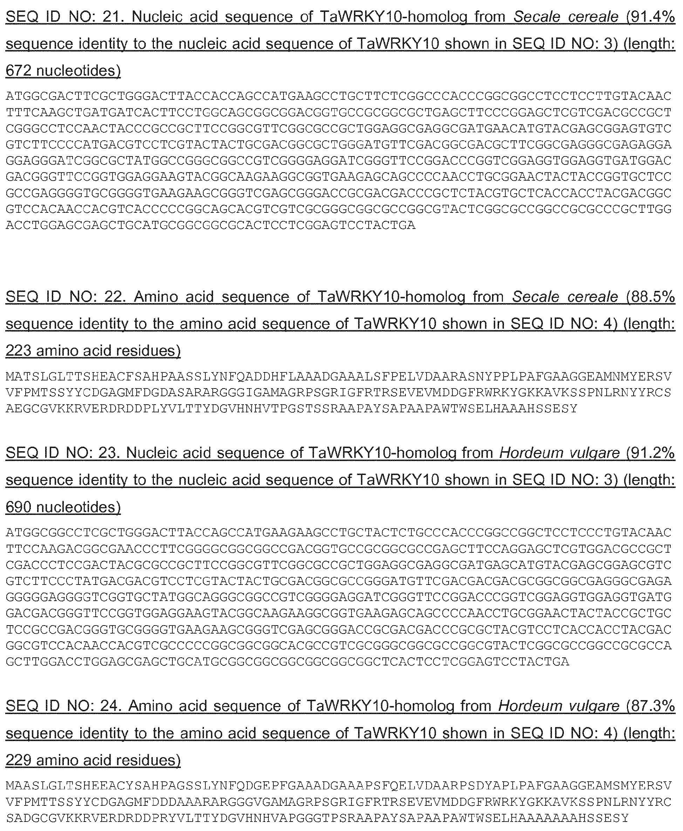

**FIGURE 9** shows nucleic acid and amino acid sequences of homologs of WRKY10 from *Triticum aestivum* (TaWRKY10) found in wheat *(Triticum aestivum),* rye (*Secale cereale*), and barley (*Hordeum vulgare*). SEQ ID NO: 17 shows the nucleic acid sequence of WRKY6 from *Triticum aestivum* having 91.1% sequence identity to the nucleic acid sequence of TaWRKY10 shown in SEQ ID NO: 3. SEQ ID NO: 18 shows the amino acid sequence of WRKY6 from *Triticum aestivum* having 83.7% sequence identity to the amino acid sequence of TaWRKY10 shown in SEQ ID NO: 4. SEQ ID NO: 19 shows the nucleic acid sequence of WRKY10A from *Triticum aestivum* having 90.9% sequence identity to the nucleic acid sequence of TaWRKY10 shown in SEQ ID NO: 3. SEQ ID NO: 20 shows the amino acid sequence of WRKY10A from *Triticum aestivum* having 86.8% sequence identity to the amino acid sequence of TaWRKY10 shown in SEQ ID NO: 4. SEQ ID NO: 21 shows the nucleic acid sequence of a TaWRKY10-homolog from *Secale cereale* having 91.4% sequence identity to the nucleic acid sequence of TaWRKY10 shown in SEQ ID NO: 3. SEQ ID NO: 22 shows the amino acid sequence of a TaWRKY10-homolog from *Secale cereale* having 88.5% sequence identity to the amino acid sequence of TaWRKY10 shown in SEQ ID NO: 4. SEQ ID NO: 23 shows the nucleic acid sequence of a TaWRKY10-homolog from *Hordeum vulgare* having 91.2% sequence identity to the nucleic acid sequence of TaWRKY10 shown in SEQ ID NO: 3. SEQ ID NO: 24 shows the amino acid sequence of a TaWRKY10-homolog from *Hordeum vulgare* having 87.3% sequence identity to the amino acid sequence of TaWRKY10 shown in SEQ ID NO: 4.

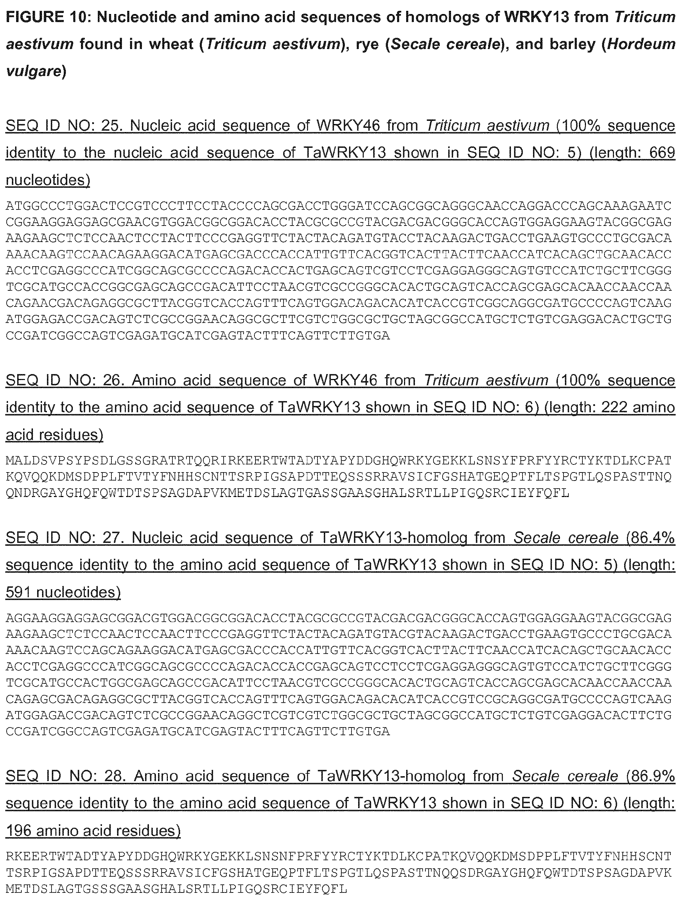

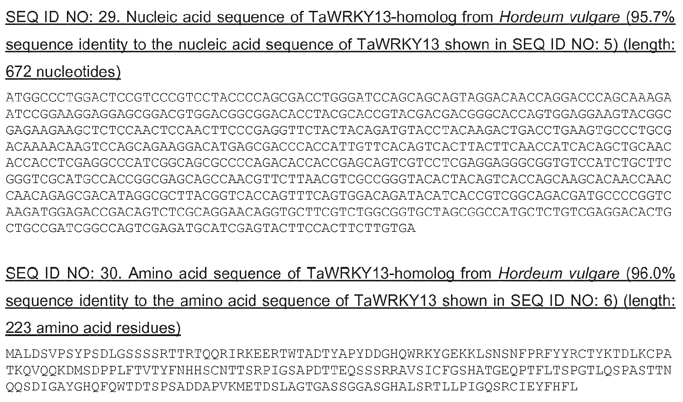

**FIGURE 10** shows nucleic acid and amino acid sequences of homologs of WRKY13 from *Triticum aestivum* (TaWRKY13) found in wheat (*Triticum aestivum*), rye (*Secale cereale*), and barley (*Hordeum vulgare*). SEQ ID NO: 25 shows the nucleic acid sequence of WRKY46 from *Triticum aestivum* having 100% sequence identity to the nucleic acid sequence of TaWRKY13 shown in SEQ ID NO: 5. SEQ ID NO: 26 shows the amino acid sequence of WRKY46 from *Triticum aestivum* having 100% sequence identity to the amino acid sequence of TaWRKY13 shown in SEQ ID NO: 6. SEQ ID NO: 27 shows the nucleic acid sequence of a TaWRKY13-homolog from *Secale cereale* having 86.4% sequence identity to the amino acid sequence of TaWRKY13 shown in SEQ ID NO: 5. SEQ ID NO: 28 shows the amino acid sequence of a TaWRKY13-homolog from *Secale cereale* having 86.9% sequence identity to the amino acid sequence of TaWRKY13 shown in SEQ ID NO: 6. SEQ ID NO: 29 shows the nucleic acid sequence of a TaWRKY13-homolog from *Hordeum vulgare* having 95.7% sequence identity to the nucleic acid sequence of TaWRKY13 shown in SEQ ID NO: 5. SEQ ID NO: 30 shows the amino acid sequence of a TaWRKY13-homolog from *Hordeum vulgare* having 96.0% sequence identity to the amino acid sequence of TaWRKY13 shown in SEQ ID NO: 6.

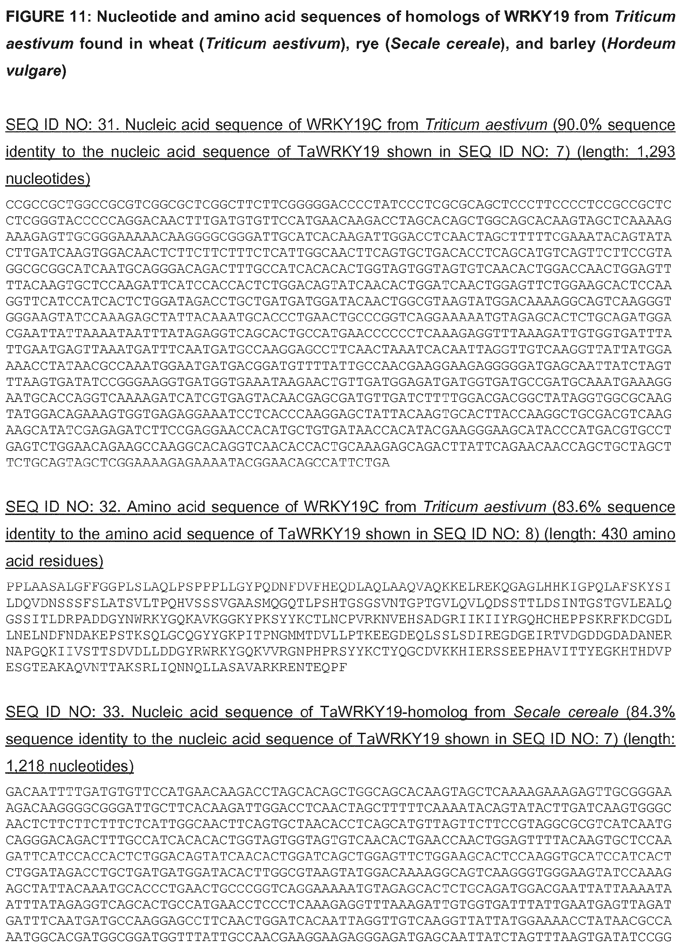

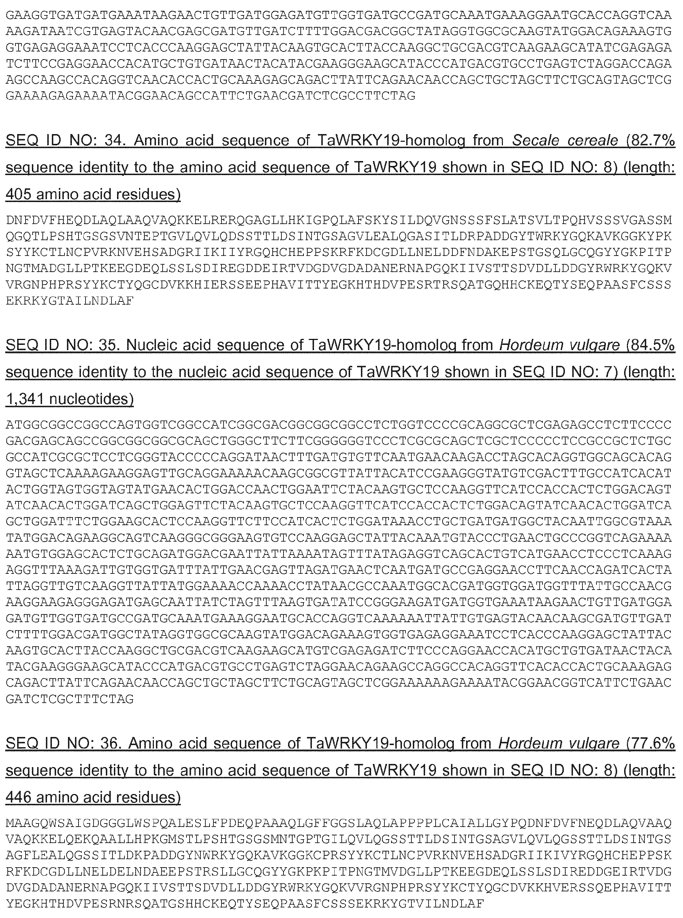

**FIGURE 11** shows nucleic acid and amino acid sequences of homologs of WRKY19 from *Triticum aestivum* (TaWRKY19) found in wheat (*Triticum aestivum*), rye (*Secale cereale*), and barley (*Hordeum vulgare*). SEQ ID NO: 31 shows the nucleic acid sequence of WRKY19C from *Triticum aestivum* having 90.0% sequence identity to the nucleic acid sequence of TaWRKY19 shown in SEQ ID NO: 7. SEQ ID NO: 32 shows the amino acid sequence of WRKY19C from *Triticum aestivum* having 83.6% sequence identity to the amino acid sequence of TaWRKY19 shown in SEQ ID NO: 8. SEQ ID NO: 33 shows the nucleic acid sequence of a TaWRKY19-homolog from *Secale cereale* having 84.3% sequence identity to the nucleic acid sequence of TaWRKY19 shown in SEQ ID NO: 7. SEQ ID NO: 34 shows the amino acid sequence of a TaWRKY19-homolog from *Secale cereale* having 82.7% sequence identity to the amino acid sequence of TaWRKY19 shown in SEQ ID NO: 8. SEQ ID NO: 35 shows the nucleic acid sequence of a TaWRKY19-homolog from *Hordeum vulgare* having 84.5% sequence identity to the nucleic acid sequence of TaWRKY19 shown in SEQ ID NO: 7. SEQ ID NO: 36 shows the amino acid sequence of a TaWRKY19-homolog from *Hordeum vulgare* having 77.6% sequence identity to the amino acid sequence of TaWRKY19 shown in SEQ ID NO: 8.

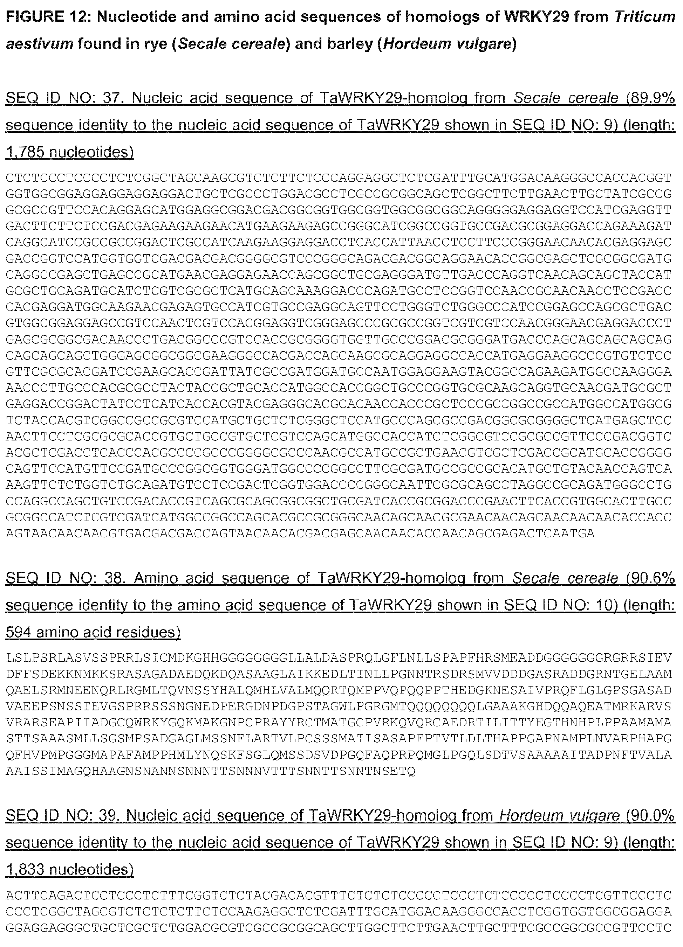

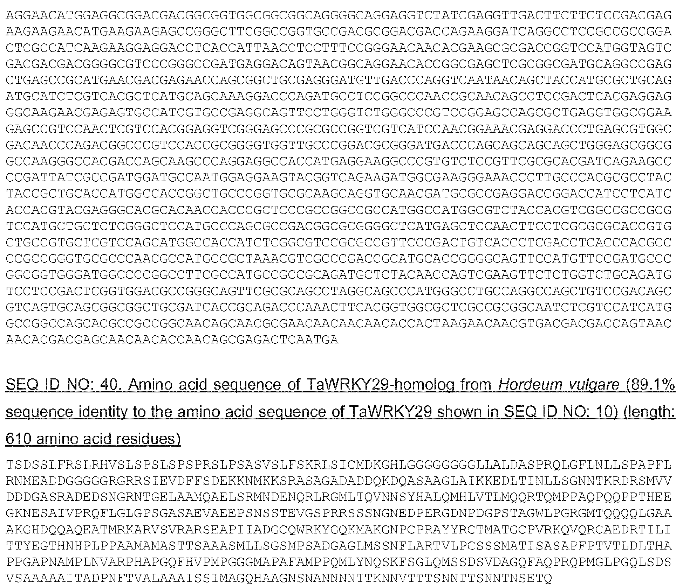

**FIGURE 12** shows nucleic acid and amino acid sequences of homologs of WRKY29 from *Triticum aestivum* (TaWRKY29) found in rye (*Secale cereale*) and barley (*Hordeum vulgare*). SEQ ID NO: 37 shows the nucleic acid sequence of a TaWRKY29-homolog from *Secale cereale* having 89.9% sequence identity to the nucleic acid sequence of TaWRKY29 shown in SEQ ID NO: 9. SEQ ID NO: 38 shows the amino acid sequence of a TaWRKY29-homolog from *Secale cereale* having 90.6% sequence identity to the amino acid sequence of TaWRKY29 shown in SEQ ID NO: 10. SEQ ID NO: 39 shows the nucleic acid sequence of a TaWRKY29-homolog from *Hordeum vulgare* having 90.0% sequence identity to the nucleic acid sequence of TaWRKY29 shown in SEQ ID NO: 9. SEQ ID NO: 40 shows the amino acid sequence of a TaWRKY29-homolog from *Hordeum vulgare* having 89.1% sequence identity to the amino acid sequence of TaWRKY29 shown in SEQ ID NO: 10.

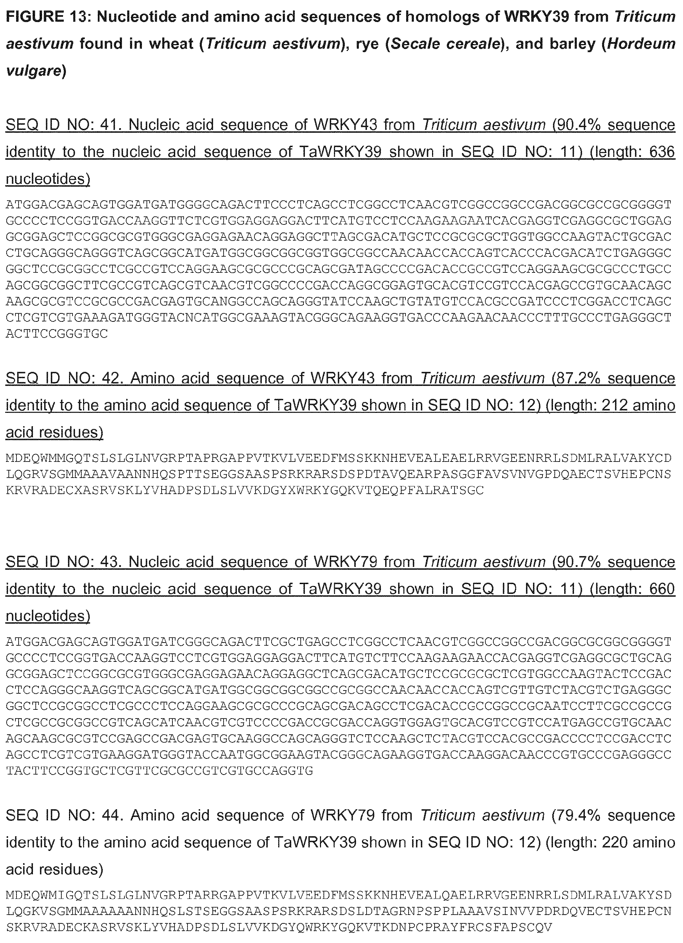

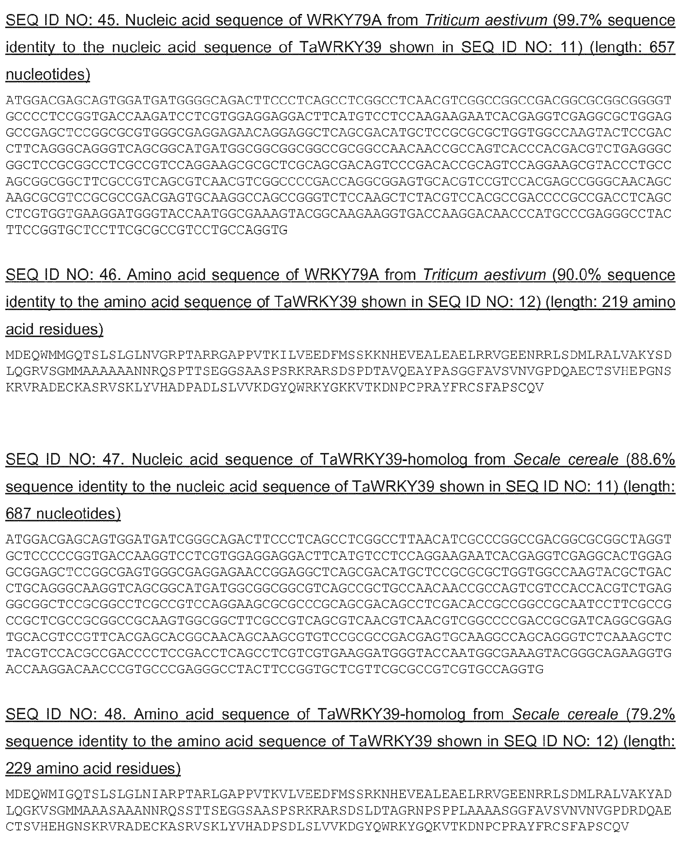

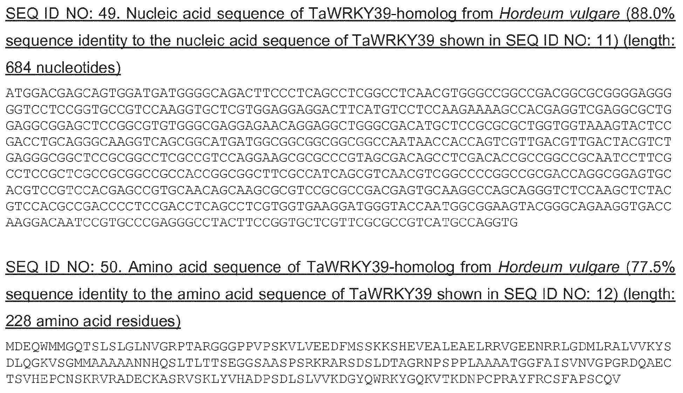

**FIGURE 13** shows nucleic acid and amino acid sequences of homologs of WRKY39 from *Triticum aestivum* (TaWRKY39) found in wheat (*Triticum aestivum*), rye *(Secale cereale),* and barley (*Hordeum vulgare*). SEQ ID NO: 41 shows the nucleic acid sequence of WRKY43 from *Triticum aestivum* having 90.4% sequence identity to the nucleic acid sequence of TaWRKY39 shown in SEQ ID NO: 11. SEQ ID NO: 42 shows the amino acid sequence of WRKY43 from *Triticum aestivum* having 87.2% sequence identity to the amino acid sequence of TaWRKY39 shown in SEQ ID NO: 12. SEQ ID NO: 43 shows the nucleic acid sequence of WRKY79 from *Triticum aestivum* having 90.7% sequence identity to the nucleic acid sequence of TaWRKY39 shown in SEQ ID NO: 11. SEQ ID NO: 44 shows the amino acid sequence of WRKY79 from *Triticum aestivum* having 79.4% sequence identity to the amino acid sequence of TaWRKY39 shown in SEQ ID NO: 12. SEQ ID NO: 45 shows the nucleic acid sequence of WRKY79A from *Triticum aestivum* having 99.7% sequence identity to the nucleic acid sequence of TaWRKY39 shown in SEQ ID NO: 11. SEQ ID NO: 46 shows the amino acid sequence of WRKY79A from *Triticum aestivum* having 90.0% sequence identity to the amino acid sequence of TaWRKY39 shown in SEQ ID NO: 12. SEQ ID NO: 47 shows the nucleic acid sequence of a TaWRKY39-homolog from *Secale cereale* having 88.6% sequence identity to the nucleic acid sequence of TaWRKY39 shown in SEQ ID NO: 11. SEQ ID NO: 48 shows he amino acid sequence of a TaWRKY39-homolog from *Secale cereale* having 79.2% sequence identity to the amino acid sequence of TaWRKY39 shown in SEQ ID NO: 12. SEQ ID NO: 49 shows the nucleic acid sequence of a TaWRKY39-homolog from *Hordeum vulgare* having 88.0% sequence identity to the nucleic acid sequence of TaWRKY39 shown in SEQ ID NO: 11. SEQ ID NO: 50 shows the amino acid sequence of a TaWRKY39-homolog from *Hordeum vulgare* having 77.5% sequence identity to the amino acid sequence of TaWRKY39 shown in SEQ ID NO: 12.

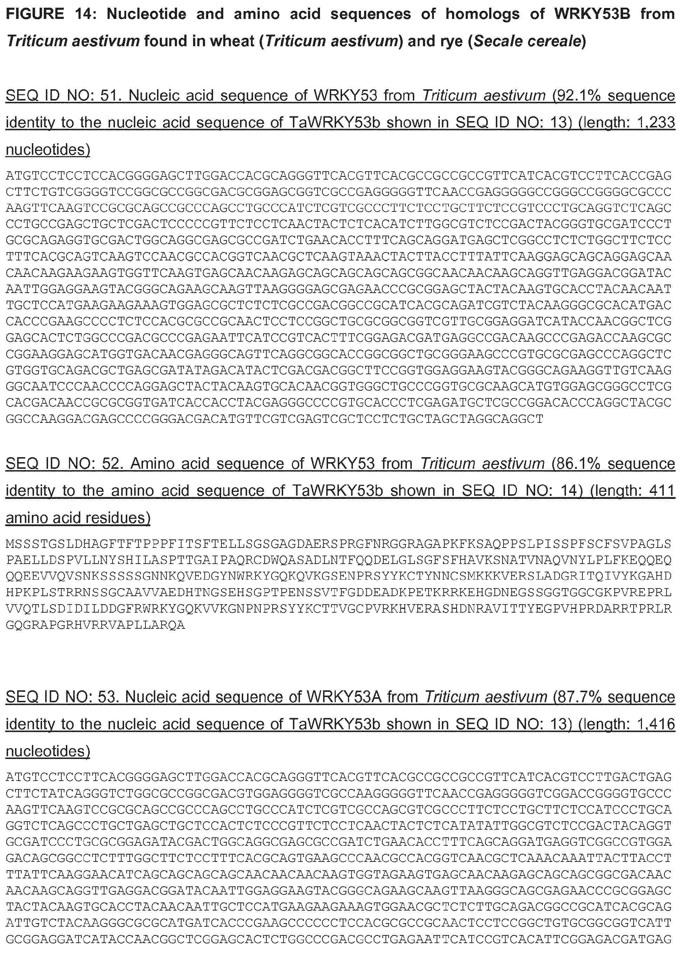

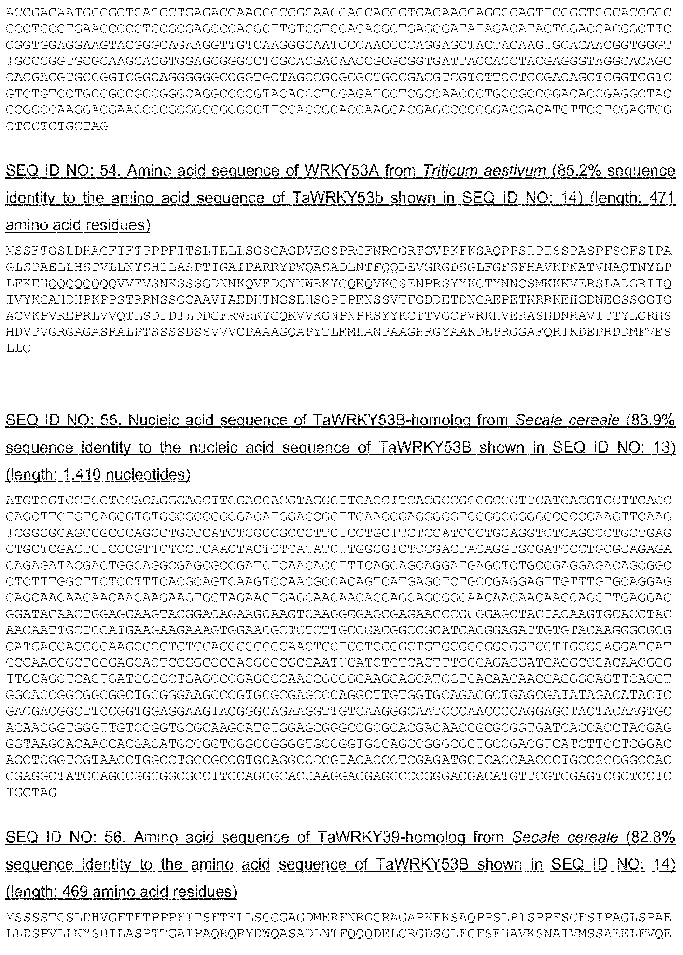

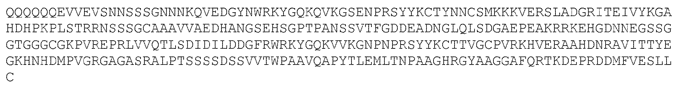

**FIGURE 14** shows nucleic acid and amino acid sequences of homologs of WRKY53B from *Triticum aestivum* (TaWRKY53B) found in wheat *(Triticum aestivum)* and rye (*Secale cereale*). SEQ ID NO: 51 shows the nucleic acid sequence of WRKY53 from *Triticum aestivum* having 92.1% sequence identity to the nucleic acid sequence of TaWRKY53B shown in SEQ ID NO: 13. SEQ ID NO: 52 shows he amino acid sequence of WRKY53 from *Triticum aestivum* having 86.1% sequence identity to the amino acid sequence of TaWRKY53B shown in SEQ ID NO: 14. SEQ ID NO: 53 shows the nucleic acid sequence of WRKY53A from *Triticum aestivum* having 87.7% sequence identity to the nucleic acid sequence of TaWRKY53B shown in SEQ ID NO: 13. SEQ ID NO: 54 shows the amino acid sequence of WRKY53A from *Triticum aestivum* having 85.2% sequence identity to the amino acid sequence of TaWRKY53B shown in SEQ ID NO: 14. SEQ ID NO: 55 shows the nucleic acid sequence of a TaWRKY53B-homolog from *Secale cereale* having 83.9% sequence identity to the nucleic acid sequence of TaWRKY53B shown in SEQ ID NO: 13. SEQ ID NO: 56 shows the amino acid sequence of a TaWRKY39-homolog from *Secale cereale* having 82.8% sequence identity to the amino acid sequence of TaWRKY53B shown in SEQ ID NO: 14.

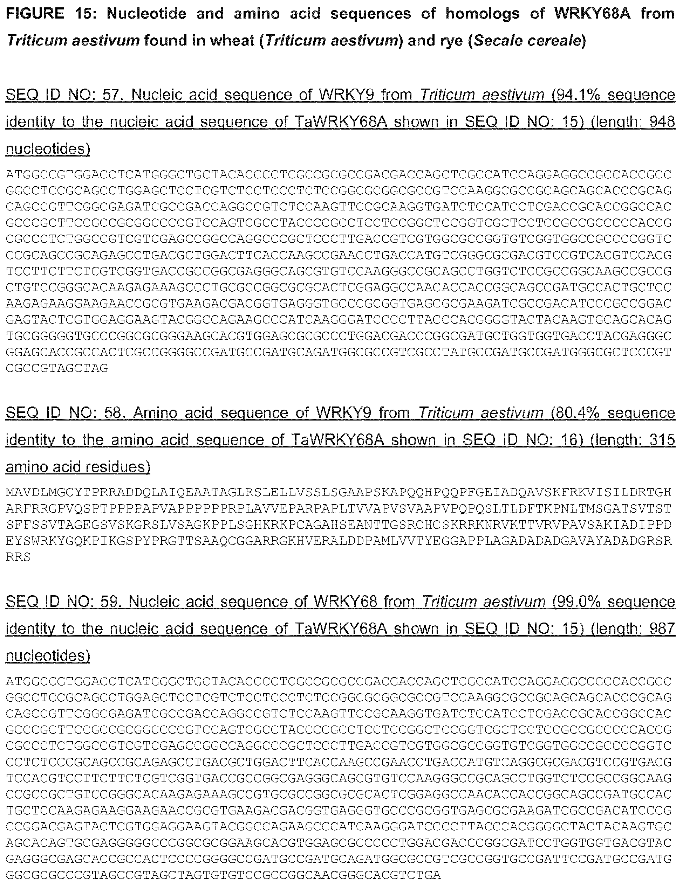

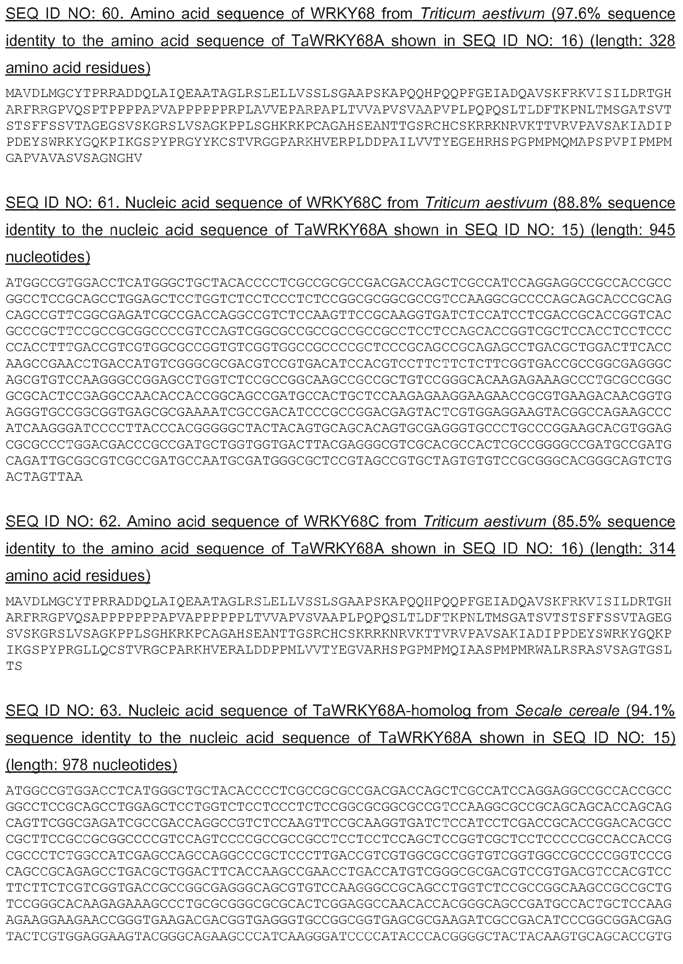

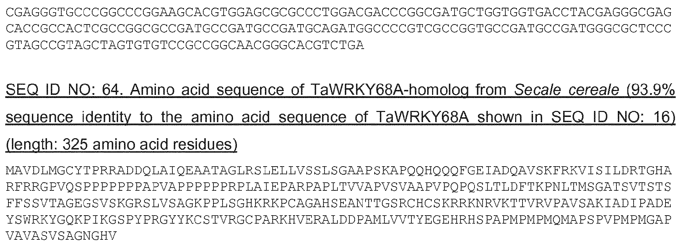

**FIGURE 15** shows nucleic acid and amino acid sequences of homologs of WRKY68A from *Triticum aestivum* (TaWRKY68A) found in wheat *(Triticum aestivum*) and rye (*Secale cereale*). SEQ ID NO: 57 shows the nucleic acid sequence of WRKY9 from *Triticum aestivum* having 94.1% sequence identity to the nucleic acid sequence of TaWRKY68A shown in SEQ ID NO: 15. SEQ ID NO: 58 shows the amino acid sequence of WRKY9 from *Triticum aestivum* having 80.4% sequence identity to the amino acid sequence of TaWRKY68A shown in SEQ ID NO: 16. SEQ ID NO: 59 shows the nucleic acid sequence of WRKY68 from *Triticum aestivum* having 99.0% sequence identity to the nucleic acid sequence of TaWRKY68A shown in SEQ ID NO: 15. SEQ ID NO: 60 shows the amino acid sequence of WRKY68 from *Triticum aestivum* having 97.6% sequence identity to the amino acid sequence of TaWRKY68A shown in SEQ ID NO: 16. SEQ ID NO: 61 shows the nucleic acid sequence of WRKY68C from *Triticum aestivum* having 88.8% sequence identity to the nucleic acid sequence of TaWRKY68A shown in SEQ ID NO: 15. SEQ ID NO: 62 shows the amino acid sequence of WRKY68C from *Triticum aestivum* having 85.5% sequence identity to the amino acid sequence of TaWRKY68A shown in SEQ ID NO: 16. SEQ ID NO: 63 shows the nucleic acid sequence of a TaWRKY68A-homolog from *Secale cereale* having 94.1% sequence identity to the nucleic acid sequence of TaWRKY68A shown in SEQ ID NO: 15. SEQ ID NO: 64 shows the amino acid sequence of a TaWRKY68A-homolog from *Secale cereale* having 93.9% sequence identity to the amino acid sequence of TaWRKY68A shown in SEQ ID NO: 16.

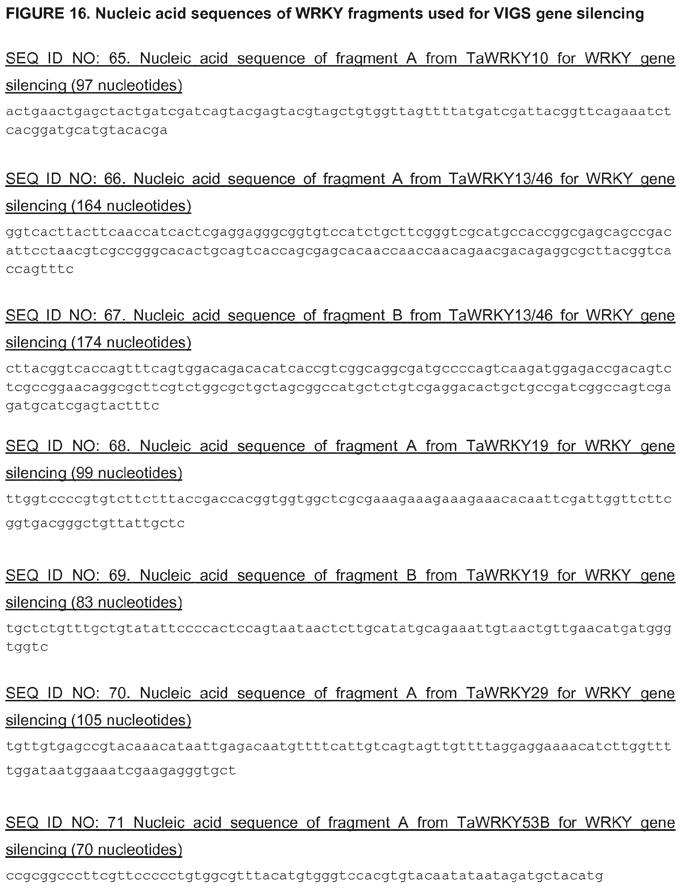

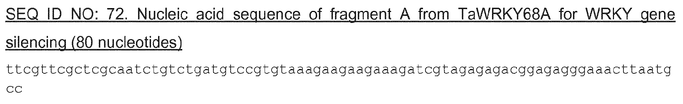

**FIGURE 16** shows nucleic acid sequences of WRKY fragments used for VIGS gene silencing. SEQ ID NO: 65 shows the nucleic acid sequence of fragment A from TaWRKY10 for WRKY gene silencing (97 nucleotides). SEQ ID NO: 66 shows the nucleic acid sequence of fragment A from TaWRKY13/46 for WRKY gene silencing (164 nucleotides). SEQ ID NO: 67 shows the nucleic acid sequence of fragment B from TaWRKY13/46 for WRKY gene silencing (174 nucleotides). SEQ ID NO: 68 shows the nucleic acid sequence of fragment A from TaWRKY19 for WRKY gene silencing (99 nucleotides). SEQ ID NO: 69 shows the nucleic acid sequence of fragment B from TaWRKY19 for WRKY gene silencing (83 nucleotides). SEQ ID NO: 70 shows the nucleic acid sequence of fragment A from TaWRKY29 for WRKY gene silencing (105 nucleotides). SEQ ID NO: 71 shows the nucleic acid sequence of fragment A from TaWRKY53B for WRKY gene silencing (70 nucleotides). SEQ ID NO: 72 shows the nucleic acid sequence of fragment A from TaWRKY68A for WRKY gene silencing (80 nucleotides).
**FIGURE 17** shows phytoene desaturase gene (PDS) silenced cv. *Avalon* (Av) vs. cv. *Cordiale* (Crd). The 18S subunit ribosomal protein was used for normalization. BSMV:00 means empty BSMV vector (=control; Ev). BSMV:PDS means BSMV vector carrying fragment for PDS silencing (Pds).
**FIGURE 18** shows the effect of WRKY10 silencing on picnidia and spores count in cv. *Cordiale* after *Septoria* infection. BSMV:00 means empty BSMV vector (=control). BSMV:WRKY10 means BSMV vector carrying gene silencing sequence for silencing WRKY10.
**FIGURE 19** shows the effect of WRKY silencing to *Septoria* infection on cv. *Avalon.* BSMV:00 means empty BSMV vector (=control). BSMV:WRKY10 means BSMV vector carrying gene silencing sequence for silencing WRKY10.
**FIGURE 20** shows the effect of WRKY10 silencing on picnidia and spores count in cv. *Cordiale* after *Septoria* infection. BSMV:00 means empty BSMV vector (=control). BSMV:WRKY10 means BSMV vector carrying gene silencing sequence for silencing WRKY10.
**FIGURE 21** shows the effect of WRKY10 silencing on picnidia and spores count in cv. *KWS Santiago* after *Septoria* infection. EV means empty vector (=control). BSMV:WRKY10 means BSMV vector carrying gene silencing sequence for silencing WRKY10.
**FIGURE 22** shows the effect of WRKY10 silencing on picnidia and spores count in cv. *KWS Lili* after *Septoria* infection. EV means empty vector (=control). BSMV:WRKY10 means BSMV vector carrying gene silencing sequence for silencing WRKY10.
**FIGURE 23** shows the susceptibility of cv. *KWS Santiago* vs. cv. *KWS Lili* towards *Septoria* infection.
**FIGURE 24** shows a comparison of *Septoria* sporulation in WRKY10 silenced cv. *KWS Santiago* vs. cv. *KWS Lili* with full WRKY10 expression. EV *KWS Lili* means cv. *KWS Lili* transformed with an empty BSMV vector. BSMV:WRKY10 *KWS Santiago* means WRKY10 silenced cv. *KWS Santiago* using BSMV vector carrying gene silencing sequence for silencing WRKY10.
**FIGURE 25** is an excerpt from Figure 1 of Yuan et al. 2011, PLoS ONE, Vol. 6(10), e26468, and shows in particular a schematic representation of pCaBS-α, pCaBS-β and pCaBS-γ used for VIGS as depicted in Figure 1 A of Yuan et al., 2011.
**Figure 26** shows a list of possible positions in the sequences of TaWRKY10, TaWRKY13, TaWRKY19, TaWRKY29, TaWRKY39, TaWRKY53B, and TaWRKY68A for mutations which effect an early stop-codon and which can be used in TILLING or genome editing approaches for inhibiting WRKYs according to the disclosure.

### DETAILED DESCRIPTION OF THE DISCLOSURE

As used herein and in the appended claims, the singular forms "a", "and", and "the" include plural referents unless the context clearly dictates otherwise. Thus, for example, reference to "a cell" or "the cell" includes reference to one or more cells and equivalents thereof (e.g., plurality of cells) known to those skilled in the art. Similarly, reference to "a composition" includes a plurality of such compositions, and refers to one or more compositions, respectively, unless the context clearly dictates otherwise. The term "about" when referring to a number or a numerical range means that the number or numerical range referred to is an approximation within experimental variability (or within statistical experimental error), and thus the number or numerical range may vary between 1% and 15% of the stated number or numerical range. The term "comprising" (and related terms such as "comprise" or "comprises" or "having" or "including") is not intended to exclude that in other certain embodiments, for example, an embodiment of any plant cell, plant or part thereof, composition, method, or process, or the like, described herein, may "consist of" or "consist essentially of" the described features. The term "at least one" comprises the integers larger than or equal to 1, such as 1, 2, 3, 4, 5, 6, 7, 8, 9, 10, 11, 12 and so forth.

As used herein "operatively linked" or "operably linked" includes reference to a functional linkage between a first sequence, such as a promoter, and a second sequence, wherein the promoter sequence initiates and mediates transcription of the second sequence, *i.e.*, the promoter is in a correct functional location and/or orientation in relation to the second nucleic acid sequence to control transcriptional initiation and/or expression of that sequence. Generally, operatively linked means that the nucleic acid sequences being linked are contiguous and, where necessary to join two protein coding regions, contiguous and in the same reading frame.

The term "promoter region" or "promoter" is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. The regulatory sequence may be homologous or heterologous to the desired gene sequence. The promoter is oriented relative to a DNA sequence such that it is capable of initiating transcription of the said DNA sequence, i.e., the term "promoter" includes reference to a region of DNA upstream from the start of transcription and involved in recognition and binding of RNA polymerase and/or other proteins to initiate transcription. A "plant promoter" is a promoter capable of initiating transcription in plant cells. Exemplary plant promoters include, but are not limited to, those that are obtained from plants, plant viruses and bacteria, which comprise genes expressed in plant cells such as Agrobacterium or Rhizobium.

The term "regulatory nucleic acid sequence" refers collectively to promoter sequences, polyadenylation signals, transcription termination sequences, upstream regulatory domains, origins of replication, enhancers and the like, which collectively provide for the replication, transcription and translation of a coding sequence in a recipient cell. Not all of these control sequences need always be present so long as the selected coding sequence is capable of being replicated, transcribed and translated in an appropriate cell. One skilled in the art can readily identify regulatory nucleic acid sequence from public databases and materials. Furthermore, one skilled in the art can identify a regulatory sequence that is applicable for the intended use.

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

The observation that cereals grown under low-input nutrient regimes are less susceptible to disease has been a "given" in agronomy for many years, although direct data showing such a link has been difficult to generate due to a range of compounding factors. Preliminary data analysis carried out by the present inventors from transcriptomic data suggested that the WRKYs may be responsible for relating nitrogen input to expression of genes involved in disease resistance. In the course of research projects, the present inventors identified specific WRKY transcription factors and their role in the interaction between wheat and Septoria disease and nitrogen input. Part of that project was the use of Virus induced gene silencing (VIGS) as a tool to knockdown the activity of key WRKYs in wheat seedlings cultivated under varying nitrogen levels and study the effect under Septoria infection.

The present disclosure provides means and methods for conferring or boosting pathogen resistance to cereals, in particular resistance against fungal pathogens such as *Septoria,* under fertilizer input growth conditions necessary for maintaining yield. The present disclosure is based on the identification of key WRKYs whose expression or activity is fertilizer-responsive and/or which are characterized by the amino acid sequence motif D-G/E-X1-X2-W-R-K-Y-G-K/E/Q-K/E, wherein X1 is a hydrophobic amino acid and X2 is a polar, non-hydrophobic amino acid. WRKYs are one of the largest families of transcriptional regulators in plants, with 72 representatives in Arabidopsis, 100 in rice and to date 111 in wheat (PLANT TFDB, Centre for Bioinformatics, Peking University). WRKY transcription factors are so called because of the presence of a highly conserved 60 amino acid domain, which contain the WRKYGQK motif or variants thereof. WRKYs transcription factors are important components of plant signaling webs, which are often found to regulate plant response to biotic and abiotic stresses.

The present inventors performed knockdown of certain WRKYs using Virus induced gene silencing (VIGS), and the effect on Septoria disease progression under varying nitrogen levels was ascertained. The appearance of disease symptoms varied, suggesting that these WRKYs play a major role in resistance.

In this WRKY gene expression screen in wheat leaf tissue upon Septoria infection, TaWRKY10, TaWRKY13, TaWRKY19, TaWRKY29, TaWRKY39, TaWRKY53B and TaWRKY68A were identified to show altered gene expression and considered as possible key WRKYs.

In all identified WRKYs the spore and pycnidia count is reduced in the knockdown data generated so far, which has the potential to be highly important for disease spread in a whole-plant/crop situation.

Data linking the key WRKYs of the present disclosure to both nitrogen regime and disease resistance has been generated in experiments carried out both in the field and in growth room trials (cf. Examples 1-3). In two winter wheat varieties studied, low Nitrogen levels resulted in lower levels of susceptibility to STB disease. Subsequent QPCR data showed that the nitrogen sensitive TaWRKYs had similar differential expression pattern during Septoria infection.

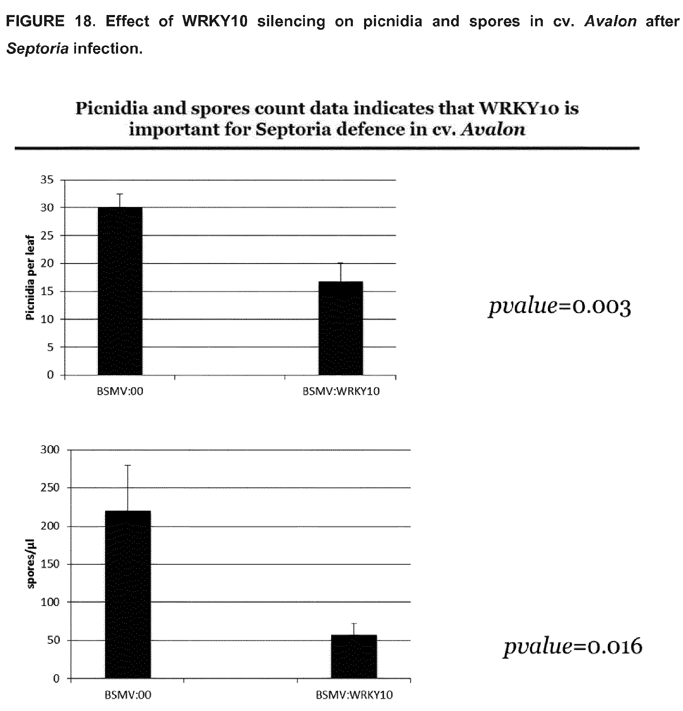

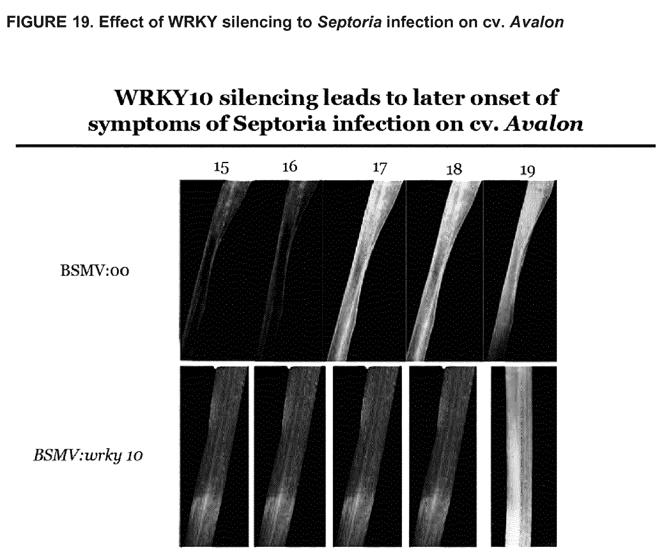

For example, by knocking down TaWRKY10 in the cv. Avalon, spore and pycnidia counts were significantly reduced (Example 7, Figure 18). This indicates that the infection of the wheat plants with Septoria is efficiently inhibited and the plant defense is improved. Consequently the resistance/tolerance of wheat against Septoria disease is enhanced. In addition, in TaWRKY10 silenced *cv Avalon* plants the visible symptoms of Septoria infection were delayed by around 4 days (Example 7, Figure 19).

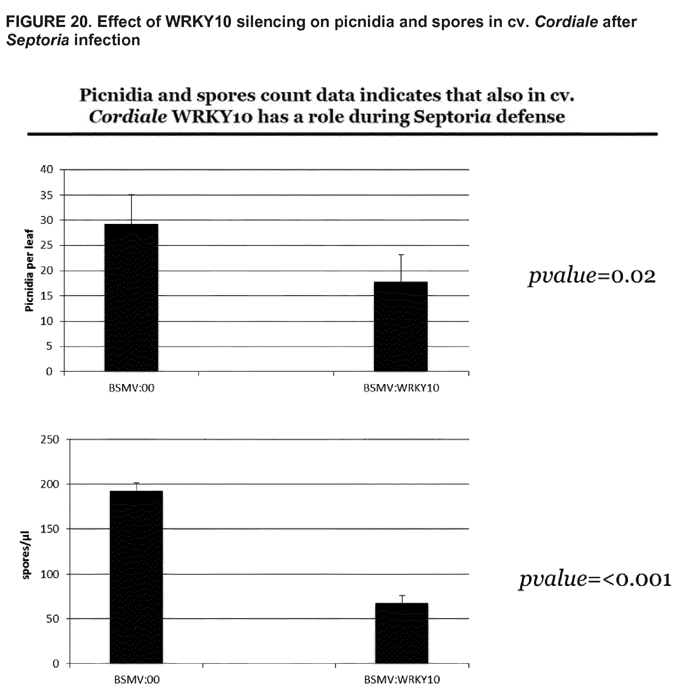

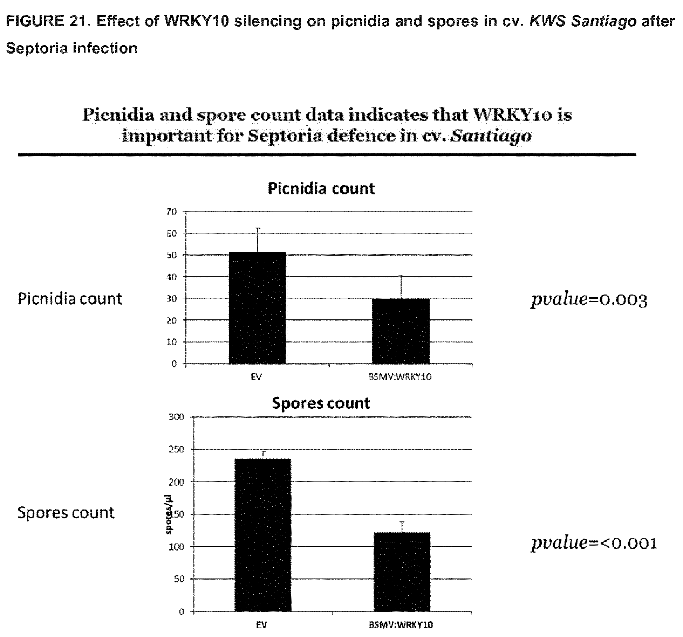

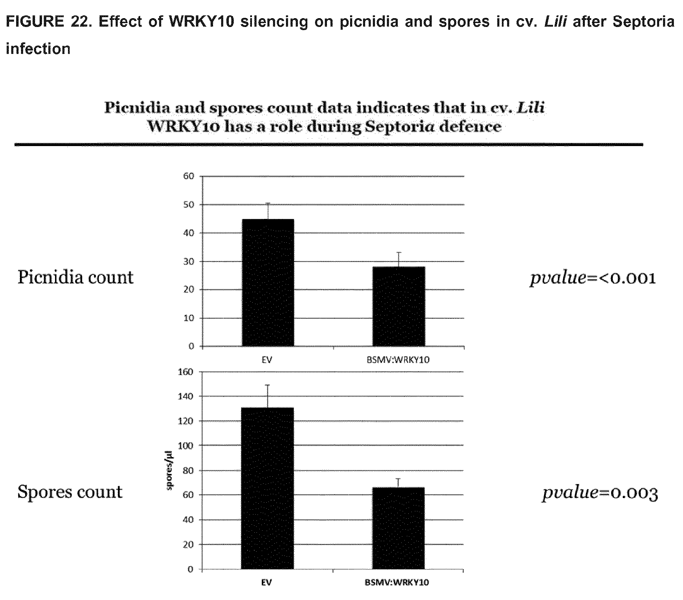

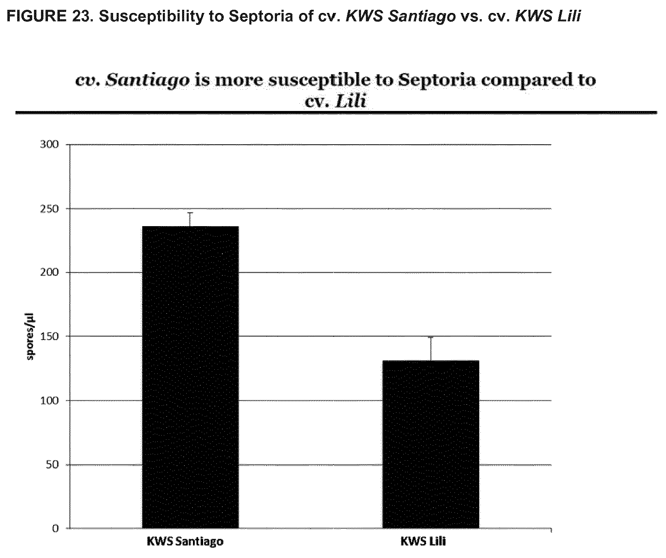

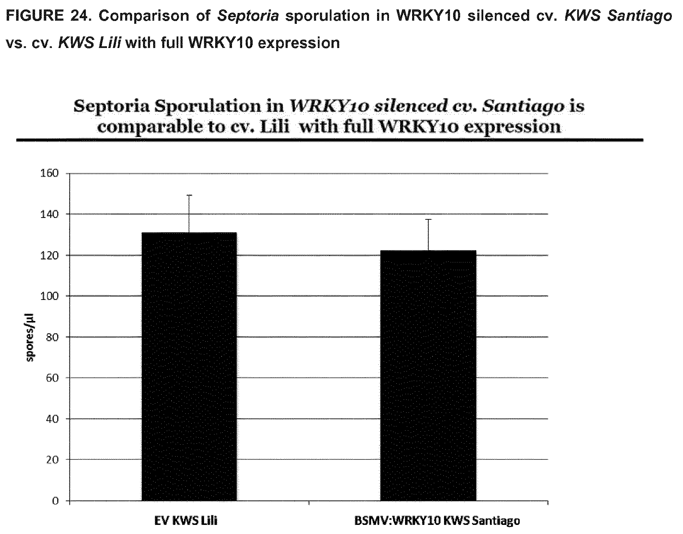

The reduced spore and pycnidia counts could be shown also in other cultivars like *Cordiale* (Figure 20), *KWS Santiago* (Figure 21) and *KWS Lili* (Figure 22). The enhanced resistance has also been demonstrated in the following experiment. Naturally the *cv. KWS Santiago* is more susceptible to Septoria compared to *cv. KWS Lili.* That can be shown by spore counts (Example 7, Figure 23) as well as in field trials. The resistant cv. *KWS Lili* has been transformed with an empty VIGS vector (EV) and the susceptible cv. *KWS Santiago* with BSMV:WRKY10. The result is that both the non-silenced cv. *KWS Lili* and the silenced cv. *KWS Santiago* show a comparable Septoria sporulation (Example 7, Figure 24).

Comparable results as seen for WRKY10 have been achieved for WRKY13, WRKY19, WRKY29, WRKY39, WRKY53B and WRKY68A. For all of these WRKYs, it is suggested that their expression or activity is fertilizer-responsive and/or are characterized by the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid.

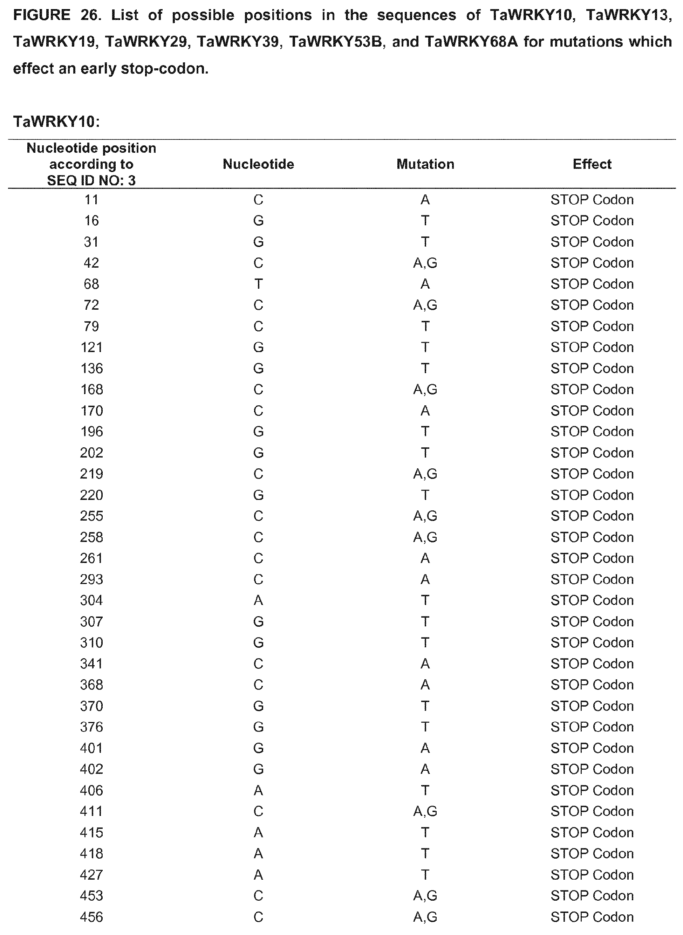

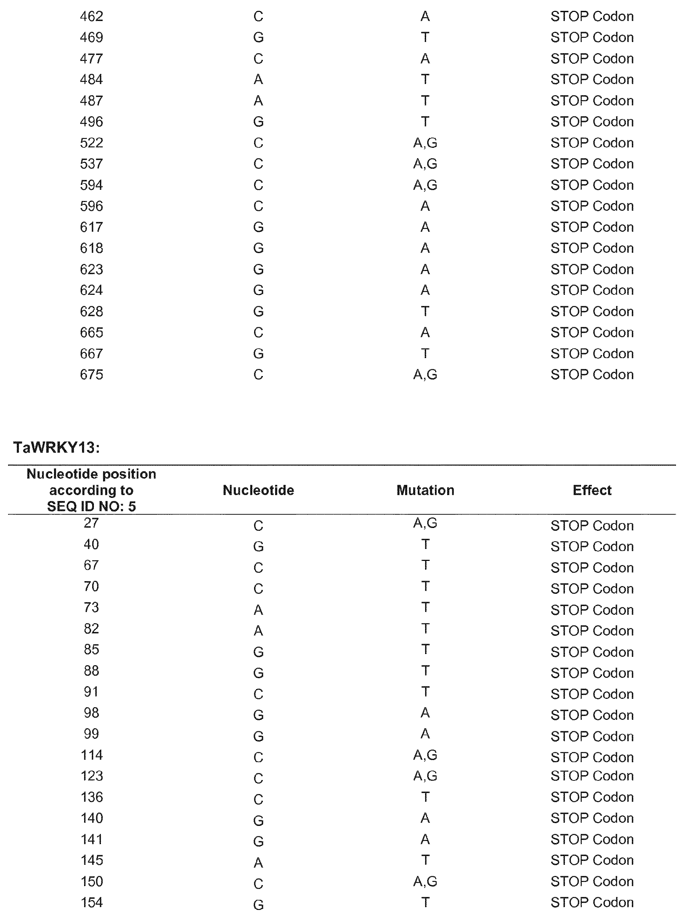

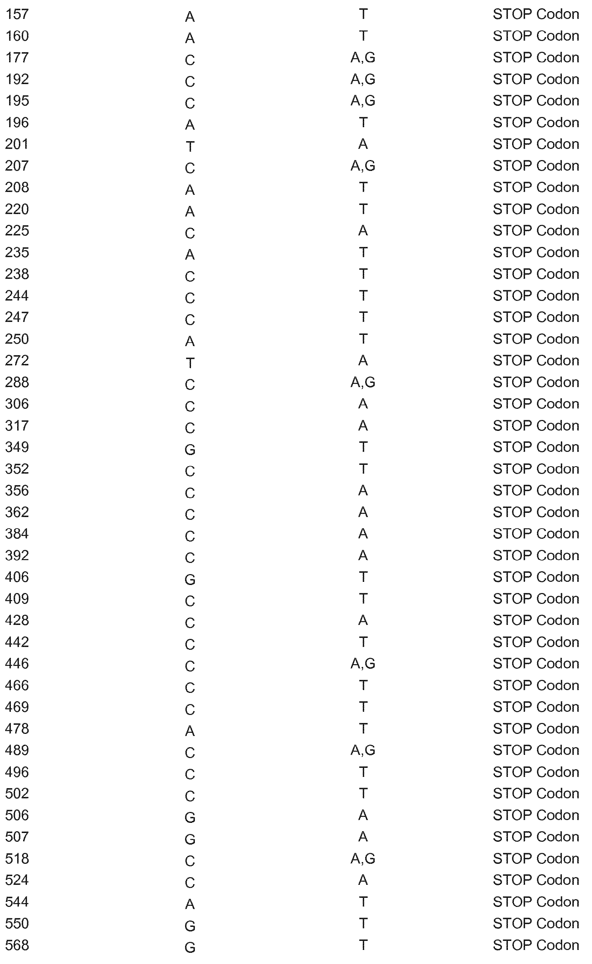

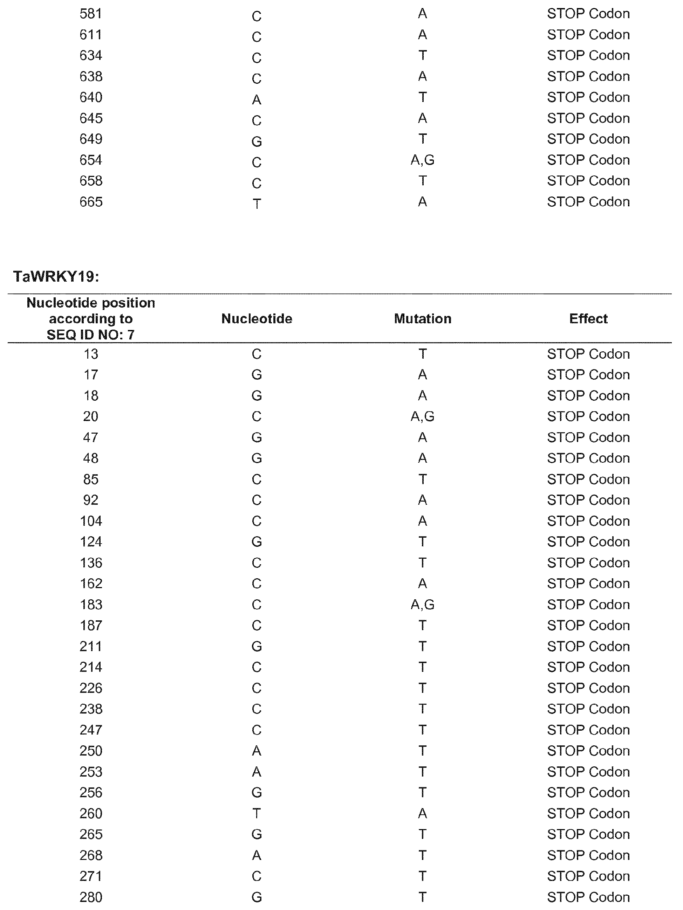

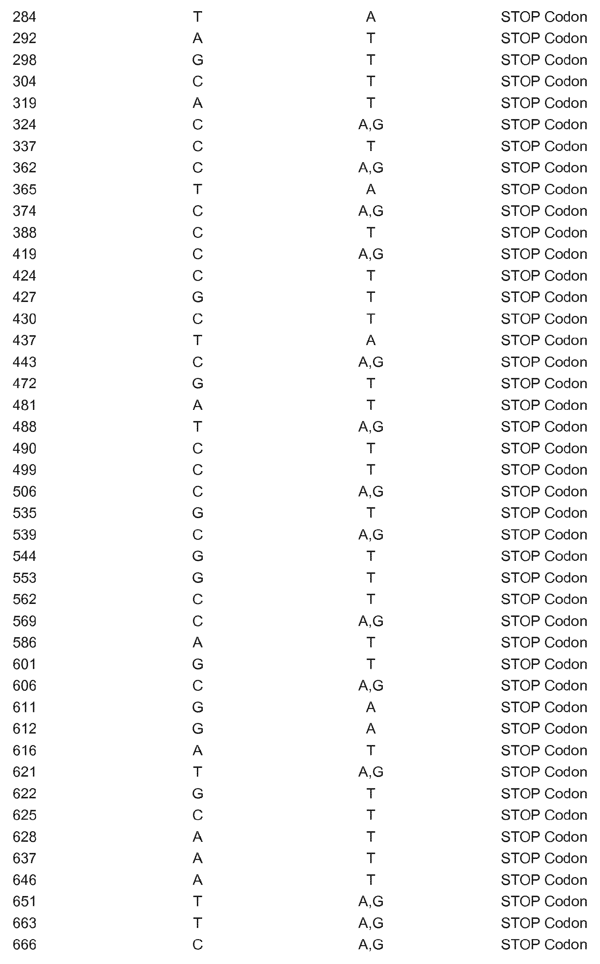

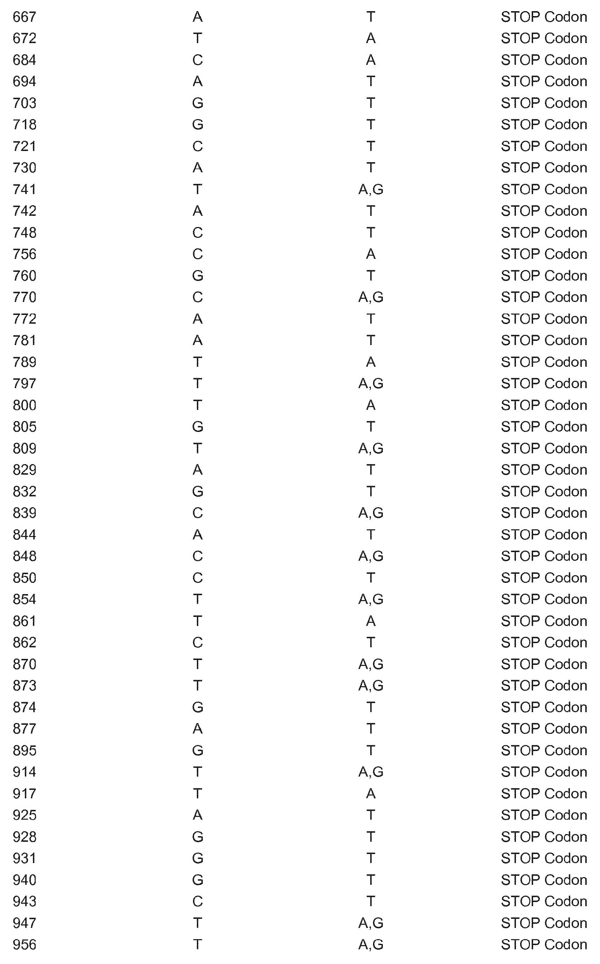

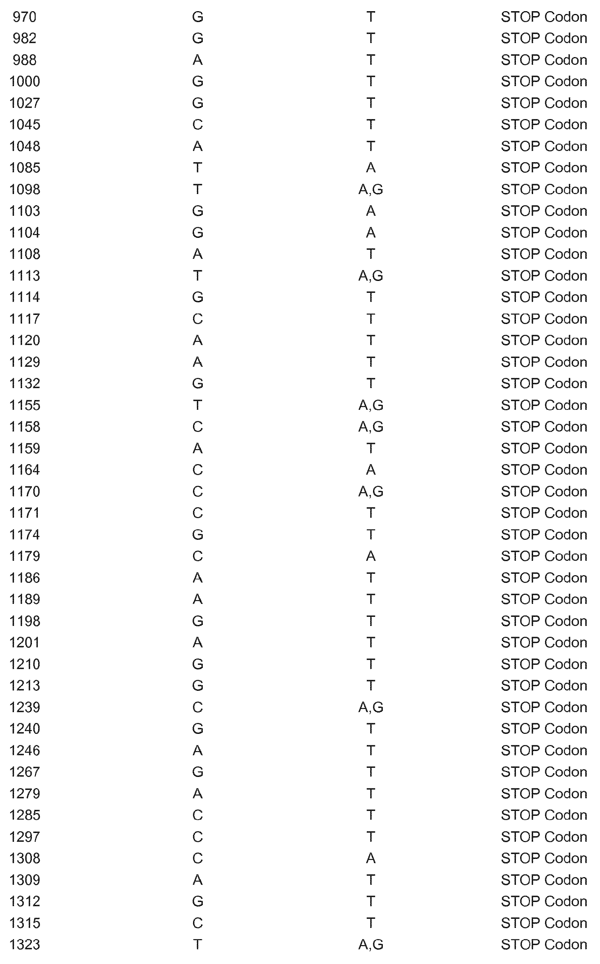

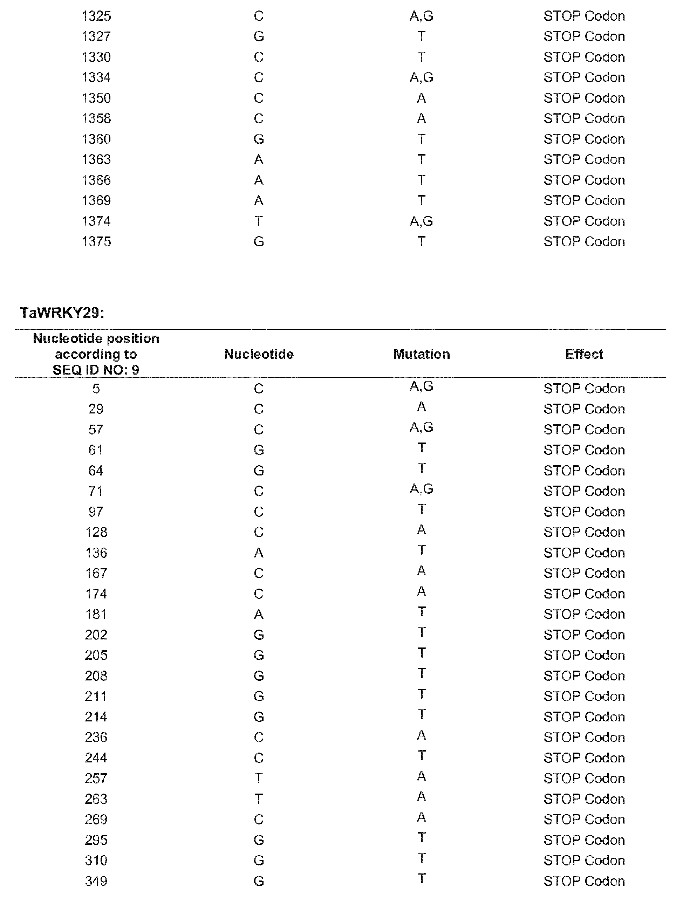

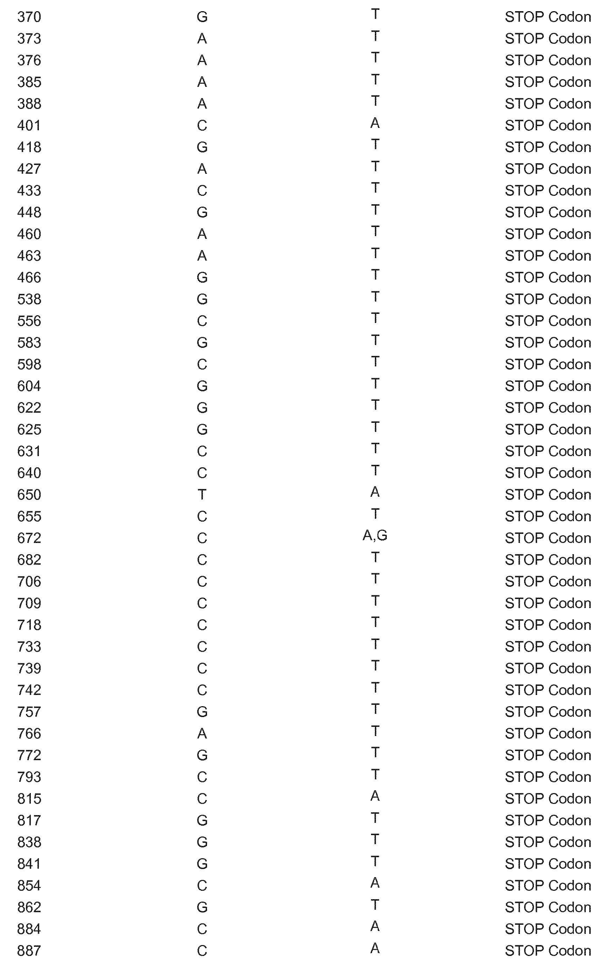

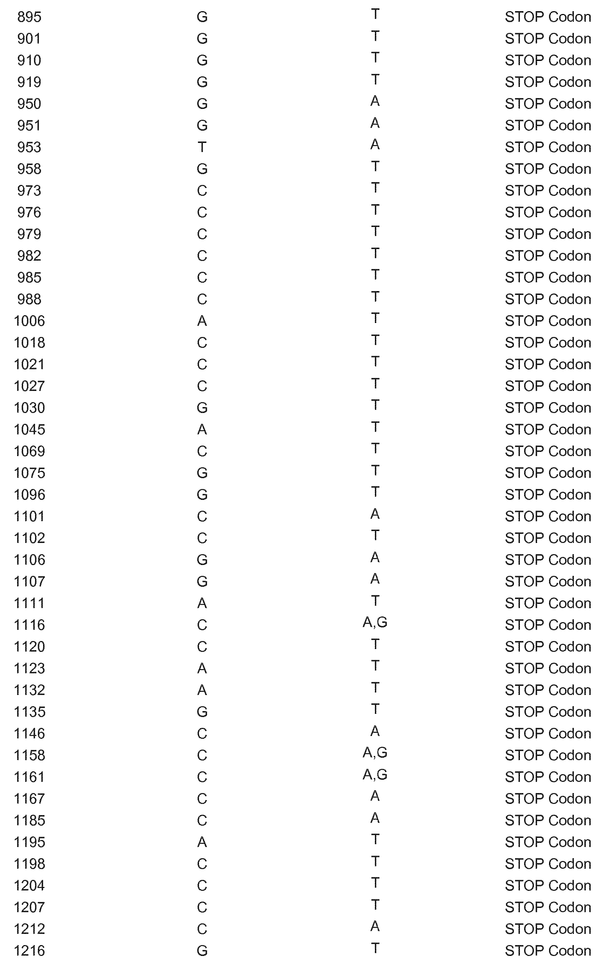

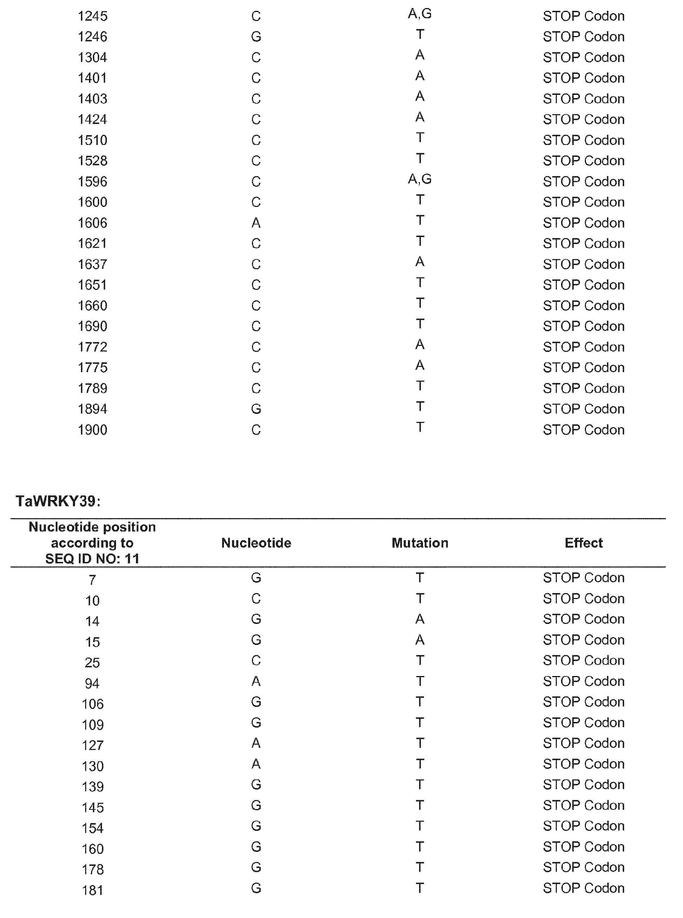

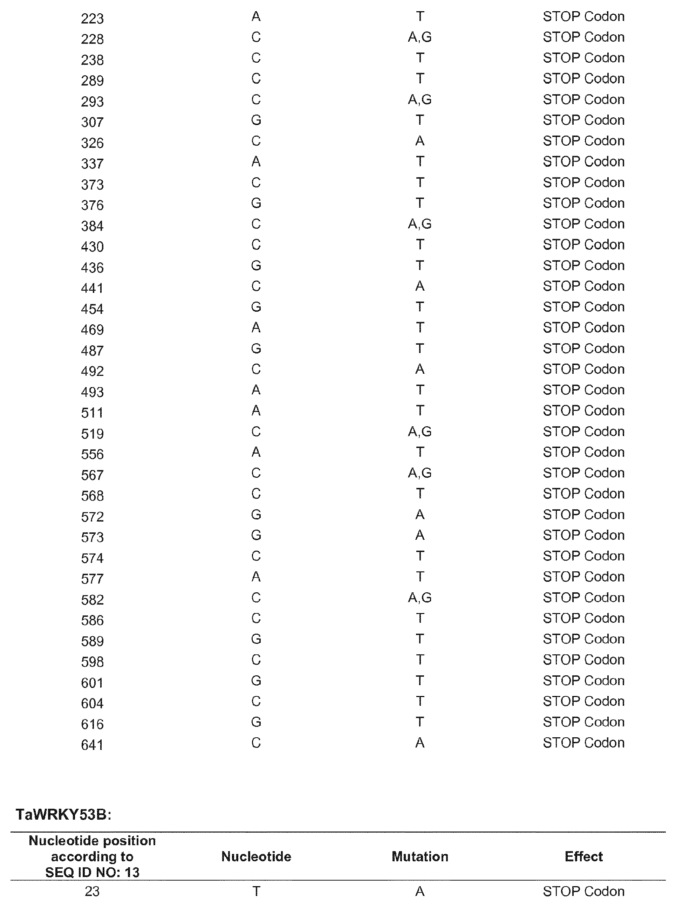

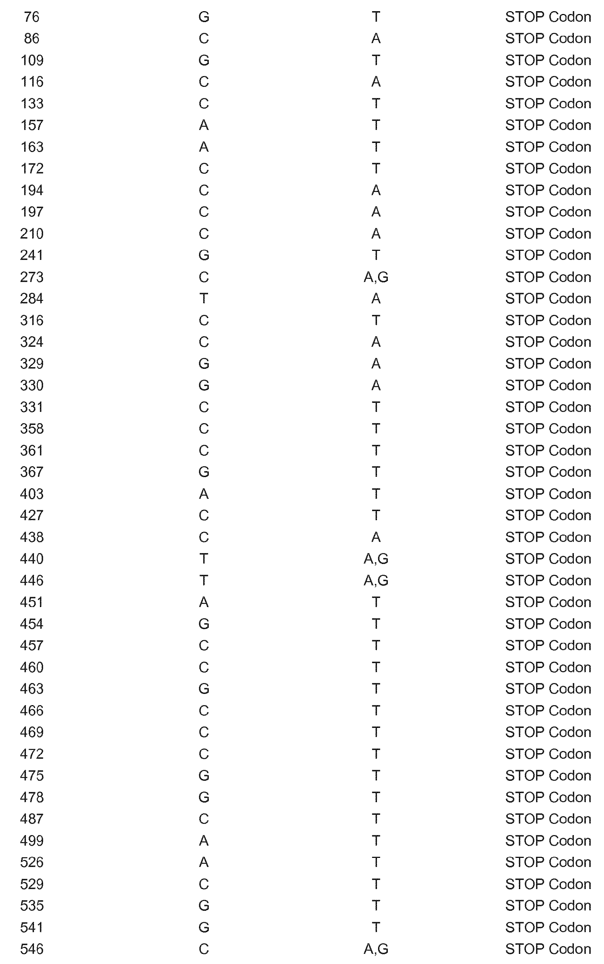

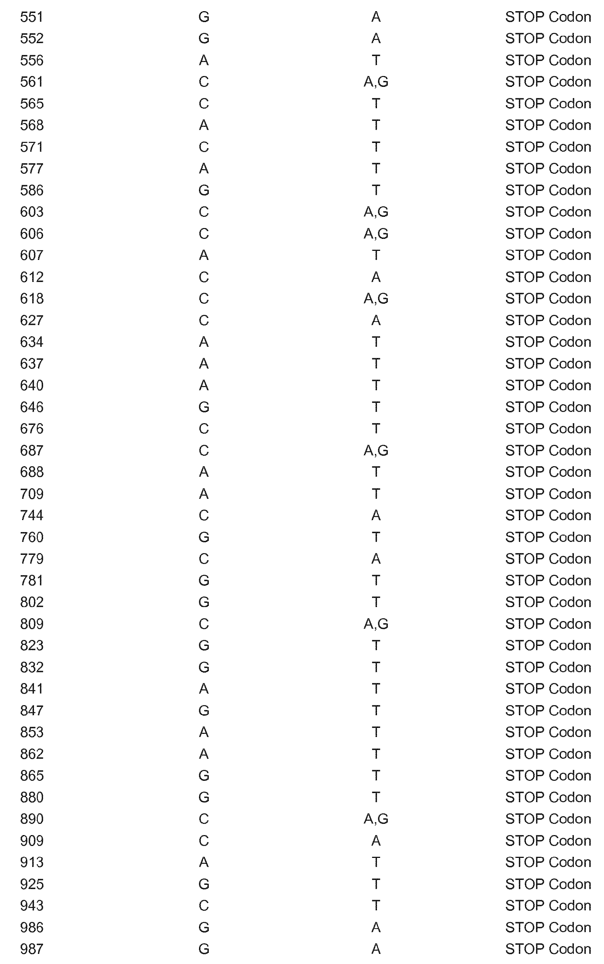

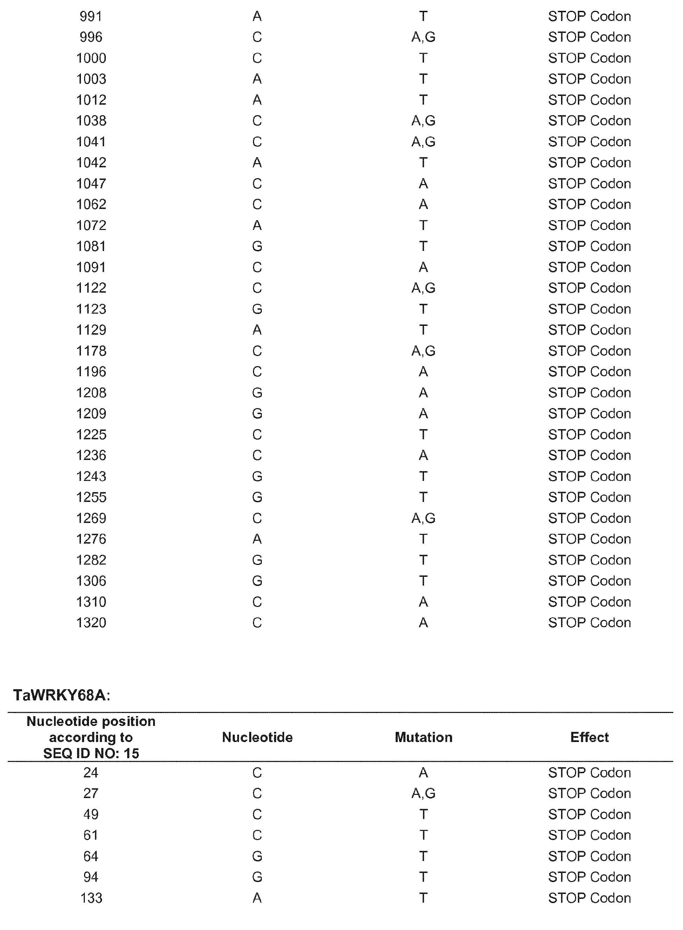

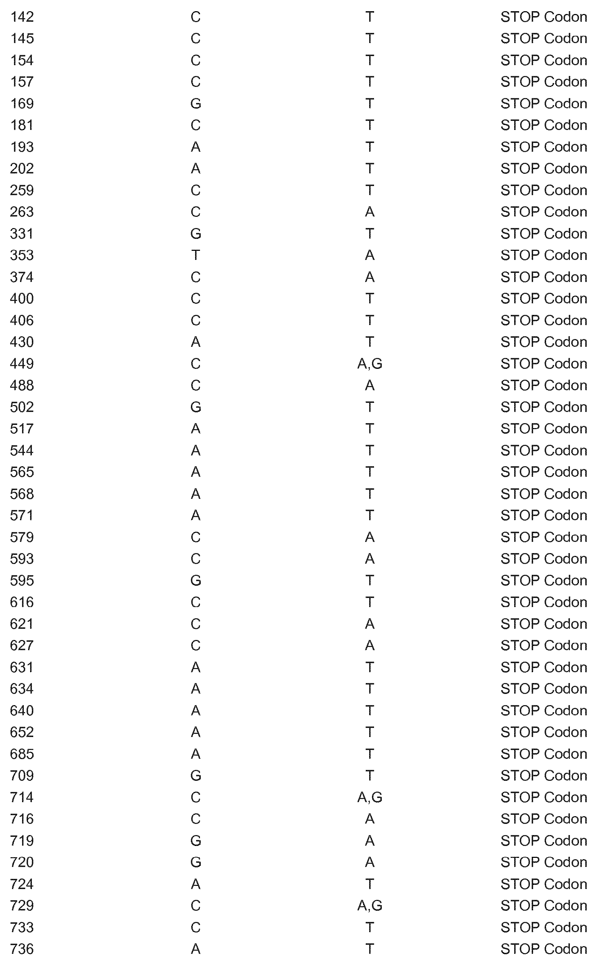

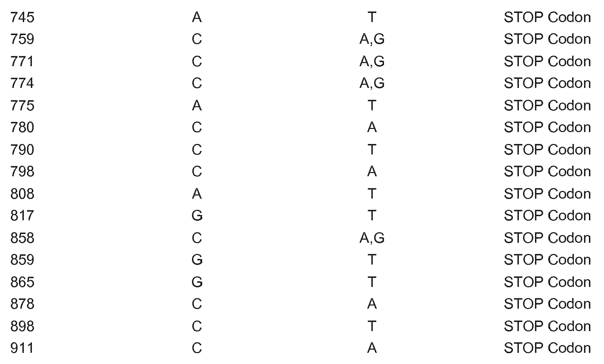

The results obtained in the present examples are based on the VIGS-induced silencing of the specific key WRKY genes. However, it is possible to achieve the same or comparable results through other methods: For example, one can build up TILLING populations of a plant and screen for mutations leading to amino acid exchanges or splicing errors. Such mutation can result in a knock-down or a knock-out of the WRKY gene (e.g., if the mutation creates an early stop codon), in an altered (reduced) activity of the WRKY protein, or in a shift of the reading frame if splicing errors occur. Besides of TILLING, such mutations can be introduced/targeted by the known genome editing tools like Zinc Finger Nucleases, TAL Effector Nucleases, CRISPR/Cas9 etc. Another example is the introduction of a DNA construct encoding a double stranded RNA construct suitable for silencing the above WRKY genes (RNAi approach, Example 8). Figure 26 shows a list of possible positions in the sequences of TaWRKY10, TaWRKY13, TaWRKY19, TaWRKY29, TaWRKY39, TaWRKY53B, and TaWRKY68A for mutations (point mutations) which effect an early stop-codon and which can be used in TILLING or genome editing approaches for inhibiting WRKYs according to the disclosure.

In line with the above findings, the present disclosure provides a cereal plant cell having introduced into its genome at least one modification inhibiting the expression or activity of at least one WRKY protein as compared to a corresponding control plant cell lacking the at least one modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In various embodiments of the present disclosure, the hydrophobic amino acid of residue X1 of the amino acid sequence motif shown in SEQ ID NO: 1 or 2 (Figure 1) means any one of Alanine (A), Isoleucine (I), Leucine (L), Methionine (M), Phenylalanine (F), Tryptophan (W), Tyrosine (Y), Valine (V), Histidine (H), Lysine (K), Threonine (T), Cysteine (C), and Glycine (G). Preferably, the hydrophobic amino acid of residue X1 of the amino acid sequence motif shown in SEQ ID NO: 1 or 2 (Figure 1) is Phenylalanine (F), Tyrosine (Y), Histidine (H), or Cysteine (C). In various embodiments of the present disclosure, the polar, non-hydrophobic amino acid of residue X2 of the amino acid sequence motif shown in SEQ ID NO: 1 or 2 (Figure 1) means any one of Arginine (R), Asparagine (N), Aspartic Acid (D), Glutamic Acid (E), Glutamine (Q), and Serine (S). Preferably, the polar, non-hydrophobic amino acid of residue X2 of the amino acid sequence motif shown in SEQ ID NO: 1 or 2 (Figure 1) is Arginine (R), Asparagine (N), Glutamine (Q), or Serine (S).

Accordingly, in the present disclosure, the at least one WRKY protein whose expression or activity is to be inhibited is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence motif is D-G/E-X1-X2-W-R-K-Y-G-K/E/Q-K/E-X-X-X3-X-X2 shown in SEQ ID NO: 2, wherein X is any amino acid, X1 is a hydrophobic amino acid, X2 is a polar, non-hydrophobic amino acid, and X3 is a polar, non-aromatic amino acid. In various embodiments of the present disclosure, the polar, non-aromatic amino acid of residue X3 of the amino acid sequence motif shown in SEQ ID NO: 2 (Figure 1) means any one of Arginine (R), Asparagine (N), Aspartic Acid (D), Glutamic Acid (E), Glutamine (Q), Lysine (K), Threonine (T), Cysteine (C), and Serine (S). Preferably, the polar, non-aromatic amino acid of residue X3 of the amino acid sequence motif shown in SEQ ID NO: 2 (Figure 1) is Arginine (R), Glutamine (Q), Lysine (K), or Serine (S).

As described herein, the terms "amino acid" and "amino acid residue" may be used herein interchangeably.

In the literature, different WRKY transcription factors are described acting as negative or positive regulators. In various embodiments of the present disclosure, the least one WRKY protein whose expression or activity is to be inhibited is a negative regulator of the cereal plant cell or plant defense mechanism/response against a pathogen, *i.e.,* is a negative regulator of the pathogen defense mechanism of the wild-type plant cell lacking the modification inhibiting the expression or activity of the at least one WRKY protein. Here, negative regulation means that the pathogen defense mechanism/response of the cereal plant cell, or cereal plant or part thereof, is reduced, inhibited or blocked.

In various embodiments of the present disclosure, the at least one WRKY gene is selected from the group consisting of TaWRKY10 (SEQ ID NO: 3), TaWRKY13 (SEQ ID NO: 5), TaWRKY19 (SEQ ID NO: 7), TaWRKY29 (SEQ ID NO: 9), TaWRKY39 (SEQ ID NO: 11), TaWRKY53B (SEQ ID NO: 13), and TaWRKY68A (SEQ ID NO: 15), or homologs thereof.

In various embodiments of the disclosure, a WRKY10 gene or protein or a variant or homolog thereof according the present disclosure is a WRKY10 from cereals. Preferably, a WRKY10 gene or protein or a variant or homolog thereof according the present disclosure is a WRKY10 gene or protein from any of wheat (*Triticum aestivum*, TaWRKY10), rye *(Secale cereale,* ScWRKY10), or barley (*Hordeum vulgare,* HvWRKY10). In certain preferred embodiments, a WRKY10 gene or protein or a variant or homolog thereof according the present disclosure is a TaWRKY10 gene or protein *(i.e.,* WRKY10 from *Triticum aestivum).*

In various embodiments of the disclosure, a WRKY13 gene or protein or a variant or homolog thereof according the present disclosure is a WRKY13 from cereals. Preferably, a WRKY13 gene or protein or a variant or homolog thereof according the present disclosure is a WRKY13 gene or protein from any of wheat *(Triticum aestivum,* TaWRKY13), rye *(Secale cereale,* ScWRKY13), or barley (*Hordeum vulgare,* HvWRKY13). In certain preferred embodiments, a WRKY13 gene or protein or a variant or homolog thereof according the present disclosure is a TaWRKY13 gene or protein *(i.e.,* WRKY13 from *Triticum aestivum).*

In various embodiments of the disclosure, a WRKY19 gene or protein or a variant or homolog thereof according the present disclosure is a WRKY19 from cereals. Preferably, a WRKY19 gene or protein or a variant or homolog thereof according the present disclosure is a WRKY19 gene or protein from any of wheat (*Triticum aestivum,* TaWRKY19), rye *(Secale cereale,* ScWRKY19), or barley (*Hordeum vulgare,* HvWRKY19). In certain preferred embodiments, a WRKY19 gene or protein or a variant or homolog thereof according the present disclosure is a TaWRKY19 gene or protein *(i.e.,* WRKY19 from *Triticum aestivum*).

In various embodiments of the disclosure, a WRKY29 gene or protein or a variant or homolog thereof according the present disclosure is a WRKY29 from cereals. Preferably, a WRKY29 gene or protein or a variant or homolog thereof according the present disclosure is a WRKY29 gene or protein from any of wheat *(Triticum aestivum,* TaWRKY29), rye *(Secale cereale,* ScWRKY29), or barley (*Hordeum vulgare,* HvWRKY29). In certain preferred embodiments, a WRKY29 gene or protein or a variant or homolog thereof according the present disclosure is a TaWRKY29 gene or protein *(i.e.,* WRKY29 from *Triticum aestivum).*

In various embodiments of the disclosure, a WRKY39 gene or protein or a variant or homolog thereof according the present disclosure is a WRKY39 from cereals. Preferably, a WRKY39 gene or protein or a variant or homolog thereof according the present disclosure is a WRKY39 gene or protein from any of wheat *(Triticum aestivum,* TaWRKY39), rye *(Secale cereale,* ScWRKY39), or barley (*Hordeum vulgare,* HvWRKY39). In certain preferred embodiments, a WRKY39 gene or protein or a variant or homolog thereof according the present disclosure is a TaWRKY39 gene or protein *(i.e.,* WRKY39 from *Triticum aestivum).*

In various embodiments of the disclosure, a WRKY53B gene or protein or a variant or homolog thereof according the present disclosure is a WRKY53B from cereals. Preferably, a WRKY53B gene or protein or a variant or homolog thereof according the present disclosure is a WRKY53B gene or protein from any of wheat *(Triticum aestivum,* TaWRKY53B), rye *(Secale cereale,* ScWRKY53B), or barley *(Hordeum vulgare,* HvWRKY53B). In certain preferred embodiments, a WRKY53B gene or protein or a variant or homolog thereof according the present disclosure is a TaWRKY53B gene or protein *(i.e.,* WRKY53B from *Triticum aestivum*).

In various embodiments of the disclosure, a WRKY68A gene or protein or a variant or homolog thereof according the present disclosure is a WRKY68A from cereals. Preferably, a WRKY68A gene or protein or a variant or homolog thereof according the present disclosure is a WRKY68A gene or protein from any of wheat (*Triticum aestivum,* TaWRKY68A), rye (*Secale cereale,* ScWRKY68A), or barley (*Hordeum vulgare,* HvWRKY68A). In certain preferred embodiments, a WRKY68A gene or protein or a variant or homolog thereof according the present disclosure is a TaWRKY68A gene or protein *(i.e.,* WRKY68A from *Triticum aestivum*).

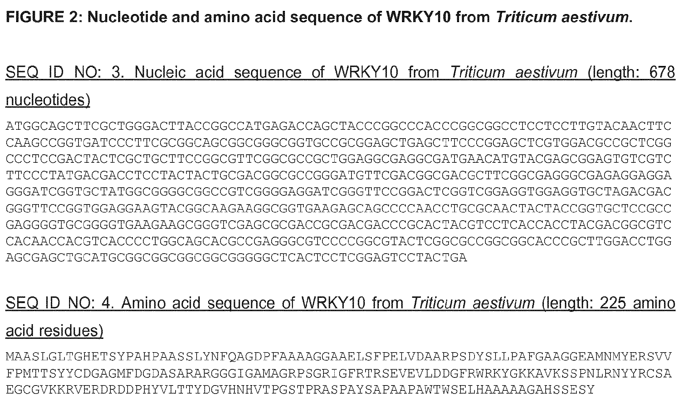

In various embodiments of the disclosure, the nucleic acid sequence of the at least one WRKY gene shows at least 70% homology or sequence identity to the nucleic acid sequence of TaWRKY10 (SEQ ID NO: 3, Figure 2), wherein the variant nucleic acid sequence encodes a WRKY protein, preferably a WRKY10 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY10 (SEQ ID NO: 3, Figure 2), wherein the variant nucleic acid sequence encodes a WRKY protein, preferably a WRKY10 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY10 (SEQ ID NO: 3, Figure 2), wherein the variant nucleic acid sequence encodes a WRKY protein, preferably a WRKY10 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY10 as described above exhibit the same fertilizer-responsive expression or activity as the native TaWRKY10 encoded by the sequence of SEQ ID NO: 3 (Figure 2). In a particularly preferred embodiment, the nucleic acid sequence of the at least one WRKY gene comprises (or consists of) the sequence of SEQ ID NO: 3 (Figure 2), or the sequence of any one SEQ ID NOs: 17, 19, 21, and 23 (Figure 9).

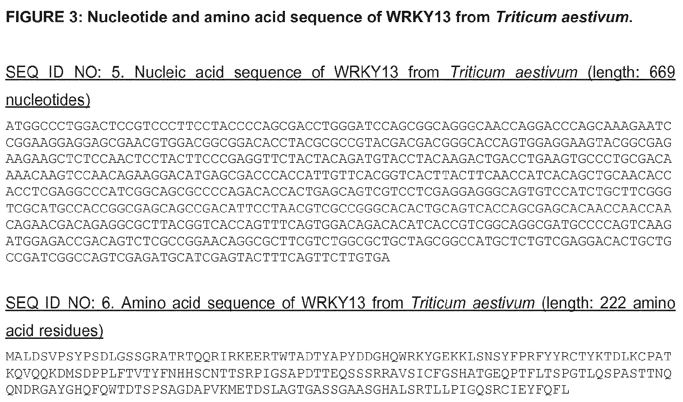

In various embodiments of the disclosure, the nucleic acid sequence of the at least one WRKY gene shows at least 70% homology or sequence identity to the nucleic acid sequence of TaWRKY13 (SEQ ID NO: 5, Figure 3), wherein the variant nucleic acid sequence encodes a WRKY protein, preferably a WRKY13 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY13 (SEQ ID NO: 5, Figure 3), wherein the variant nucleic acid sequence encodes a WRKY protein, preferably a WRKY13 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY13 (SEQ ID NO: 5, Figure 3), wherein the variant nucleic acid sequence encodes a WRKY protein, preferably a WRKY13 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY13 as described above exhibit the same fertilizer-responsive expression or activity as the native TaWRKY13 encoded by the sequence of SEQ ID NO: 5 (Figure 3). In a particularly preferred embodiment, the nucleic acid sequence of the at least one WRKY gene comprises (or consists of) the sequence of SEQ ID NO: 5 (Figure 3), or the sequence of any one SEQ ID NOs: 25, 27, and 29 (Figure 10).

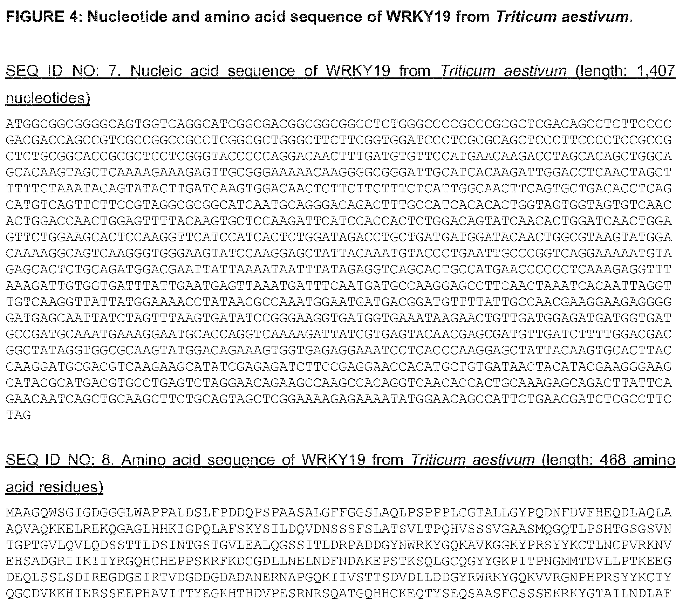

In various embodiments of the disclosure, the nucleic acid sequence of the at least one WRKY gene shows at least 70% homology or sequence identity to the nucleic acid sequence of TaWRKY19 (SEQ ID NO: 7, Figure 4), wherein the variant nucleic acid sequence encodes a WRKY protein, preferably a WRKY19 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY19 (SEQ ID NO: 7, Figure 4), wherein the variant nucleic acid sequence encodes a WRKY protein, preferably a WRKY19 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY19 (SEQ ID NO: 7, Figure 4), wherein the variant nucleic acid sequence encodes a WRKY protein, preferably a WRKY19 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY19 as described above exhibit the same fertilizer-responsive expression or activity as the native TaWRKY19 encoded by the sequence of SEQ ID NO: 7 (Figure 4). In a particularly preferred embodiment, the nucleic acid sequence of the at least one WRKY gene comprises (or consists of) the sequence of SEQ ID NO: 7 (Figure 4), or the sequence of any one SEQ ID NOs: 31, 33, and 35 (Figure 11).

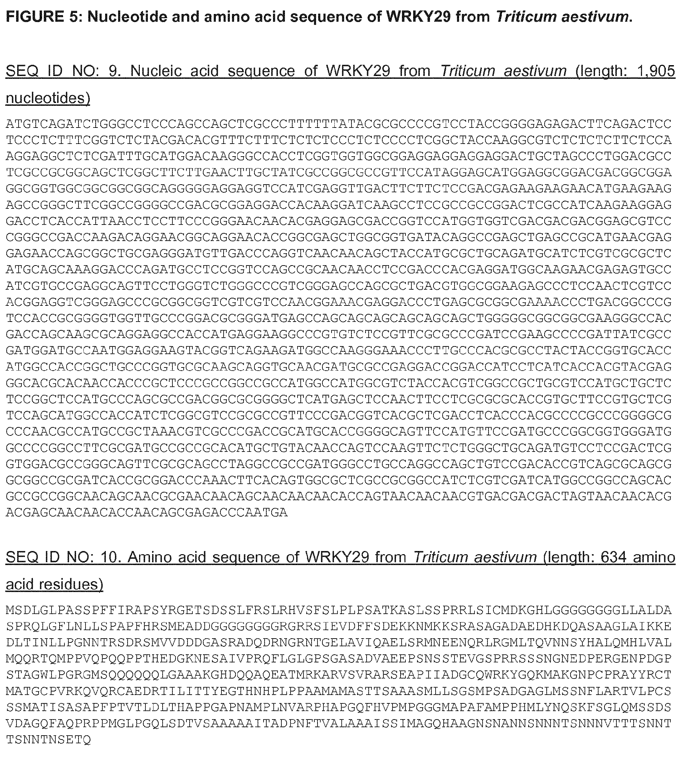

In various embodiments of the disclosure, the nucleic acid sequence of the at least one WRKY gene shows at least 70% homology or sequence identity to the nucleic acid sequence of TaWRKY29 (SEQ ID NO: 9, Figure 5), wherein the variant nucleic acid sequence encodes a WRKY protein, preferably a WRKY29 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY29 (SEQ ID NO: 9, Figure 5), wherein the variant nucleic acid sequence encodes a WRKY protein, preferably a WRKY29 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY29 (SEQ ID NO: 9, Figure 5), wherein the variant nucleic acid sequence encodes a WRKY protein, preferably a WRKY29 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY29 as described above exhibit the same fertilizer-responsive expression or activity as the native TaWRKY29 encoded by the sequence of SEQ ID NO: 9 (Figure 5). In a particularly preferred embodiment, the nucleic acid sequence of the at least one WRKY gene comprises (or consists of) the sequence of SEQ ID NO: 9 (Figure 5), or the sequence of SEQ ID NO: 37 or 39 (Figure 12).

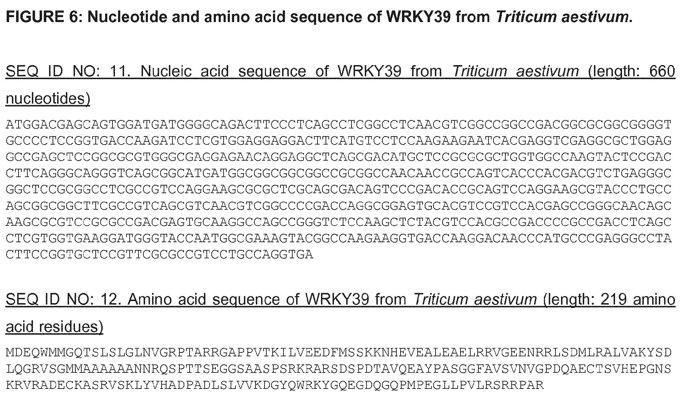

In various embodiments of the disclosure, the nucleic acid sequence of the at least one WRKY gene shows at least 70% homology or sequence identity to the nucleic acid sequence of TaWRKY39 (SEQ ID NO: 11, Figure 6), wherein the variant nucleic acid sequence encodes a WRKY protein, preferably a WRKY39 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY39 (SEQ ID NO: 11, Figure 6), wherein the variant nucleic acid sequence encodes a WRKY protein, preferably a WRKY39 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY39 (SEQ ID NO: 11, Figure 6), wherein the variant nucleic acid sequence encodes a WRKY protein, preferably a WRKY39 protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY39 as described above exhibit the same fertilizer-responsive expression or activity as the native TaWRKY39 encoded by the sequence of SEQ ID NO: 11 (Figure 6). In a particularly preferred embodiment, the nucleic acid sequence of the at least one WRKY gene comprises (or consists of) the sequence of SEQ ID NO: 11 (Figure 6), or the sequence of any one SEQ ID NOs: 41, 43, 45, 47, and 49 (Figure 13).

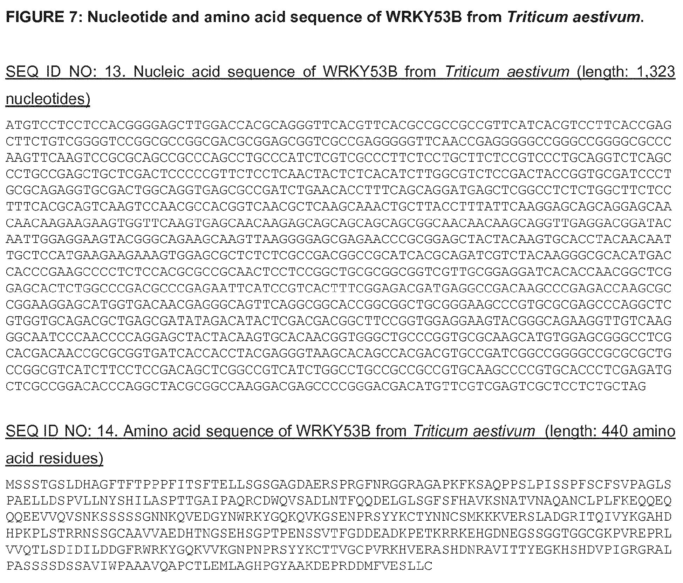

In various embodiments of the disclosure, the nucleic acid sequence of the at least one WRKY gene shows at least 70% homology or sequence identity to the nucleic acid sequence of TaWRKY53B (SEQ ID NO: 13, Figure 7), wherein the variant nucleic acid sequence encodes a WRKY protein, preferably a WRKY53B protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY53B (SEQ ID NO: 13, Figure 7), wherein the variant nucleic acid sequence encodes a WRKY protein, preferably a WRKY53B protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY53B (SEQ ID NO: 13, Figure 7), wherein the variant nucleic acid sequence encodes a WRKY protein, preferably a WRKY53B protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY53B as described above exhibit the same fertilizer-responsive expression or activity as the native TaWRKY53B encoded by the sequence of SEQ ID NO: 13 (Figure 7). In a particularly preferred embodiment, the nucleic acid sequence of the at least one WRKY gene comprises (or consists of) the sequence of SEQ ID NO: 13 (Figure 7), or the sequence of any one SEQ ID NOs: 51, 53, and 55 (Figure 14).

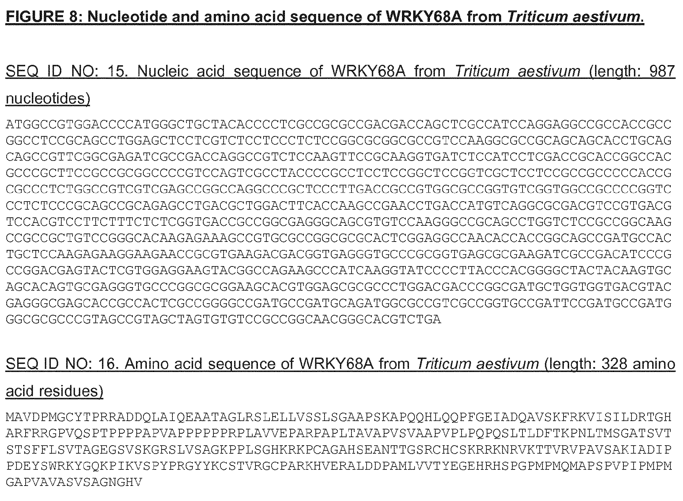

In various embodiments of the disclosure, the nucleic acid sequence of the at least one WRKY gene shows at least 70% homology or sequence identity to the nucleic acid sequence of TaWRKY68A (SEQ ID NO: 15, Figure 8), wherein the variant nucleic acid sequence encodes a WRKY protein, preferably a WRKY68A protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the nucleic acid sequence of the at least one WRKY gene shows at least 80% or at least 90% homology or sequence identity to the nucleic acid sequence of TaWRKY68A (SEQ ID NO: 15, Figure 8), wherein the variant nucleic acid sequence encodes a WRKY protein, preferably a WRKY68A protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the nucleic acid sequence of the at least one WRKY gene shows at least 95%, 96%, 97%, 98%, or 99% homology or sequence identity to the nucleic acid sequence of TaWRKY68A (SEQ ID NO: 15, Figure 8), wherein the variant nucleic acid sequence encodes a WRKY protein, preferably a WRKY68A protein, which is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY68A as described above exhibit the same fertilizer-responsive expression or activity as the native TaWRKY68A encoded by the sequence of SEQ ID NO: 15 (Figure 8). In a particularly preferred embodiment, the nucleic acid sequence of the at least one WRKY gene comprises (or consists of) the sequence of SEQ ID NO: 15 (Figure 8), or the sequence of any one SEQ ID NOs: 57, 59, 61, and 63 (Figure 15).

In various embodiments of the present disclosure, the at least one WRKY protein is selected from the group consisting of TaWRKY10 (SEQ ID NO: 4), TaWRKY13 (SEQ ID NO: 6), TaWRKY19 (SEQ ID NO: 8), TaWRKY29 (SEQ ID NO: 10), TaWRKY39 (SEQ ID NO: 12), TaWRKY53B (SEQ ID NO: 14), and TaWRKY68A (SEQ ID NO: 16), or homologs thereof.

In various embodiments of the disclosure, the amino acid sequence of the at least one WRKY protein shows at least 70% homology or sequence identity to the amino acid sequence of TaWRKY10 (SEQ ID NO: 4, Figure 2), wherein the variant WRKY10 protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY10 (SEQ ID NO: 4, Figure 2), wherein the variant protein is a WRKY protein, preferably a WRKY10 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY10 (SEQ ID NO: 4, Figure 2), wherein the variant protein is a WRKY protein, preferably a WRKY10 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY10 as described above exhibit the same fertilizer-responsive expression or activity as the native TaWRKY10 having the sequence of SEQ ID NO: 4 (Figure 2). In a particularly preferred embodiment, the amino acid sequence of the at least one WRKY protein comprises (or consists of) the sequence of SEQ ID NO: 4 (Figure 2), or the sequence of any one SEQ ID NOs: 18, 20, 22, and 24 (Figure 9).

In various embodiments of the disclosure, the amino acid sequence of the at least one WRKY protein shows at least 70% homology or sequence identity to the amino acid sequence of TaWRKY13 (SEQ ID NO: 6, Figure 3), wherein the variant protein is a WRKY protein, preferably a WRKY13 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY13 (SEQ ID NO: 6, Figure 3), wherein the variant protein is a WRKY protein, preferably a WRKY13 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY13 (SEQ ID NO: 6, Figure 3), wherein the variant WRKY13 protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY13 as described above exhibit the same fertilizer-responsive expression or activity as the native TaWRKY13 having the sequence of SEQ ID NO: 6 (Figure 3). In a particularly preferred embodiment, the amino acid sequence of the at least one WRKY protein comprises (or consists of) the sequence of SEQ ID NO: 6 (Figure 3), or the sequence of any one SEQ ID NOs: 26, 28, and 30 (Figure 10).

In various embodiments of the disclosure, the amino acid sequence of the at least one WRKY gene shows at least 70% homology or sequence identity to the amino acid sequence of TaWRKY19 (SEQ ID NO: 8, Figure 4), wherein the variant protein is is a WRKY protein, preferably a WRKY19 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY19 (SEQ ID NO: 8, Figure 4), wherein the variant protein is is a WRKY protein, preferably a WRKY19 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY19 (SEQ ID NO: 8, Figure 4), wherein the variant protein is is a WRKY protein, preferably a WRKY19 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY19 as described above exhibit the same fertilizer-responsive expression or activity as the native TaWRKY19 having the sequence of SEQ ID NO: 8 (Figure 4). In a particularly preferred embodiment, the amino acid sequence of the at least one WRKY gene comprises (or consists of) the sequence of SEQ ID NO: 8 (Figure 4), or the sequence of any one SEQ ID NOs: 32, 34, and 36 (Figure 11).

In various embodiments of the disclosure, the amino acid sequence of the at least one WRKY protein shows at least 70% homology or sequence identity to the amino acid sequence of TaWRKY29 (SEQ ID NO: 10, Figure 5), wherein the variant protein is a WRKY protein, preferably a WRKY29 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY29 (SEQ ID NO: 10, Figure 5), wherein the variant protein is a WRKY protein, preferably a WRKY29 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY29 (SEQ ID NO: 10, Figure 5), wherein the variant protein is a WRKY protein, preferably a WRKY29 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY29 as described above exhibit the same fertilizer-responsive expression or activity as the native TaWRKY29 having the sequence of SEQ ID NO: 10 (Figure 5). In a particularly preferred embodiment, the amino acid sequence of the at least one WRKY protein comprises (or consists of) the sequence of SEQ ID NO: 10 (Figure 5), or the sequence of SEQ ID NO: 38 or 40 (Figure 12).

In various embodiments of the disclosure, the amino acid sequence of the at least one WRKY protein shows at least 70% homology or sequence identity to the amino acid sequence of TaWRKY39 (SEQ ID NO: 12, Figure 6), wherein the variant protein is a WRKY protein, preferably a WRKY39 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY39 (SEQ ID NO: 12, Figure 6), wherein the variant protein is a WRKY protein, preferably a WRKY39 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY39 (SEQ ID NO: 12, Figure 6), wherein the variant protein is a WRKY protein, preferably a WRKY39 protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY39 as described above exhibit the same fertilizer-responsive expression or activity as the native TaWRKY39 having the sequence of SEQ ID NO: 12 (Figure 6). In a particularly preferred embodiment, the amino acid sequence of the at least one WRKY protein comprises (or consists of) the sequence of SEQ ID NO: 12 (Figure 6), or the sequence of any one SEQ ID NOs: 42, 44, 46, 48, and 50 (Figure 13).

In various embodiments of the disclosure, the amino acid sequence of the at least one WRKY protein shows at least 70% homology or sequence identity to the amino acid sequence of TaWRKY53B (SEQ ID NO: 14, Figure 7), wherein the variant protein is a WRKY protein, preferably a WRKY53B protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY53B (SEQ ID NO: 14, Figure 7), wherein the variant protein is a WRKY protein, preferably a WRKY53B protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY53B (SEQ ID NO: 14, Figure 7), wherein the variant protein is a WRKY protein, preferably a WRKY53B protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY53B as described above exhibit the same fertilizer-responsive expression or activity as the native TaWRKY53B having the sequence of SEQ ID NO: 14 (Figure 7). In a particularly preferred embodiment, the amino acid sequence of the at least one WRKY protein comprises (or consists of) the sequence of SEQ ID NO: 14 (Figure 7), or the sequence of any one SEQ ID NOs: 52, 54, and 56 (Figure 14).

In various embodiments of the disclosure, the amino acid sequence of the at least one WRKY protein shows at least 70% homology or sequence identity to the amino acid sequence of TaWRKY68A (SEQ ID NO: 16, Figure 8), wherein the variant protein is a WRKY protein, preferably a WRKY68A protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the amino acid sequence of the at least one WRKY protein shows at least 80% or at least 90% homology or sequence identity to the amino acid sequence of TaWRKY68A (SEQ ID NO: 16, Figure 8), wherein the variant protein is a WRKY protein, preferably a WRKY68A protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. More preferably, the amino acid sequence of the at least one WRKY protein shows at least 95%, 96%, 97%, 98%, or 99% homology or sequence identity to the amino acid sequence of TaWRKY68A (SEQ ID NO: 16, Figure 8), wherein the variant protein is a WRKY protein, preferably a WRKY68A protein, characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. In certain embodiments, variants of TaWRKY68A as described above exhibit the same fertilizer-responsive expression or activity as the native TaWRKY68A having the sequence of SEQ ID NO: 16 (Figure 8). In a particularly preferred embodiment, the amino acid sequence of the at least one WRKY protein comprises (or consists of) the sequence of SEQ ID NO: 16 (Figure 8), or the sequence of any one SEQ ID NOs: 58, 60, 62, and 64 (Figure 15).

In the present disclosure, the at least one modification inhibiting the expression or activity of the at least one WRKY protein (as compared to a corresponding control plant cell lacking the at least one modification) results in an increased pathogen resistance of a cereal plant comprising the cereal plant cell having introduced into its genome the said at least one modification inhibiting the expression or activity of the at least one WRKY protein as compared to a control plant lacking the said cereal plant cell. In other words, the at least one modification inhibiting the expression or activity of the at least one WRKY protein provides for an activation or upregulation of plant immunity against pathogens.

In accordance with the present disclosure, a pathogen as described herein generally is a plant pathogen (phytopathogen). In various preferred embodiments of the present disclosure, the at least one modification results in an increased resistance against fungal pathogens. More preferably, the fungal pathogens are pathogenic filamentous fungi. In various other preferred embodiments, the fungal pathogen is a pathogenic ascomycete, *i.e.,* a fungal pathogen belonging to the Phylum *Ascomycota.* More preferably, the pathogenic ascomycetes are filamentous ascomycete fungi. In various embodiments of the present disclosure, the pathogenic ascomycete fungi belong to the family *Mycosphaerellaceae.* Preferably, the fungal pathogen or pathogenic ascomycete is a member of the genus *Zymoseptoria.* In particular preferred embodiments, the fungal pathogen or pathogenic ascomycete is *Zymoseptoria tritici.* As described herein, the terms *Zymoseptoria tritici* and *Septoria tritici* may be used herein interchangeably. More preferably, Thus, in particularly preferred embodiments of the present disclosure, the at least one modification inhibiting the expression or activity of the at least one WRKY protein results in an increased resistance against Septoria leaf blotch disease, which is caused by fungal pathogens belonging to the genus *Zymoseptoria, in particular Zymoseptoria tritici.* In other particular preferred embodiments, the fungal pathogen or pathogenic ascomycete is *Fusarium graminearum, Fusarium culmorum, Fusarium avenaceum, Fusarium poae, Fusarium equiseti, Puccinia triticina, Puccinia striiformis* f. sp. *tritici, or Puccinia graminis*.

As described herein, the terms "Septoria disease", "Septoria leaf blotch disease", or *"Septoria tritici* blotch" may be used herein interchangeably.

In various embodiments of the present disclosure, the least one modification inhibiting the expression or activity of the at least one WRKY protein is caused or effected by mutagenesis. In various embodiments of the present disclosure, the least one modification inhibiting the expression or activity of the at least one WRKY protein is caused or effected by random mutagenesis. In various other embodiments of the present disclosure, the least one modification inhibiting the expression or activity of the at least one WRKY protein is caused or effected by targeted mutagenesis. Preferably, mutagenesis according to the present disclosure means a mutagenesis of the endogenous WRKY gene.

In various embodiments of the present disclosure, the at least one modification is caused by RNA interference, sense suppression, insertional mutagenesis, chemically induced mutagenesis or genome editing of the at least one endogenous WRKY gene. In various embodiments of the present disclosure, the least one modification is caused or effected by targeting induced local lesions in genomes (TILLING), which is a technique that was introduced at the beginning of this century using the model plant *Arabidopsis thaliana.* In various embodiments of the present disclosure, the least one modification is caused or effected by the known genome editing tools like Zinc Finger Nucleases, TAL Effector Nucleases, CRISPR/Cas9 etc. In various preferred embodiments, the least one modification is caused by virus-induced gene silencing (VIGS). In various other embodiments of the present disclosure, the at least one modification comprises one or more transposons, one or more insertions, one or more additions, one or more deletions or one or more point mutations in the at least one endogenous WRKY gene. In various embodiments of the present disclosure, the least one modification is a disruption of the at least one endogenous WRKY gene.

In various embodiments of the present disclosure, the at least one modification is introduced into the cereal plant cell, or cereal plant or part thereof, by (a) introducing at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), or (ii) at least 20 contiguous nucleotides of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, into the plant cell such that the at least one polynucleotide sequence is operatively linked to a promoter in a sense or antisense orientation; or (b) introducing at least one polynucleotide sequence comprising one or more subsequences of a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKYI9), SEQ ID NO: 9 (TaWRKY29), SEQ ID NO: 11 (TaWRKY39), SEQ ID NO: 13 (TaWRKY53B), and SEQ ID NO: 15 (TaWRKY68A), into the plant cell, wherein the at least one polynucleotide sequence is configured for RNA interference, preferably wherein the at least one polynucleotide sequence is configured for endogenous (post transcriptional) gene silencing or virus-induced gene silencing.

In various embodiments of the present disclosure, the fertilizer is a mineral fertilizer, preferably nitrogen, phosphorus, or potassium. Thus, in various embodiments of the present disclosure the fertilizer-responsive expression or activation of the at least one WRKY protein means mineral fertilizer-responsive expression or activation of the at least one WRKY protein.

In various embodiments of the present disclosure, fertilizer-responsive expression or activation of the at least one WRKY protein means nitrogen-responsive expression or activation of the at least one WRKY protein. In various other embodiments of the present disclosure, fertilizer-responsive expression or activation of the at least one WRKY protein means potassium-responsive expression or activation of the at least one WRKY protein. In still other embodiments of the present disclosure, fertilizer-responsive expression or activation of the at least one WRKY protein means phosphorus-responsive expression or activation of the at least one WRKY protein. Preferably, fertilizer-responsive expression or activation of the at least one WRKY protein means nitrogen-responsive expression or activation of the at least one WRKY protein.

The term "nitrogen fertilization", "potassium fertilization", or "phosphorus fertilization" respectively as used herein shall be interpreted to mean any man-made or artificial environmental condition in which plant-available nitrogen, potassium, or phosphorus respectively in the soil is more than would be available under natural environmental conditions, wherein the more of plant-available nitrogen in the soil causes a significant increase in yield or allows to tend to or to exploit the maximum yield potential of a specific crop or cultivar grown on the soil.

As used herein the term "inhibition of the expression or activity of a WRKY protein" or "inhibiting the expression or activity of a WRKY protein" shall be interpreted to mean any change in a level of synthesis of the functional WRKY protein or a WRKY biological activity, which can include a reduced level of functional WRKY protein present in a plant cell, reduced efficacy of the WRKY protein, reduced ability to recognize or bind the DNA at the corresponding cis-regulatory elements like the W box ((T)TGAC(C/T)) or variants thereof, or any other means which affects one or more of the biological properties of a WRKY protein of the present disclosure in relation to its role as transcriptional regulator, in particular its role as negative regulator of genes involved in plant pathogen defense. Accordingly, "inhibition of the expression or activity of a WRKY protein" encompasses a reduction in the efficacy of the protein or a reduction in the level of WRKY protein present in a plant cell, for example, due to a reduction in the expression of a WRKY gene.

In various embodiments of the present disclosure, inhibiting the expression or activity of at least one WRKY protein means inhibiting the level of expression or level of activity of the at least one WRKY gene or the at least one WRKY protein. Furthermore, in various embodiments of the present disclosure, inhibiting the expression of at least one WRKY protein is effected or targeting at the level of expression of the WRKY gene encoding the at least one WRKY protein. Inhibition of gene expression may be performed or can occur, but is not limited to, at the level of mRNA transcription or at the post-transcriptional level.

In the present disclosure, inhibiting the expression or activity of at least one WRKY protein does not necessarily mean, *i.e.,* is not limited to, a total inhibition of the expression or activity of the at least one WRKY protein. Rather, inhibiting the expression of at least one WRKY protein means that the expression of the at least one WRKY gene encoding the at least one WRKY protein is reduced or decreased to an extent that no or no significant mRNA or protein expression transcribed or encoded, respectively, by the at least one WRKY gene occurs. Techniques for measuring and determining gene expression by measuring and determining mRNA or protein expression are well known to the one of ordinary skill in the art. Thus, in various embodiments of the present disclosure, inhibiting the expression of at least one WRKY protein means inhibiting the expression of the mRNA (or mRNA transcript) derived from the at least one WRKY gene encoding the at least one WRKY protein. Additionally or alternatively, inhibiting the expression of at least one WRKY protein may mean that the expression of the at least one WRKY gene encoding the at least one WRKY protein is reduced or decreased to an extent that no or no significant activity of the at least one WRKY protein is measurable. Techniques for measuring and determining the activity of WRKYs are well known to the one of ordinary skill in the art.

In various embodiments of the present disclosure, expression of the at least one WRKY protein may mean over-expression of the at least one WRKY protein.

In various preferred embodiments of the present disclosure, "activity of at least one WRKY protein" describes the (transcriptional) activation of at least one gene regulated by the at least one WRKY protein. More preferably, the at least one gene regulated by the at least one WRKY protein is involved in the cereal plant cell or cereal plant defense mechanism/response against a pathogen. Still more preferably, activation of the at least one gene, which is regulated by the at least one WRKY protein and which is involved in the cereal plant cell defense mechanism/response against a pathogen, results in, or forms part of, a negative regulation of the defense mechanism/response of the cereal plant cell or cereal plant against a pathogen.

As described herein, the terms "WRKY" and "WRKY transcription factor" or "WRKY-type transcription factor" may be used herein interchangeably. WRKYs are transcription factors specifically found in plants. Thus, a "WRKY" according to the present disclosure may inherently be considered a "plant WRKY" or "plant cell WRKY".

Furthermore, in various embodiments of the disclosure, corresponding control plant cell, or control plant or part thereof, means that the control plant cell, or control plant or part thereof, is of the same species, preferably of the same variety or cultivar, as the cereal plant cell, or cereal plant or part thereof, having introduced into its genome the at least one modification inhibiting the expression and/or activity of the at least one WRKY protein of the present disclosure. For example, if the cereal plant cell of the disclosure is a cell of a wheat plant (*e.g.,* cv. Avalon), the corresponding control plant cell is a cell of the same wheat plant cultivar (*e.g*., cv. Avalon), but is actually lacking the at least one modification inhibiting the expression or activity of the at least one WRKY protein.

In various embodiments of the present disclosure, the cereal plant cell, or cereal plant or part thereof, is a dicotyledonous or a monocotyledonous cereal plant cell, or cereal plant or part thereof. In various embodiments of the present disclosure, the cereal plant cell is the cell of a wheat plant (*Triticum aestivum*), the cell of a rye plant *(Secale cereale),* the cell of a barley plant (*Hordeum vulgare*), the cell of a maize plant (*Zea mays*), the cell of a rice plant (*Oryza sativa*), the cell of an oat plant (*Avena sativa*), or the cell of a millet plant (*Panicum miliaceum*). In preferred embodiments, the cereal plant cell is the cell of a wheat plant (*Triticum aestivum*). In other preferred embodiments, the cereal plant cell is the cell of a rye plant (*Secale cereale*). In still other preferred embodiments, the cereal plant cell is the cell of a barley plant (*Hordeum vulgare*).

In various embodiments of the present disclosure, the cereal plant or part thereof is a wheat plant (*Triticum aestivum*) or part thereof, a rye plant (*Secale cereale*) or part thereof, a barley plant (*Hordeum vulgare*) or part thereof, a maize plant (*Zea mays*) or part thereof, a rice plant (*Oryza sativa*) or part thereof, an oat plant (*Avena sativa*) or part thereof, or a millet plant (*Panicum miliaceum*) or part thereof. In preferred embodiments, the cereal plant or part thereof is a wheat plant (*Triticum aestivum*). In other preferred embodiments, the cereal plant or part thereof is a rye plant (*Secale cereale*) or part thereof. In still other preferred embodiments, the cereal plant or part thereof is a barley plant (*Hordeum vulgare*) or part thereof.

The present disclosure provides a cereal plant comprising or containing a cereal plant cell of the disclosure and having a phenotype of increased pathogen resistance, preferably a phenotype of increased resistance against a fungal pathogen, more preferably a phenotype of increased resistance against Septoria leaf blotch disease (or *Septoria tritici blotch, STB),* Fusarium disease (*Fusarium sp*.) or rust diseases (*Puccinia sp*.).

The present disclosure provides a cereal plant having an increased pathogen resistance, preferably having an increased resistance against a fungal pathogen, more preferably having an increased resistance against Septoria leaf blotch disease (or *Septoria tritici blotch, STB),* Fusarium disease (*Fusarium sp.*) or rust diseases (*Puccinia sp*.), wherein the increased pathogen resistance results from at least one modification introduced into the genome of the plant inhibiting the expression or activity of at least one WRKY protein as compared to a corresponding control plant lacking the at least one modification, wherein the at least one WRKY protein is characterized by (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid. Preferably, the increased pathogen resistance results from: (a) a modification in at least one endogenous WRKY gene comprising a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), wherein the modification comprises one or more transposons, one or more insertions, one or more additions, one or more deletions or one or more point mutations in the at least one endogenous WRKY gene; (b) at least one introduced transgene comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), or (ii) at least 20 contiguous nucleotides of the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, wherein the transgene is operatively linked to a promoter in a sense and/or antisense orientation; or (c) at least one introduced polynucleotide sequence comprising one or more subsequences of a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), wherein the at least one polynucleotide sequence is in RNA interference configuration, preferably wherein the at least one polynucleotide sequence is configured for endogenous (post transcriptional) gene silencing or virus-induced gene silencing.

In various embodiments of the present disclosure, the cereal plant is a hybrid cereal plant.

In various embodiments of the disclosure, the promoter is a tissue-specific promoter, a temporally regulated promoter, or an inducible promoter, preferably a pathogen inducible promoter.

The present disclosure provides a method for conferring pathogen resistance to, or increasing pathogen resistance of, a cereal plant cell, or a cereal plant or part thereof, the method comprising the step of inhibiting the expression or activity of at least one WRKY protein of the present disclosure in a cereal plant cell, or a cereal plant or part thereof, as compared to a corresponding control plant cell, or control plant or part thereof, lacking the inhibition.

The present disclosure also provides a method of producing a cereal plant cell, or a cereal plant or part thereof, having an increased pathogen resistance as compared to a corresponding control plant cell, or control plant or part thereof, the method comprising the step of inhibiting the expression or activity of at least one WRKY protein of the present disclosure in a cereal plant cell, or a cereal plant or part thereof, as compared to a corresponding control plant cell, or plant or part thereof, lacking the inhibition.

In various embodiments of the methods of the present disclosure, the step of inhibiting the expression or activity of at least one WRKY protein comprises introducing into the genome of the cereal plant cell, or a cereal plant or part thereof, at least one modification inhibiting the expression or activity of the at least one WRKY protein as compared to a corresponding control plant cell, or control plant or part thereof, lacking the at least one modification.

In various embodiments of the methods of the present disclosure, the at least one modification comprises one or more transposons, one or more insertions, one or more additions, one or more deletions or one or more point mutations in the at least one endogenous WRKY gene.

In various embodiments of the methods of the present disclosure, the at least one modification is introduced into the cereal plant cell, or cereal plant or part thereof, by (a) introducing at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A) or (ii) at least 20 contiguous nucleotides of the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, into the cereal plant cell, or cereal plant or part thereof, such that the at least one polynucleotide sequence is operatively linked to a promoter in a sense and/or antisense orientation; or (b) introducing at least one polynucleotide sequence comprising one or more subsequences of a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), into the plant cell, or plant or part thereof, wherein the at least one polynucleotide sequence is configured for RNA interference, preferably wherein the at least one polynucleotide sequence is configured for endogenous (post transcriptional) gene silencing or virus-induced gene silencing.

The present disclosure provides a method of identifying a cereal plant cell, or a cereal plant or part thereof, having an increased pathogen resistance as compared to a corresponding control plant cell, or control plant or part thereof, the method comprising the step of identifying a cereal plant cell, or a cereal plant or part thereof, wherein the expression or activity of at least one WRKY protein of the present disclosure is inhibited as compared to a corresponding control plant cell, or plant or part thereof, lacking the inhibition.

In various embodiments of the present disclosure, the cereal plant cell, or cereal plant or part thereof, comprises a heterozygous or homozygous mutation in the at least one WRKY gene encoding the at least one WRKY protein, wherein the mutation causes the inhibition of the expression or activity of the at least one WRKY protein encoded by the at least one WRKY gene.

In various embodiments of the present disclosure, the above mutant form is introduced into the cereal plant cell, or cereal plant or part thereof, by Agrobacterium-mediated transfer, electroporation, micro-projectile bombardment, or sexual cross. The techniques of Agrobacterium-mediated transfer, electroporation, micro-projectile bombardment, and sexual cross are well-known to the skilled person and methods are described in the literature. For example, transformation of cereals via particle bombardment may be performed as described in Sparks and Jones, 2014, Genetic transformation of wheat via particle bombardment, Methods Mol. Biol. 1099, 201-218.

In various embodiments of the present disclosure, the cereal plant cell, or cereal plant or part thereof, has an increased resistance against a fungal pathogen, preferably an increased resistance against Septoria leaf blotch disease (or *Septoria tritici blotch, STB),* Fusarium disease (*Fusarium sp*.) or rust diseases (*Puccinia sp*.).

The present disclosure provides a cereal plant cell, or a cereal plant or part thereof, produced by any of the methods of the present disclosure.

The present invention provides a recombinant DNA construct comprising a polynucleotide sequence, or a fragment thereof, operatively linked to a heterologous promoter, wherein the polynucleotide sequence, or a fragment thereof, produces upon expression in a cereal plant cell, or cereal a plant or part thereof: (a) a protein that interferes with the expression or activity of at least one endogenous WRKY protein of the present disclosure; (b) an RNA molecule that suppresses the expression of at least one endogenous WRKY protein of the present disclosure; or (c) a loss-of-function mutation in at least one endogenous WRKY gene of the present disclosure, wherein the polynucleotide sequence comprises a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and homologs thereof, preferably in sense and/or in antisense.

The present disclosure provides a method of producing a cereal plant cell, or a cereal plant or part thereof, having an increased pathogen resistance as compared to a corresponding control plant cell, or control plant or part thereof, the method comprising the steps of (a) incorporating into a cereal plant cell, or a cereal plant or part thereof, a recombinant DNA construct of the present disclosure; and (b) selecting a cereal plant cell, or a cereal plant or part thereof, comprising the said recombinant DNA construct and having an increased pathogen resistance as compared to a corresponding control plant cell, or control plant or part thereof, that does not comprise the said recombinant DNA construct.

The present disclosure provides a cereal plant cell, or a cereal plant or part thereof, comprising a recombinant DNA construct of the present disclosure and having an increased pathogen resistance as compared to a corresponding control plant cell, or plant or part thereof, that does not comprise the said recombinant DNA construct.

The present disclosure provides a method of producing a cereal plant having an increased pathogen resistance as compared to a corresponding control plant, the method comprising obtaining and planting seeds produced by a cereal plant of the present disclosure, wherein the seed comprises a recombinant DNA construct of the present disclosure, and wherein the plant grown from the planted seed is a progeny plant having an increased pathogen resistance as compared to a corresponding control plant that does not comprise the said recombinant DNA construct.

The present disclosure provides a double-stranded RNA (dsRNA) molecule comprising: (a) a first strand comprising a nucleotide sequence having a length of at least 19, 20, 21, 22, 23, 24, 25, 30, 40 or 50 nucleotides, preferably at least 60, 70, 80, 90 or 100 nucleotides, more preferably at least 150, 200, 250, 300, 400, 500, 750 or 1000 nucleotides and having at least 90%, 91%, 92%, 93% or 94%, more preferably 95%, 96%, 97%, 98%, 99% or even 100% sequence identity to consecutive nucleotides of a target nucleic acid sequence; and (b) a second strand comprising in the 5' to 3' direction a nucleotide sequence which is at least 90%, 91%, 92%, 93% or 94%, more preferably 95%, 96%, 97%, 98%, 99% or even 100% complementary to the first strand and/or is able to hybridize with the first strand, preferably under stringent conditions, wherein the target nucleic acid sequence is a coding region of an mRNA deduced from a nucleic acid sequence, or a complement thereof, comprising at least 70% sequence identity to the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A).

The present disclosure provides a composition comprising a dsRNA molecule of the present disclosure.

The present disclosure provides a method of regulating the expression of at least one WRKY gene of the present disclosure in a cereal plant cell, or cereal plant or part thereof, the method comprising applying a composition comprising a dsRNA molecule of the present disclosure to a cereal plant cell, or cereal plant or part thereof, preferably wherein the composition is applied onto the surface of the plant cell, or plant or part thereof.

The present disclosure provides a cereal plant cell, or a cereal plant or part thereof, comprising a dsRNA molecule of the present disclosure and having an increased pathogen resistance as compared to a corresponding control plant cell, or control plant or part thereof, that does not comprise the said dsRNA molecule.

The present disclosure provides the use of at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKYI3), SEQ ID NO: 7 TaWRKYI9), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, as molecular marker for identifying a cereal plant cell, or a cereal plant or part thereof, having an increased pathogen resistance as compared to a corresponding control plant cell.

The present disclosure provides a method of identifying a cereal plant cell, or a cereal plant or part thereof, having an increased pathogen resistance as compared to a corresponding control plant cell, the method comprising the use of at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, as a molecular marker.

The present disclosure provides a composition for topical application to a cereal plant cell, or a cereal plant or part thereof, comprising at least one polynucleotide sequence comprising a nucleic acid sequence, or a complement thereof, comprising (i) at least 70% sequence identity to the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleic acid sequence of any one of SEQ ID NO: 3 (TaWRKY10), SEQ ID NO: 5 (TaWRKY13), SEQ ID NO: 7 TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, preferably in sense and/or in antisense, wherein the at least one polynucleotide sequence is configured for RNA interference, preferably wherein the at least one polynucleotide sequence is configured for endogenous (post transcriptional) gene silencing or virus-induced gene silencing.

The present disclosure provides the use of a recombinant DNA construct of the present disclosure for conferring pathogen resistance to, or increasing pathogen resistance of, a cereal plant cell, or a cereal plant or part thereof.

The present disclosure provides the use of a dsRNA molecule of the present disclosure for conferring pathogen resistance to, or increasing pathogen resistance of, a cereal plant cell, or a cereal plant or part thereof.

The present disclosure provides the use of the composition for topical application to a cereal plant cell, or a cereal plant or part thereof, of the present disclosure for conferring pathogen resistance to, or increasing pathogen resistance of, a cereal plant cell, or a cereal plant or part thereof.

In various embodiments of the present disclosure, the said nucleic acid sequence, or a complement thereof, comprising (i) at least 70% sequence identity to SEQ ID NO: 3 (TaWRKY10), or (ii) at least 20 contiguous nucleotides of SEQ ID NO: 3 (TaWRKY10), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 3 (TaWRKY10), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 3 (TaWRKY10), optionally in sense and/or in antisense. In particularly preferred embodiments, the said nucleic acid sequence, or a complement thereof, comprises (or consists of) the sequence of SEQ ID NO: 3 (TaWRKY10).

In various other embodiments of the present disclosure, the said nucleic acid sequence, or a complement thereof, comprising (i) at least 70% sequence identity to SEQ ID NO: 5 (TaWRKY13), or (ii) at least 20 contiguous nucleotides of SEQ ID NO: 5 (TaWRKY13), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 5 (TaWRKY13), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 5 (TaWRKY13), optionally in sense and/or in antisense. In particularly preferred embodiments, the said nucleic acid sequence, or a complement thereof, comprises (or consists of) the sequence of SEQ ID NO: 5 (TaWRKY13).

In various other embodiments of the present disclosure, the said nucleic acid sequence, or a complement thereof, comprising (i) at least 70% sequence identity to SEQ ID NO: 7 (TaWRKY19), or (ii) at least 20 contiguous nucleotides of SEQ ID NO: 7 (TaWRKY19), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 7 (TaWRKY19), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 7 (TaWRKY19), optionally in sense and/or in antisense. In particularly preferred embodiments, the said nucleic acid sequence, or a complement thereof, comprises (or consists of) the sequence of SEQ ID NO: 7 (TaWRKY19).

In various other embodiments of the present disclosure, the said nucleic acid sequence, or a complement thereof, comprising (i) at least 70% sequence identity to SEQ ID NO: 9 (TaWRKY29), or (ii) at least 20 contiguous nucleotides of SEQ ID NO: 9 (TaWRKY29), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 9 (TaWRKY29), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 9 (TaWRKY29), optionally in sense and/or in antisense. In particularly preferred embodiments, the said nucleic acid sequence, or a complement thereof, comprises (or consists of) the sequence of SEQ ID NO: 9 (TaWRKY29).

In various other embodiments of the present disclosure, the said nucleic acid sequence, or a complement thereof, comprising (i) at least 70% sequence identity to SEQ ID NO: 11 (TaWRKY39), or (ii) at least 20 contiguous nucleotides of SEQ ID NO: 11 (TaWRKY39), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 11 (TaWRKY39), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 11 (TaWRKY39), optionally in sense and/or in antisense. In particularly preferred embodiments, the said nucleic acid sequence, or a complement thereof, comprises (or consists of) the sequence of SEQ ID NO: 11 (TaWRKY39).

In various other embodiments of the present disclosure, the said nucleic acid sequence, or a complement thereof, comprising (i) at least 70% sequence identity to SEQ ID NO: 13 (TaWRKY53B), or (ii) at least 20 contiguous nucleotides of any one of SEQ ID NO: 13 (TaWRKY53B), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 13 (TaWRKY53B), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 13 (TaWRKY53B), optionally in sense and/or in antisense. In particularly preferred embodiments, the said nucleic acid sequence, or a complement thereof, comprises (or consists of) the sequence of SEQ ID NO: 13 (TaWRKY53B).

In various other embodiments of the present disclosure, the said nucleic acid sequence, or a complement thereof, comprising (i) at least 70% sequence identity to SEQ ID NO: 15 (TaWRKY68A), or (ii) at least 20 contiguous nucleotides of SEQ ID NO: 15 (TaWRKY68A), optionally in sense and/or in antisense, preferably comprises (i) at least 80%, 85%, or 90%, more preferably at least 95%, 96%, 97%, 98% or 99% sequence identity to the nucleotide sequence of SEQ ID NO: 15 (TaWRKY68A), or (ii) at least 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of SEQ ID NO: 15 (TaWRKY68A), optionally in sense and/or in antisense. In particularly preferred embodiments, the said nucleic acid sequence, or a complement thereof, comprises (or consists of) the sequence of SEQ ID NO: 15 (TaWRKY68A).

In the present disclosure, the terms "cereals", "crops" and "cereal crops" may be used interchangeably. As described herein, the term "cereals" encompasses "cereal plants" and "cereal crops".

A "control" or "control plant" or "control plant cell" provides a reference point for measuring changes in phenotype of a subject plant or plant cell in which genetic modification or alteration, such as transformation or mutation or knock-down or knock-out, has been effected to a gene of interest. A control plant or control plant cell may comprise, *for example:* (a) a wild-type plant or cell, *i.e.,* of the same genotype as the starting material for the genetic modification or alteration which resulted in the subject plant or cell; (b) a plant or plant cell of the same genotype as the starting material but which has been transformed with a null construct (*i.e.,* with a construct which has no known effect on the trait of interest, such as a construct comprising a marker gene); or (c) a plant or plant cell which is a non-transformed segregant among progeny of a subject plant or plant cell. A control plant or control plant cell may also be a plant or plant cell transformed with an alternative WRKY down-regulation construct.

By "encoding" or "encoded" with respect to a specified nucleic acid, is meant comprising the information for translation into the specified protein. A nucleic acid encoding a protein may comprise non-translated sequences (*e.g*., introns) within translated regions of the nucleic acid, or may lack such intervening non-translated sequences (*e.g*., as in cDNA). The information by which a protein is encoded is specified by the use of codons. Typically, the amino acid sequence is encoded by the nucleic acid using the "universal" genetic code.

The term "down-regulation" or "knock-down" refers to a partial reduction, the term "knock-out" to a complete reduction. For example, down-regulation of a WRKY polynucleotide in a plant or cell encompasses a reduction in expression to a level that is 99%, 95%, 90%, 85%, 80%, 75%, 70%, 65%, 60%, 55%, 50%, 45%, 40%, 35%, 30%, 25%, 20%, 15%, 10%, 5% or 0 % of the expression level of the corresponding WRKY polynucleotide in a control plant or control plant cell.

As used herein, "nucleic acid" includes reference to a deoxyribonucleotide or ribonucleotide polymer in either single- or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids).

As used herein, the term "plant" includes reference to whole plants, plant organs (e.g., leaves, stems, roots, etc.), embryos, calluses, seeds and progeny of same. Plant cell, as used herein includes, without limitation, cells in or from seeds, suspension cultures, embryos, meristematic regions, callus tissue, leaves, roots, shoots, gametophytes, sporophytes, pollen, ovules and microspores. The class of plants which can be used in the methods of the disclosure is generally as broad as the class of higher plants amenable to transformation techniques, including both monocotyledonous and dicotyledonous plants including species from the genera: Oryza, Avena, Hordeum, Secale, Panicum, Zea and Triticum. A particularly preferred plant is Triticum aestivum.

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

As used herein, the term "WRKY protein" or "WRKY polypeptide" is also inclusive of fragments, variants, homologs, alleles or precursors (e.g., preproproteins or proproteins) thereof. A "WRKY protein" comprises a WRKY polypeptide.

The term "subsequence" refers to a sequence of a nucleic acid that comprises a part of a longer sequence of a nucleic acid. As used herein, a subsequence suitable as molecular marker, e.g. as a hybridization probe, is about 10-140 nucleotides in length, preferably about 15-100 nucleotides in length, more preferably about 20-50 nucleotides in length; a subsequence, as used in the context of an RNA interference configuration, consists of at least 19, 20, 21, 22, 23, 24, 25, 30, 40 or 50 contiguous nucleotides, preferably at least 60, 70, 80, 90 or 100 contiguous nucleotides, more preferably at least 150, 200, 250, 300, 400, 500, 750 or 1000 contiguous nucleotides of a given longer (reference) sequence of a nucleic acid.

As used herein, "sequence identity" or "identity" in the context of two nucleic acid or polypeptide sequences includes reference to the residues in the two sequences, which are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. Where sequences differ in conservative substitutions, the percent sequence identity may be adjusted upwards to correct for the conservative nature of the substitution. Sequences, which differ by such conservative substitutions, are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., according to the algorithm of Meyers and Miller, (1988) Computer Applic. Biol. Sci. 4:1 1-17, e.g., as implemented in the program PC/GENE (Intelligenetics, Mountain View, California, USA).

As used herein, "percentage of sequence identity" means the value determined by comparing two optimally aligned sequences over a comparison window, wherein the portion of the polynucleotide sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. The term "homology" refers to analogies or similarities in the nucleotide sequence of two nucleic acid molecules or the amino acid sequence of two proteins or peptides. The presence of homology between two nucleic acids or proteins can be detected by comparing one position in one sequence with the corresponding position in the other sequence and determining whether identical or similar residues are present here. Two compared sequences are homologous if there is a minimum level of identical or similar nucleotides. "Identical" means that when comparing two sequences at equivalent points in each case there is the same nucleotide or the same amino acid. It may be necessary to take into account gaps in sequence to produce the best possible alignment comparison of sequences. Similar nucleotides/amino acids are non-identical nucleotides/amino acids with the same or equivalent physical and chemical properties. Exchanging a nucleotide (an amino acid) with a different nucleotide (another amino acid) with the same or equivalent physical and chemical properties is called a "conservative exchange." Examples of physico-chemical properties of an amino acid include, for example, the hydrophobicity or charge. In the context of nucleic acids there is also understood a conservative or a similar nucleotide exchange when replacing a nucleotide in a coding sequence in a codon by another, but, due to the degeneration of the genetic code, the same amino acid or a similar amino acid sequence as in the comparison of the codon affected by the exchange is encoded. The skilled worker knows which nucleotide or amino acid exchange is a conservative exchange. To determine the degree of similarity or identity between two nucleic acids, a minimum length of 60 nucleotides or base pairs is assumed, preferably a minimum length of 70, 80, 90, 100, 110, 120, 140, 160, 180, 200, 250, 300, 350 or 400 nucleotides or base pairs, more preferably the full length of the compared nucleic acids, and in the case of proteins / peptides a minimum length of 20 amino acids is assumed, preferably a minimum length of 25, 30, 35, 40, 45, 50, 60, 80, 100, 150, 200, 250 or 300 amino acids, and particularly preferably the full length of the compared amino acid sequences. The degree of similarity ("positives") or identity of two sequences can be determined using, for example, the computer program BLAST (Altschul S.F. et al (1990), Basic Local Alignment Search Tool, J. Mol Biol 215: 403-410; see e.g. http://www.ncbi.nlm.nih.gov/BLAST/) using standard parameters. The determination of homology depends on the length of the compared sequences. For the purposes of the present invention a homology between two nucleic acid sequences, whose shorter one's length is at least 100 nucleotides, is established if at least 70%, at least 75%, at least 80%, at least 85%, at least 90%, at least 95%, at least 97%, at least 98%, or at least 99% of nucleotides are identical and/or similar ("identities" or "positives" according to BLAST), and preferably are identical. In the case of a sequence length of 50-99 nucleotides a homology between sequences is understood where there is identity or similarity of at least 80%, preferably at least 85%, 86%, 87%, 88% or 89%, with a sequence length of 15-49 nucleotides with an identity or similarity of at least 90%, preferably at least 95%, 96%, 97%, 98% or 99%. In the case of proteins a homology is assumed, when using the computer program BLAST using standard parameters and the BLOSUM62 substitution matrix (Henikoff, S., and Henikoff, J., Amino acid substitution matrices from protein blocks. Proc. Natl. Acad. Sci. USA 89: 10915-10919, 1992) an identity ("identities") and/or likeness ("positive"), preferably identity, at least 25%, at least 26%, at least 27%, at least 28%, at least 29% at least 30%, preferably at least 35%, at least 40%, at least 45%, at least 50%, at least 55%, at least 60%, at least 65%, at least 70%, at least 75%, at least 80%, at least 85%, at least 90%, at least 95%, at least 97%, at least 98%, or at least 99% is obtained, preferably the entire length of the protein/peptide, which is compared with another protein, e.g. the length of 225 amino acids in the case of SEQ ID NO: 4. The person working in this art is able with his expert knowledge to use readily available BLAST programs (e.g. BLASTn, BLASTp, BLASTx, tBLASTn or tBLASTx) to determine the homology in question. In addition, there are other programs that the expert knows, and which he can use in the case in assessing the homology of two or more comparative sequences or partial sequences. Such programs include those that can be found, for example on the website of the European Bioinformatics Institute (EMBL) (see, e.g. http://www.ebi.ac.uk/Tools/similarity.html).

The term "hybridizing" or "hybridization" means a process in which a single-stranded nucleic acid molecule attaches itself to a complementary nucleic acid strand, *i.e.,* agrees with this base pairing. Standard procedures for hybridization are described, for example, in Sambrook et al. (Molecular Cloning. A Laboratory Manual, Cold Spring Harbor Laboratory Press, 3rd edition 2001). Preferably this will be understood that at least 50%, more preferably at least 55%, 60%, 65%, 70%, 75%, 80% or 85%, more preferably 90%, 91%, 92%, 93%, 94% received 95%, 96%, 97%, 98% or 99% of the bases of the nucleic acid strand shows a base pairing with the complementary nucleic acid strand. The possibility of such attachment depends on the stringency of the hybridization conditions. The term "stringency" refers to hybridization conditions. High stringency is when base pairing is more difficult, low stringency, when a base-pairing is facilitated. The stringency of hybridization conditions depends for example on the salt concentration or ionic strength and temperature. Generally, the stringency can be increased by increasing the temperature and/or decreasing salinity. "Stringent hybridization conditions" are defined as conditions in which hybridization occurs predominantly only between homologous nucleic acid molecules. The term "hybridization conditions" refers not only to the actual attachment of the nucleic acids at the prevailing conditions, but also in the subsequent washing steps prevailing conditions. Stringent hybridization conditions are, for example, conditions under which predominantly only those nucleic acid molecules having at least 70%, preferably at least 75%, at least 80%, at least 85%, at least 90% or at least 95% sequence identity hybridize. Less stringent hybridization conditions include: hybridization in 4 x SSC at 37°C, followed by repeated washing in 1 x SSC at room temperature. Stringent hybridization conditions include: hybridization in 4 x SSC at 65°C, followed by repeated washing in 0.1 x SSC at 65°C for a total of about 1 hour. The term "complementary" refers to the ability of purine and pyrimidine nucleotides to form base pairs with each other via bridging hydrogen bonds. Complementary base pairs are, for example, guanine and cytosine, adenine and thymine and adenine and uracil. A complementary nucleic acid strand is accordingly a nucleic acid strand that can, by pairing with complementary bases of another nucleic acid strand, form a double strand.

### Plant Transformation Methods

Numerous methods for introducing foreign or heterologous DNA into plants are known and can be used in the present disclosure, including biological and physical plant transformation protocols. See, e.g., Miki, et al., "Procedure for Introducing Foreign DNA into Plants", in Methods in Plant Molecular Biology and Biotechnology, Glick and Thompson, eds., CRC Press, Inc., Boca Raton, pp. 67-88 (1993). The methods chosen vary with the host plant and include chemical transfection methods such as calcium phosphate, microorganism-mediated gene transfer such as Agrobacterium (Horsch et al., Science 227:1229-31 (1985)), electroporation, micro-injection and biolistic bombardment. Expression cassettes and vectors and in vitro culture methods for plant cell or tissue transformation and regeneration of plants are known and available. See, e.g., Gruber, et al., "Vectors for Plant Transformation" in Methods in Plant Molecular Biology and Biotechnology, supra, pp. 89-1 19. The isolated polynucleotides or polypeptides may be introduced into the plant by one or more techniques typically used for direct delivery into cells. Such protocols may vary depending on the type of organism, cell, plant or plant cell, e.g., monocot or dicot, targeted for gene modification. Suitable methods of transforming plant cells include microinjection (Crossway, et al., (1986) Biotechniques 4:320-334 and US Patent Number 6,300,543), electroporation (Riggs, et al., (1986) Proc. Natl. Acad. Sci. USA 83:5602-5606, direct gene transfer (Paszkowski et al., (1984) EMBO J. 3:2717-2722) and ballistic particle acceleration (see, for example, Sanford, et al., US Patent Number 4,945,050; WO 91/10725 and McCabe, et al., (1988) Biotechnology 6:923-926). Also see, Tomes, et al., "Direct DNA Transfer into Intact Plant Cells Via Microprojectile Bombardment", pp. 197- 213 in Plant Cell, Tissue and Organ Culture, Fundamental Methods, eds. Gamborg and Phillips, Springer-Verlag Berlin Heidelberg New York, 1995.

A generally applicable method of plant transformation is microprojectile-mediated transformation, where DNA is carried on the surface of microprojectiles measuring about 1 to 4 µm. The expression vector is introduced into plant tissues with a biolistic device that accelerates the microprojectiles to speeds of 300 to 600 m/s which is sufficient to penetrate the plant cell walls and membranes (Sanford, et al., (1987) Part. Sci. Technol. 5:27; Sanford, (1988) Trends Biotech 6:299; Sanford, (1990) Physiol. Plant 79:206 and Klein, et al., (1992) Biotechnology 10:268).

### Inhibiting/reducing the Activity and/or Level of a WRKY Polypeptide

Means and methods are provided to reduce or eliminate the level or activity of a WRKY polypeptide by transforming a plant cell with an expression cassette that expresses a polynucleotide that reduces the expression of the WRKY polypeptide. The polynucleotide may reduce the expression of the WRKY polypeptide directly, by preventing transcription or translation of the WRKY messenger RNA, or indirectly, by encoding a polypeptide that reduces the transcription or translation of a WRKY gene encoding a WRKY polypeptide. Methods for reducing or eliminating the expression of a gene in a plant are well known in the art and any such method may be used in the present disclosure to reduce the expression of a WRKY polypeptide. The expression of a WRKY polypeptide is reduced or inhibited if the level of the WRKY polypeptide is less than 100%, 99% 95%, 90%, 85%, 80%, 75%, 70%, 65%, 60%, 55%, 50%, 45%, 40%, 35%, 30%, 25%, 20%, 15%, 10%, 5% or 1 % of the level of the same WRKY polypeptide in a control plant cell or plant. In particular embodiments, the level of the WRKY polypeptide in a modified plant is less than 60%, less than 50%, less than 40%, less than 30%, less than 20%, less than 10%, less than 5% or less than 2% of the level of the same or a related WRKY polypeptide in a control plant cell or plant. The WRKY polynucleotide expression level and/or polypeptide level and/or activity may be reduced such that the reduction is phenotypically sufficient to provide resistance against a pathogen, in particular resistance against a fungal pathogen. The level or activity of one or more WRKY polynucleotides, polypeptides or enzymes may be impacted. The expression level of the WRKY polypeptide may be measured directly, for example, by assaying for the quantity of WRKY polypeptide expressed in the plant cell or plant, or indirectly, for example, by measuring the WRKY activity in the plant cell or plant, *i.e.,* the efficacy or ability to recognize or bind the (genomic) DNA at the corresponding *cis*-regulatory elements like the W box ((T)TGAC(C/T)) or variants thereof, or by measuring the phenotypic changes in the plant.

In certain embodiments of the disclosure, the activity of a WRKY polypeptide may be reduced or eliminated by disrupting or excising at least a part of the gene encoding the WRKY polypeptide. Mutagenized plants that carry mutations in WRKY genes also result in reduced expression of the WRKY gene and/or reduced activity of the encoded WRKY polypeptide. Thus, many methods may be used to reduce or eliminate the activity of a WRKY polypeptide. One or more methods may be used to reduce the activity of a single WRKY polypeptide. One or more methods may be used to reduce the activity of multiple WRKY polypeptides.

As described herein, the term "vector" may encompass both single-stranded and double-stranded vectors, including, but not limited to, single-stranded and double-stranded DNA vectors.

In various embodiments, a vector used for WRKY gene silencing according to the present disclosure is plasmid DNA. Plasmid DNA containing one or more target polynucleotides as described herein is readily constructed using standard techniques well known in the art. The vector may be typically constructed in a plasmid form that can then be used for transfection or transformation. The plasmid generally comprises sequences useful for replication of the plasmid in plant cells. Such plasmids are well known in the art. In addition, vectors may also include a gene whose expression confers a detectable or selectable marker such as a drug resistance. As described herein, the vector may comprise at least one regulatory expression sequence (expression control sequence).

In various embodiments, the promoter may be a tissue specific promoter. In some embodiments, the promoter is a target cell-specific promoter.

When targeting delivery of a vector to a particular target cell or target tissue, the vector genome will usually contain a promoter that is recognized by the target cell or target tissue and that is operatively linked to the sequence(s) of interest.

Promoters may be inducible, constitutive, temporally active or tissue specific. Inducible promoters are useful tools in genetic engineering because the expression of genes to which they are operatively linked can be turned on or off, *e.g.*, in a particular tissue. Inducible promoters can be grouped as chemically-regulated promoters, and physically-regulated promoters. Typical chemically-regulated promoters and typical physically-regulated promoters are well known to the person skilled in the art.

The terms nucleic acid sequence(s) and polynucleotide(s) may be used interchangeably herein. As described herein, the term "nucleic acid sequence" may encompass both single-stranded and double-stranded nucleic acid sequences. In various embodiments, a nucleic acid sequence is DNA sequence.

It is to be acknowledged that the present invention is not limited to the particular methodology, protocols, cell lines, genera, and reagents described herein, as such may vary. It is also to be acknowledged that the terminology used herein is for the purpose of describing particular embodiments only, and is not intended to be limiting the scope of the present invention.

The following examples are offered by way of illustration and not by way of limitation.

Other embodiments and uses will be apparent to one skilled in the art in light of the present disclosures. The following examples are provided merely as illustrative of various embodiments and shall not be construed to limit the invention in any way.

### EXAMPLES

### Example 1: Glasshouse experiments linking WRKYs to nitrogen regimen

### Wheat varieties, germination procedure and growth conditions

Sixty wheat *(Triticum aestivum)* seeds from each of two varieties (*Cordiale* and *Gallant*) were germinated in plastic trays. Four germinated seeds were planted 1 cm below the surface in each of 15 cm plastic plant pots filled with SilverSand. A fine mesh was placed in the base of the pots to contain the sand. The pots were split into 5 groups at random, and each group assigned a different nitrogen level - 100%, 90%, 70%, 50% or 30%. This gave 3 biological replicates for each nutrient level. Plants were stood in Hoagland's solution containing differing levels of nutrient according to Table 1.

**Table 1: Volume (in cm³) of each stock solution added per litre of final Hoaglands solution.**

| **Nutrient stock solution** | **100% N** | **90% N** | **70% N** | **50% N** | **30% N** |
|---|---|---|---|---|---|
| **Ca(NO₃)₂.4H₂O** | 2.5 | 2.25 | 1.75 | 1.25 | 0.75 |
| **CaCl2** | 0 | 0.25 | 0.75 | 1.25 | 1.75 |
| **KNO3** | 2.5 | 2.25 | 1.75 | 1.25 | 0.75 |
| **K2SO4** | 0 | 0.25 | 0.75 | 1.25 | 1.75 |
| **KH2PO4** | 1 | 1 | 1 | 1 | 1 |
| **MgSO4.7H2O** | 1 | 1 | 1 | 1 | 1 |
| **"Micronutrients"** | 0.5 each | 0.5 each | 0.5 each | 0.5 each | 0.5 each |

Plants were grown in a controlled environment chamber under standard conditions.

### Tissue collection, RNA extraction and cDNA synthesis

After 5 weeks, when plants were between the 4th to 6th leaf stages, all leaves were removed from all plants and snap frozen in liquid nitrogen. All the 4 plants in a single pot were pooled to form a single biological replicate. Leaf samples were freeze-dried for 48 hours, before being placed in 2ml Eppendorf tubes and homogenized using a TissueLyser (QIAGEN). PureLink RNA Mini Kit (Life Technology) was used for RNA extraction followed by and on-column DNase treatment.

RNA was quantified using a spectrophotometer before diluting to achieve 2 µg RNA in 16 µL. cDNA synthesis was done using the iScript Reverse Transcriptase kit (Life Technologies) following the manufactures protocol.

### Primer design and qRT-PCR

Primers for WRKY10, WRKY13, WRKY19, WRKY29, WRKY39, WRKY53B and WRKY68B were designed using Primer3Plus software (http://primer3plus.com/).

qPCR was performed using SYBR green-based dsDNA dye in the RotorGene platform (QIAGEN). The RotorDisc was heat sealed with a permanent seal, and run using the following conditions: 95°C for 300 seconds, followed by 40 cycles of 95°C for 5 second and 70°C for 10 seconds. For melt curve analysis, the temperature was increased by 10°C for between 60°C and 95°C with 5 readings per temperature.

### Data analysis and statistics

Relative expression was calculated using the ΔΔCT method as described in Livak and Schmittgen (2001, Analysis of relative gene expression data using real-time quantitative PCR and the 2-ΔΔCT method. *methods* 25.4 (2001): 402-408) using the reference gene ubiquitin. ANOVA was used to determine if the difference in ΔCT was significant between the control and treatment. A Tukey post-hoc test was applied to determine which treatments differed significantly from the control.

### Example 2: Glasshouse experiments linking the WRKYs Septoria infection

### Wheat varieties, germination procedure and growth conditions

Sixty wheat *(Triticum aestivum)* seeds from each of two varieties (*Cordiale* and *Gallant*) were germinated in plastic trays. Six seeds were planted in 15cm plastic plant pots filled with John Innes No2 compost. They were grown for 3 weeks in a temperature controlled growth room under standard conditions.

### Septoria infection

After 3 weeks, three of the pots from each variety were removed from the growth room for inoculation. A solution of *Zymoseptoria tritici* spores containing between 500 000 and 1 million spores per ml was made. This solution was applied to the underside of one leaf from each plant, with all other leaves removed. These pots were covered with a plastic lid to maintain humidity, and placed in a growth cabinet at 25°C. Both infected and healthy plants were watered as required, and grown for a further three weeks to allow for infection to develop.

### Tissue collection, RNA extraction and cDNA synthesis

Tissue was collected from infected and controls plants by taking just the one leaf per plant, with a pool of the 5 plants from each pot making a single biological replicate. Leaves were snap frozen in liquid nitrogen. RNA extraction, cDNA synthesis, qRT-PCR and statistical analysis is as previously described.

### Example 3: Field experiments linking WRKYs to Septoria disease and sensitivity to nitrogen

### Wheat varieties, trial establishment and agronomic conditions

An experimental trial was sown at a field site near Rosemaund, Herefordshire, UK (SO 55793 48437) in September using standard agronomic practices. A total of 128 treatment plots were arranged in a compete randomised block design consisting of 8 winter wheat varieties, 2 nitrogen rates and 2 fungicide levels and 4 replicates.

### Treatment conditions

Fungicide treatments consistened of treatemenst to selectively control for all expected diseases expect Septoria, whereas the control tratment contained active ingretidents which are effective again the disease and following normal agricultural practice Nitrogen treatment consisted of a reduced artificial N application relative to the control which contained ∼200kg/ha of N.

### Data collection and analysis

Flag leaf tissue is collected at GS39 in duplicate from 15 plants per plot and pooled to form 2 technical replicates. Leaves were snap frozen in liquid nitrogen. RNA extraction, cDNA synthesis, qRT-PCR and statistical analysis are as previously described.

*Septoria* disease is scored in the plots in the last 2 weeks of June according to normal practice. The plots are harvested at maturity to measure yield.

### Example 4: Construction of Agrobacterium mediated BSMV vectors for VIGS

*Barley stripe mosaic virus* (BSMV) vectors suitable for cereal virus-induced gene silencing (VIGS) have been described previously (*see*, *e.g.,* Yuan et. al., 2011, PLoS ONE, Vol. 6(10), e26468, and references cited therein). The tripartite genome consists of RNAs α, β, and γ, which each of the genomic (g) RNAs has a methylated 5' cap and a 3' polyadenylate sequence followed by a tyrosine accepting tRNA-like structure. RNAα of the BSMV ND18 strain encodes the methyltransferase/helicase subunit of the RNA-dependent RNA polymerase (RdRp). RNAβ specifies the coat protein (CP) and three major triple gene block (TGB) proteins (TGB1, TGB2 and TGB3) that are essential for cell-to-cell movement of the virus. RNAγ encodes the polymerase (GDD) subunit of the RdRp and the γb protein, which has significant roles in viral pathogenesis, long distance movement and suppression of host RNA silencing defences.

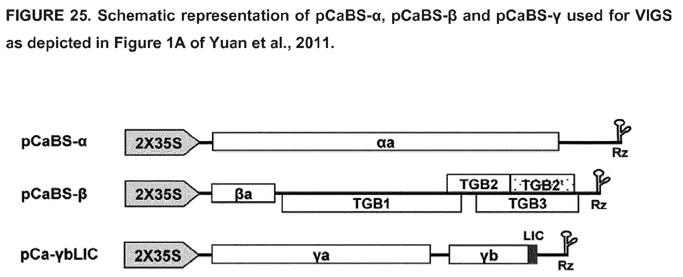

Each vector contains cDNA one of the tripartite genomic RNAs (α, β and γ) between the double Cauliflower mosaic virus 35S promoter (2X35S) and a ribozyme sequence (Rz) from *Tobacco ringspot virus* (TRSV) satellite RNA (see scheme above or Figure 25, respectively). A ligation independent cloning (LIC) site was also included after the γ cDNA for cloning in of the target PCR products.

### Cloning of target PCR products

For cloning the target sequence, BSMVγ was first digested with Apal (Promega, Madison, USA) by incubating a 20 µl mixture at a final concentration of 1x Buffer A (Promega), 0.1x acetylated BSA (Promega), 50 µg/ml BSMVγ, 2.5% Apal in sterile distilled water at 37°C for 4 hours and then 65°C for 15 minutes. 4 µl of this mixture was then added to 16 µl of a reaction mixture to create a 20 µl mixture (Mix A) at a final concentration of 5 mM dTTP, 0.1x BSA, 1x T4 buffer (NEB), 2% T4 DNA Polymerase (NEB) in sterile distilled water.

Simultaneously, a 10 µl reaction (Mix B) was made to a final concentration of 5% Target PCR product, 5 mM dATP, 0.1x BSA, 1x T4 buffer (NEB), 2% T4 DNA Polymerase (NEB) in sterile distilled water.

Both mix A and mix B were incubated at room temperature for 30 minutes and then 75°C for 15 minutes. 2 µl of mix A was then added to mix B and incubated at 65°C for 2 minutes then room temperature for 10 minutes, before transforming into competent DH5α *E. coli* cells.

### Example 5: Virus induced gene silencing (VIGS)

The technique is well known as a tool for delivery of dsRNA into plants (Padmanabhan, Meenu, and Savithramma P. Dinesh-Kumar. "Virus-induced gene silencing as a tool for delivery of dsRNA into plants." Cold Spring Harbor Protocols 2009.2 (2009): pdb-prot5139).

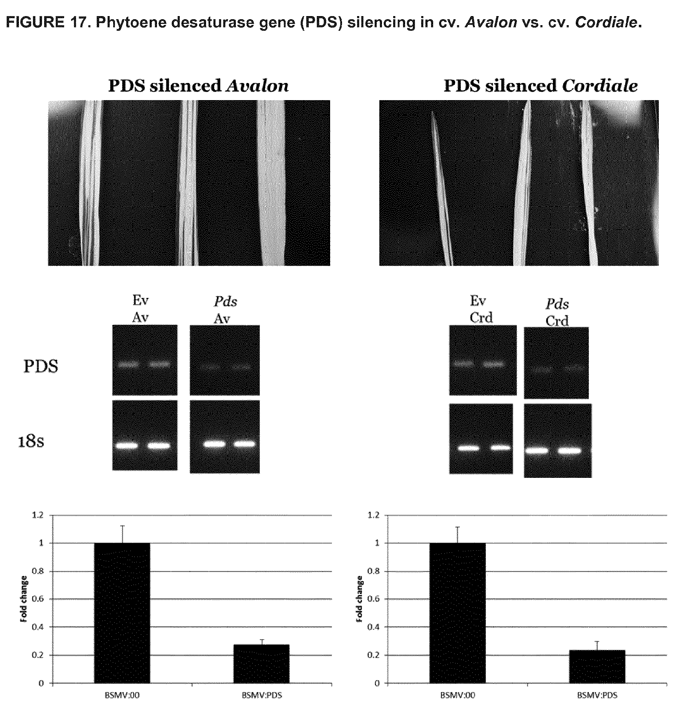

As positive control for VIGS experiments the PDS (phytoene desaturase gene) gene has been used. The gene silencing constructs have been introduced into the plants by means of BMSV (Barley stripe mosaic virus) as VIGS vector. Figure 17 shows exemplary the positive control in two different wheat cultivars 'Avalon' (Av) and 'Cordiale' (Crd). Efficient gene silencing is shown by loss of greenness in leaf tissues.

A structural description of the VIGS vectors is given in the publication Yuan et al. 2011, PLoS ONE, Vol. 6(10), e26468.

### Example 6: Loss-of-function studies in Triticum aestivum

Loss-of-function studies in *T. aestivum* were carried out through the virus-induced gene silencing (VIGS) system (Baulcombe, David C. "Fast forward genetics based on virus-induced gene silencing." Current opinion in plant biology 2.2 (1999): 109-113.) based on the Barley Stripe Mosaic Virus (BSMV) as previously described (Yuan et al., 2011, PLoS ONE, Vol. 6(10), e26468). The specificity and silencing efficiency of the constructs was predicted using siRNA finder si-fi (labtools.ipk-gatersleben.de/index.html). Briefly, the LIC strategy was used to clone a series of WRKY fragments. The selected sequences were amplified with primer pairs, in which the forward primers consisted of 5'-AAGGAAGTTTAA-3' fusions to a 5' plant sequence, and the reverse primers contained 5'-AACCACCACCACCGT-3' fusions to each 3' gene sequence. PCR products were purified, treated at room temperature with T4 DNA polymerase (New England Biolabs, MA, USA) in 1X reaction buffer containing 5 mM dATP for 30 min to generate sticky ends, and heat-treated at 75°C for 10 min to inactivate the polymerase. The Apal-linearized pCa-γbLIC vector was also treated with T4 DNA polymerase in the presence of 5 mM dTTP to generate sticky ends complementary to those of the PCR products. The PCR products (∼200 ng) and pCa-γbLIC vector (20 ng) were mixed, incubated at 66°C for 2 min and slowly cooled to room temperature to anneal their complementary termini. Then, 10 µl aliquots were used for cloning into *Escherichia coli* DH10B, and transformants identified by colony PCR and endonuclease digestions.

### Agroinfiltration of N. benthamiana and viral inoculation of cereals

The BSMV pCa-γbLIC WRKY derivatives were transformed into A. tumefaciens strain GV3101. For agroinfiltration, single colonies were grown overnight at 28°C with constant shaking in 3 ml of LB containing rifampicin (25 µg/ml) and kanamycin (100 µg/ml). Then, 0.5 ml of the cultures were used to inoculate 50 ml LB containing the same antibiotics and grown at 28°C for 10 to 12 h. Bacterial cells were pelleted at 2200 *g* for 10 min, resuspended in infiltration buffer [10 mM MgCl2, 10 mM 2-(N-morpholino) ethanesulfonic acid (MES), pH 5.2, and 0.1 mM acetosyringone] to 0.7 OD600 and incubated at room temperature for at least 3 h. For agroinfiltration, equal amounts of bacteria harboring pCaBS-α, pCaBS-β and pCa-γbLIC (or its derivatives) were mixed, and infiltrated into four to eight N. benthamiana leaves immediately above the cotyledons with a 1-ml needleless syringe. After maintenance in a growth chamber for 5 days post infiltration (dpi), the infiltrated leaves were harvested, ground in 20 mM Na-phosphate buffer (pH7.2) containing 1% celite, and the sap was mechanically inoculated onto the two-leaf stages of wheat.

### Septoria infection assays

The plants to be infected were trimmed so that only the leaf to be infected was left. The remaining leaves were then stuck down flat onto black card, leaving ∼5cm of leaf to be infected. The Septoria strain IPO323, K5090 (GFP strain) and K5097 (GFP strain) were spread onto YPD plates containing no antibiotics (IPO323) or YPD + hygromycin (K5090 and K5097, 25µg/ml) and left for 4 days to grow at 18°C. A few streaks of the Septoria were taken from the plate and dissolved into 10ml of distilled water. To measure the concentration of the Septoria spores 10ul of Septoria mixture was pipetted onto a haemocytometer slide. Using an Axkiospop microscope at x20 magnification the spores were counted. The concentration was then adjusted to, on average, 4 spores 0.05*0.05mm square (1,000,000 spores per ml). 0.1% Tween 20 was added to the Septoria mixture.

Using a cotton bud the Septoria was infected onto the leaves by rubbing the mixture onto the leaves. A new cotton bud was used for each new silenced line of wheat. The tray under the plants was filled with water and the plants were covered with a lid to generate a high humidity for the Septoria to infect. The lids were removed after 4 days. The plants infected with IPO323 were left for 28 days with pictures taken daily and samples taken weekly to check for silencing. The final infected leaves were collected for the spore count, pycnidia count and for extraction of RNA to measure the amount of a fungal gene present. The plants infected with the GFP Septoria strains (K5090 and K5097) were grown and small sections of 0.5cm² were cut and mounted on a slide to be visualized on a SP5 confocal microscope.

### Spore and pycnidia count assays

The leaves were collected and suspended in a sealed box containing damp tissue to increase the humidity and cause the fungus to produce spores. This was left at 18°C for 4 days. The pycnidia were counted over a length of 2 cm of each leaf and averaged for 5 leaves. 5 leaves were then submerged in 10 ml of distilled water, vortexed for 2 minutes and left to stand for 3 hours. The leaves were vortexed again for 2 minutes and 10µl of the water loaded onto a haemocytometer slide. The spores were counted for 4 of the 0.2*0.2mm squares on the Akioskop light microscope at x20 magnification and an average taken from these 4 numbers for the spore count.

A list of exemplary WRKY VIGS fragments used for the corresponding gene silencing is given in Figure 16.

### Example 7: Exemplary results on WRKY10

By knocking down TaWRKY10 in the cv. Avalon, spore and pycnidia counts were significantly reduced (Figure 18). This indicates that the infestation of the wheat plants with Septoria is efficiently inhibited and the plant defense is improved. Consequently the resistance/tolerance of wheat against Septoria is enhanced. In addition, in TaWRKY10 silenced Avalon plants the visible symptoms of Septoria infection were delayed by around 4 days (Figure 19).

The reduced spore and pycnidia counts could be shown also in other cultivars like Cordiale (Figure 20), *KWS Santiago* (Figure 21) and *KWS Lili* (Figure 22).

The enhanced resistance has also been demonstrated in the following experiment. Naturally the cv. *KWS Santiago* is more susceptible to Septoria compared to cv. *KWS Lili.* That can be shown by spore counts (Figure 23) as well as in field trials. The resistant cv. *KWS Lili* has been transformed with an empty VIGS vector (EV) and the susceptible cv. *KWS Santiago* with BSMV:WRKY10. The result is that both the non-silenced cv. *KWS Lili* and the silenced cv. *KWS Santiago* show a comparable Septoria sporulation (Figure 23).

Comparable results as seen for WRKY10 have been achieved for WRKY13, WRKY19, WRKY29, WRKY39, WRKY53B and WRKY68A. For all of these WRKYs, it is suggested that their expression or activity is fertilizer-responsive and/or are characterized by the the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid.

The present example is based on the VIGS-induced silencing of the WRKY genes. However, it is possible to achieve the same or comparable results through other methods: For example, one can build up TILLING populations of a plant and screen for mutations leading to amino acid exchanges or splicing errors. Such mutation can result in a knock-down or knock-out of the WRKY gene (e.g., if the mutation creates an early stop codon), in an altered (reduced) activity of the WRKY protein, or in a shift of the reading frame if splicing errors occur. Besides of TILLING, such mutations can be introduced/targeted by the known genome editing tools like Zinc Finger Nucleases, TAL Effector Nucleases, CRISPR/Cas etc. Another example is the introduction of a DNA construct encoding a double stranded RNA construct suitable for silencing the above WRKY genes (RNAi approach, Example 8).

### Example 8: hpRNA-mediated gene-silencing of WRKYs

### Effectiveness of RNAi in wheat for obtaining lines with silenced WRKY genes

RNAi is a very effective approach for obtaining WRKY silenced wheat lines.

### Plasmid design and construction

For the synthesis of the RNAi vectors, conserved regions of the WRKYs are PCR-amplified from genomic DNA isolated from wheat (*Triticum aestivum*) and cloned in sense and antisense orientation, separated by the *Ubi1* intron to form the inverted repeat (IR) sequences. The vectors are synthesized using the GATEWAY (Invitrogen) recombination technology. Different pairs of primers are used for the amplification of the IR fragments of the vectors. The expression of the IRs is driven by a constitutive promoter, with the nopaline synthase (nos) as terminator sequence.

### Plant material and genetic transformation

Plasmids carrying the RNAi fragments are used in combination with plasmid pAHC25 containing the selectable *bar* gene (Christensen and Quail, 1996, Transgenic Res., 5, 213-218). For bombardment, plasmids are precipitated onto 0.6-µm gold particles at 0.75 pmol/mg gold for the plasmids containing the RNAi fragments. Putative transgenic plants are transferred to soil and grown to maturity in the greenhouse. Homozygous progeny of plants containing the RNAi plasmids are identified by PCR. Then, PCR-positive transgenic plants are further analysed for pathogen resistance as described elsewhere herein.

All references and publications cited or referred to herein are incorporated by reference into the present disclosure in their entirety.

## Claims

1. A cereal plant cell having introduced into its genome at least one modification inhibiting the expression or activity of at least one WRKY protein as compared to a corresponding control plant cell lacking the at least one modification, wherein the at least one WRKY protein is **characterized by** (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid, preferably wherein the at least one modification is introduced into the at least one endogenous WRKY gene encoding the at least one WRKY protein.

2. The cereal plant cell of claim 1, wherein the amino acid sequence motif is D-G/E-X1-X2-W-R-K-Y-G-K/E/Q-K/E-X-X-X3-X-X2 shown in SEQ ID NO: 2, wherein X is any amino acid, X1 is a hydrophobic amino acid, X2 is a polar, non-hydrophobic amino acid, and X3 is a polar, non-aromatic amino acid.

3. The plant cell of claim 1 or 2, wherein the cereal plant cell is the cell of a wheat plant *(Triticum aestivum),* the cell of a rye plant (*Secale cereale*)*,* the cell of a barley plant *(Hordeum vulgare),* or the cell of a maize plant (*Zea mays*).

4. The cereal plant cell of any one of claims 1-3, wherein
(i) the at least one WRKY gene is selected from the group consisting of TaWRKY10 (SEQ ID NO: 3), TaWRKY13 (SEQ ID NO: 5), TaWRKY19 (SEQ ID NO: 7), TaWRKY29 (SEQ ID NO: 9), TaWRKY39 (SEQ ID NO: 11), TaWRKY53B (SEQ ID NO: 13), and TaWRKY68A (SEQ ID NO: 15), or homologs thereof; or
(ii) the at least one WRKY protein is selected from the group consisting of TaWRKY1 0 (SEQ ID NO: 4), TaWRKY13 (SEQ ID NO: 6), TaWRKY19 (SEQ ID NO: 8), TaWRKY29 (SEQ ID NO: 10), TaWRKY39 (SEQ ID NO: 12), TaWRKY53B (SEQ ID NO: 14), and TaWRKY68A (SEQ ID NO: 16), or homologs thereof.

5. The cereal plant cell of any one of claims 1-4, wherein the at least one modification is caused by RNA interference, sense suppression, insertional mutagenesis, mutagenesis or genome editing of the endogenous WRKY gene.

6. A cereal plant, or part thereof, containing the cereal plant cell of any one of claims 1-5, and having an increased pathogen resistance.

7. A cereal plant having an increased pathogen resistance resulting from at least one modification introduced into the genome of the cereal plant inhibiting the expression or activity of at least one WRKY protein as compared to a corresponding control plant lacking the at least one modification, wherein the at least one WRKY protein is **characterized by** (i) a fertilizer-responsive expression or activity, and/or (ii) the amino acid sequence motif D-G/E-X₁-X₂-W-R-K-Y-G-K/E/Q-K/E shown in SEQ ID NO: 1, wherein X₁ is a hydrophobic amino acid and X₂ is a polar, non-hydrophobic amino acid,
preferably wherein the at least one modification is introduced into the at least one endogenous WRKY gene encoding the at least one WRKY protein.

8. The cereal plant of claim 7, wherein the increased pathogen resistance results from:
(a) a modification in at least one endogenous WRKY gene comprising a nucleic acid sequence, or a complement thereof, comprising at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), wherein the modification comprises one or more transposons, one or more insertions, one or more additions, one or more deletions, or one or more point mutations in the at least one endogenous WRKY gene;
(b) at least one introduced transgene comprising a nucleic acid sequence, or a complement thereof, comprising (i) at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), or (ii) at least 20 contiguous nucleotides of the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), SEQ ID NO: 15 (TaWRKY68A), and a homolog thereof, wherein the transgene is operatively linked to a promoter in a sense or antisense orientation; or
(c) at least one introduced polynucleotide sequence comprising one or more subsequences of a nucleic acid sequence, or a complement thereof, comprising at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), wherein the at least one polynucleotide sequence is in RNA interference configuration, preferably wherein the at least one polynucleotide sequence is configured for endogenous gene silencing or virus-induced gene silencing.

9. A method for conferring pathogen resistance to, or increasing pathogen resistance of, a cereal plant cell, or a cereal plant or part thereof, the method comprising the step of inhibiting the expression or activity of at least one WRKY protein of a cereal plant cell, or a cereal plant or part thereof, as compared to a corresponding control plant cell, or plant or part thereof, lacking the disruption, wherein the at least one WRKY protein is **characterized by** (i) a fertilizer-responsive expression or activity, and/or (ii) the amino acid sequence motif D-G/E-X1-X2-W-R-K-Y-G-K/E/Q-K/E shown in SEQ ID NO: 1, wherein X1 is a hydrophobic amino acid and X2 is a polar, non-hydrophobic amino acid, preferably wherein the amino acid sequence motif is D-G/E-X1-X2-W-R-K-Y-G-K/E/Q-K/E-X-X-X3-X-X2 shown in SEQ ID NO: 2, wherein X is any amino acid, X1 is a hydrophobic amino acid, X2 is a polar, non-hydrophobic amino acid, and X3 is a polar, non-aromatic amino acid.

10. The method of claim 9, wherein the step of inhibiting the expression or activity of at least one WRKY protein comprises introducing into the genome of the cereal plant cell, or a cereal plant or part thereof, at least one modification inhibiting the expression or activity of the at least one WRKY protein as compared to a corresponding control plant cell, or a control plant or part thereof, lacking the at least one modification, preferably wherein the at least one modification is introduced into the at least one endogenous WRKY gene encoding the at least one WRKY protein, wherein the at least one WRKY protein is selected from the group consisting of TaWRKY10 (SEQ ID NO: 4), TaWRKY13 (SEQ ID NO: 6), TaWRKY19 (SEQ ID NO: 8), TaWRKY29 (SEQ ID NO: 10), TaWRKY39 (SEQ ID NO: 12), TaWRKY53B (SEQ ID NO: 14), and TaWRKY68A (SEQ ID NO: 16), or homologs thereof.

11. A recombinant DNA construct comprising a polynucleotide sequence, or a fragment thereof, operatively linked to a heterologous promoter, wherein the polynucleotide sequence, or a fragment thereof, produces upon expression in a cereal plant cell, or cereal a plant or part thereof:
(a) a protein that interferes with the expression or activity of at least one endogenous WRKY protein;
(b) an RNA molecule that suppresses the expression of at least one endogenous WRKY protein; or
(c) a loss-of-function mutation in at least one endogenous WRKY gene,
wherein the polynucleotide sequence comprises a nucleic acid sequence, or a complement thereof, comprising (i) at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A).

12. A double-stranded RNA (dsRNA) molecule comprising:
(a) a first strand comprising a nucleotide sequence having a length of at least 19nucleotides and having at least 90% sequence identity to consecutive nucleotides of a target nucleotide sequence; and
(b) a second strand comprising in the 5' to 3' direction a nucleotide sequence which is at least 90% complementary to the first strand and/or is able to hybridize with the first strand,
wherein the target nucleotide sequence is a coding region of an mRNA deduced from a nucleic acid sequence, or a complement thereof, comprising at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A), or homologs thereof.

13. A cereal plant cell, or a cereal plant or part thereof, comprising
(i) the recombinant DNA construct of claim 11, or
(ii) the dsRNA molecule of claim 12,
and having an increased pathogen resistance as compared to a corresponding control plant cell, or plant or part thereof, that does not comprise the said recombinant DNA construct or the said dsRNA molecule, respectively.

14. A composition for topical application to a cereal plant cell, or a cereal plant or part thereof, comprising at least one polynucleotide sequence comprising a nucleic acid sequence, or a complement thereof, comprising (i) at least 70% sequence identity to the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A); or (ii) at least 20 contiguous nucleotides of the nucleotide sequence of any one of SEQ ID NO: 3 (TaWRKY10), SEQ ID NO: 5 (TaWRKY13), SEQ ID NO: 7 (TaWRKY19), SEQ ID NO: 9 (TaWRKY29), SEQ ID NO: 11 (TaWRKY39), SEQ ID NO: 13 (TaWRKY53B), and SEQ ID NO: 15 (TaWRKY68A) or a homolog thereof,
wherein the at least one polynucleotide sequence is configured for RNA interference, preferably wherein the at least one polynucleotide sequence is configured for endogenous gene silencing or virus-induced gene silencing.

15. A method of regulating the expression of at least one WRKY gene in a cereal plant cell, or cereal plant or part thereof, the method comprising applying
(i) a composition comprising the recombinant DNA construct of claim 11,
(ii) a composition comprising the the dsRNA molecule of claim 12, or
(iii) the composition of claim 14,
to a cereal plant cell, or cereal plant or part thereof, preferably wherein the composition is applied onto the surface of the plant cell, or plant or part thereof.

16. The cereal plant, or part thereof, according to any one of claims 6-8, the method of claim 9 or 10, or the cereal plant cell, or a cereal plant or part thereof, according to claim 13, wherein the pathogen resistance is resistance against a fungal pathogen, preferably resistance against *Zymoseptoria tritici.*

17. The cereal plant cell of any one of claims 1-5, the cereal plant, or part thereof, according to any one of claims 6-8, or the method of claim 9 or 10, wherein the fertilizer-responsive expression or activity is nitrogen-responsive expression or activity.
**FIGURE 1: Sequence motifs common to WRKY proteins whose expression or activity is to be inhibited in accordance with the invention.**
SEQ ID NO: 1. Amino acid sequence motif common to WRKY proteins of the invention (length: 11 amino acid residues)
**D-G/E-X₁-X₂-W-R-K-Y-G-K/E/Q-K/E**
wherein X₁ is a hydrophobic amino acid and X₂ is a polar, non-hydrophobic amino acid.
SEQ ID NO: 2. Amino acid sequence motif common to WRKY proteins of the invention (length: 16 amino acid residues)
**D-G/E-X₁-X₂-W-R-K-Y-G-K/E/Q-K/E-X-X-X₃-X-X₂**
wherein X is any amino acid, X₁ is a hydrophobic amino acid, X₂ is a polar, non-hydrophobic amino acid, and X₃ is a polar, non-aromatic amino acid.
